# EUROPEAN PATENT APPLICATION

(11) **EP 3 757 133 A1**
(43) Date of publication of application: **30.12.2020**
(21) Application number: 19751451.6
(22) Date of filing: 01.02.2019
(51) Int. Cl.: C07K 19/00, C12N 15/113, A61P 35/00

(54) **ISOLATED CHIMERIC ANTIGEN RECEPTOR, MODIFIED T CELL COMPRISING SAME AND USE THEREOF**

(30) Priority: 11.02.2018 CN 201810140396
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: ZHANG, Hua, Shanghai 200245 (CN); SHEN, Lianjun, Shanghai 200245 (CN); SU, Qing, Shanghai 200245 (CN); TAO, Weikang, Shanghai 200245 (CN)
(74) Representative: Regimbeau
(86) International application number: PCT/CN2019/074392
(87) International publication number: WO 2019/154313

(57) **Abstract**

An isolated CD19-binding chimeric antigen receptor, a CRISPR-Cas9 system-based method for knocking out TRAC, B2M and PD-1 genes in T cells in vitro, crRNA used in the method, gene knockout T cells obtained according to the method, and a use thereof.

Sequence listing

<110> JIANGSU HENGRUI MEDICINE CO.,LTD.
      SHANGHAI HENGRUI PHARMACEUTICAL CO.,LTD.

<120>  ISOLATED CHIMERIC ANTIGEN RECEPTOR, MODIFIED T CELL COMPRISING SAME
AND USE THEREOF

<130>  B76263EP D40817

<140>  EP19751451.6
<141>  2019 02 01

<160>  57

<170>  SIPOSequenceListing 1.0

<210>  1
<211>  22
<212>  DNA
<213>  Artificial Sequence

<220>
<221>  primer
<223>  sequencing primer PLVX-PF


<400>  1
cattcgatta gtgaacggat ct                                                22

<210>  2
<211>  18
<212>  DNA
<213>  Artificial Sequence

<220>
<221>  primer
<223>  sequencing primer PCDHI-R


<400>  2
gacggcaata tggtggaa                                                     18

<210>  3
<211>  21
<212>  DNA
<213>  Artificial Sequence

<220>
<221> primer
<223> sequencing primer Xd-SR


<400>  3
aaagccatac gggaagcaat a                                                 21

<210>  4
<211>  544
<212>  DNA
<213>  Artificial Sequence

<220>
<221>  promoter
<223>  EF1 promoter


<400>  4
ggatctgcga tcgctccggt gcccgtcagt gggcagagcg cacatcgccc acagtccccg      60
agaagttggg gggaggggtc ggcaattgaa cgggtgccta gagaaggtgg cgcggggtaa     120
actgggaaag tgatgtcgtg tactggctcc gcctttttcc cgagggtggg ggagaaccgt     180
atataagtgc agtagtcgcc gtgaacgttc tttttcgcaa cgggtttgcc gccagaacac     240
agctgaagct tcgaggggct cgcatctctc cttcacgcgc ccgccgccct acctgaggcc     300
gccatccacg ccggttgagt cgcgttctgc cgcctcccgc ctgtggtgcc tcctgaactg     360
cgtccgccgt ctaggtaagt ttaaagctca ggtcgagacc gggcctttgt ccggcgctcc     420
cttggagcct acctagactc agccggctct ccacgctttg cctgaccctg cttgctcaac     480
tctacgtctt tgtttcgttt tctgttctgc gccgttacag atccaagctg tgaccggcgc     540
ctac                                                                  544

<210>  5
<211>  63
<212>  DNA
<213>  Artificial Sequence

<220>
<221>  gene
<223>  Nucleotide sequence of human CD8-alpha leading signal region


<400>  5
atggccttac cagtgaccgc cttgctcctg ccgctggcct tgctgctcca cgccgccagg      60
ccg                                                                    63

<210>  6
<211>  21
<212>  PRT
<213>  Artificial Sequence

<220>
<221>  peptide
<223>  Amino acid sequence of human CD8-alpha leading signal region


<400>  6
Met Ala Leu Pro Val Thr Ala Leu Leu Leu Pro Leu Ala Leu Leu Leu
1               5                   10                  15
His Ala Ala Arg Pro
            20

<210>  7
<211>  135
<212>  DNA
<213>  Artificial Sequence

<220>
<221>  gene
<223>  Nucleotide sequence of human CD8-alpha hinge region


<400>  7
accacgacgc cagcgccgcg accaccaaca ccggcgccca ccatcgcgtc gcagcccctg      60
tccctgcgcc cagaggcgtg ccggccagcg gcggggggcg cagtgcacac gagggggctg     120
gacttcgcct gtgat                                                      135

<210>  8
<211>  45
<212>  PRT
<213>  Artificial Sequence

<220>
<221>  peptide
<223>  Amino acid sequence of human CD8-alpha hinge region


<400>  8
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp
        35                  40                  45

<210>  9
<211>  72
<212>  DNA
<213>  Artificial Sequence

<220>
<221>  gene
<223>  Nucleotide sequence of human CD8-alpha transmembrane region


<400>  9
atctacatct gggcgccctt ggccgggact tgtggggtcc ttctcctgtc actggttatc        60
accctttact gc                                                            72

<210>  10
<211>  24
<212>  PRT
<213>  Artificial Sequence

<220>
<221>  peptide
<223>  Amino acid sequence of human CD8-alpha transmembrane region


<400>  10
Ile Tyr Ile Trp Ala Pro Leu Ala Gly Thr Cys Gly Val Leu Leu Leu
1               5                   10                  15
Ser Leu Val Ile Thr Leu Tyr Cys
            20

<210>  11
<211>  126
<212>  DNA
<213>  Artificial Sequence

<220>
<221>  gene
<223>  Nucleotide sequence of human 4-1BB intracellular region

<400>  11
aaacggggca gaaagaaact cctgtatata ttcaaacaac catttatgag accagtacaa        60
actactcaag aggaagatgg ctgtagctgc cgatttccag aagaagaaga aggaggatgt       120

gaactg                                                                126

<210>  12
<211>  41
<212>  PRT
<213>  Artificial Sequence

<220>
<221>  peptide
<223>  Amino acid sequence of human 4-1BB intracellular region


<400>  12
Lys Arg Gly Arg Lys Lys Leu Leu Tyr Ile Phe Lys Gln Pro Phe Met
1               5                   10                  15
Arg Pro Val Gln Thr Thr Gln Glu Glu Asp Gly Cys Ser Cys Arg Phe
            20                  25                  30
Pro Glu Glu Glu Glu Gly Gly Cys Glu Leu
        35                  40

<210>  13
<211>  336
<212>  DNA
<213>  Artificial Sequence

<220>
<221>  gene
<223>  Nucleotide sequence of human CD3-zeta intracellular region


<400>  13
agagtgaagt tcagcaggag cgcagacgcc cccgcgtaca agcagggcca gaaccagctc      60
tataacgagc tcaatctagg acgaagagag gagtacgatg ttttggacaa gagacgtggc     120
cgggaccctg agatgggggg aaagccgaga aggaagaacc ctcaggaagg cctgtacaat     180
gaactgcaga aagataagat ggcggaggcc tacagtgaga ttgggatgaa aggcgagcgc     240
cggaggggca aggggcacga tggcctttac cagggtctca gtacagccac caaggacacc     300
tacgacgccc ttcacatgca ggccctgccc cctcgc                               336

<210>  14
<211>  112
<212>  PRT
<213>  Artificial Sequence

<220>
<221>  peptide
<223>  Amino acid sequence of human CD3-zeta intracellular region


<400>  14
Arg Val Lys Phe Ser Arg Ser Ala Asp Ala Pro Ala Tyr Lys Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210> 15
<211> 120
<212> PRT
<213> Artificial Sequence

<220>
<221> domain
<223> Heavy chain variable region amino acid sequence of FMC63 antibody

<400> 15
Glu Val Lys Leu Gln Glu Ser Gly Pro Gly Leu Val Ala Pro Ser Gln
1               5                   10                  15
Ser Leu Ser Val Thr Cys Thr Val Ser Gly Val Ser Leu Pro Asp Tyr
            20                  25                  30
Gly Val Ser Trp Ile Arg Gln Pro Pro Arg Lys Gly Leu Glu Trp Leu
        35                  40                  45
Gly Val Ile Trp Gly Ser Glu Thr Thr Tyr Tyr Asn Ser Ala Leu Lys
    50                  55                  60
Ser Arg Leu Thr Ile Ile Lys Asp Asn Ser Lys Ser Gln Val Phe Leu
65                  70                  75                  80
Lys Met Asn Ser Leu Gln Thr Asp Asp Thr Ala Ile Tyr Tyr Cys Ala
                85                  90                  95
Lys His Tyr Tyr Tyr Gly Gly Ser Tyr Ala Met Asp Tyr Trp Gly Gln
            100                 105                 110
Gly Thr Ser Val Thr Val Ser Ser
        115                 120

<210> 16
<211> 120
<212> PRT
<213> Artificial Sequence

<220>
<221> domain
<223> Light chain variable region amino acid sequence of FMC63 antibody

<400> 16
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Arg Ala Asp Ala Ala
            100                 105                 110
Pro Thr Val Ser Ile Phe Pro Pro
        115                 120

<210> 17
<211> 726

<212>  DNA
<213>  Artificial Sequence

<220>
<221>  gene
<223>  Nucleotide sequence of CD19-N1scFv


<400>  17
gacatccaga tgacacagac tacatcctcc ctgtctgcct ctctgggaga cagagtcacc      60
atcagttgca gggcaagtca ggacattagt aaatatttaa attggtatca gcagaaacca     120
gatggaactg ttaaactcct gatctaccat acatcaagat tacactcagg agtcccatca     180
aggttcagtg gcagtgggtc tggaacagat tattctctca ccattagcaa cctggagcaa     240
gaagatattg ccacttactt ttgccaacag ggtaatacgc ttccgtacac gttcggaggg     300
gggactaagt tggaaataac aggtggcggt ggctcgggcg gtggtgggtc gggtggcggc     360
ggatctgagg tgaaactgca ggagtcagga cctggcctgg tggcgccctc acagagcctg     420
tccgtcacat gcactgtctc aggggtctca ttacccgact atggtgtaag ctggattcgc     480
cagcctccac gaaagggtct ggagtggctg ggagtaatat ggggtagtga aaccacatac     540
tataattcag ctctcaaatc cagactgacc atcatcaagg acaactccaa gagccaagtt     600
ttcttaaaaa tgaacagtct gcaaactgat gacacagcca tttactactg tgccaaacat     660
tattactacg gtggtagcta tgctatggac tactggggcc aaggaacctc agtcaccgtc     720
tcctca                                                                726

<210>  18
<211>  242
<212>  PRT
<213>  Artificial Sequence

<220>
<221>  domain
<223>  Amino acid sequence of CD19-N1scFv


<400>  18
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Gly Gly Gly Gly Ser
            100                 105                 110
Gly Gly Gly Gly Ser Gly Gly Gly Gly Ser Glu Val Lys Leu Gln Glu
        115                 120                 125
Ser Gly Pro Gly Leu Val Ala Pro Ser Gln Ser Leu Ser Val Thr Cys
    130                 135                 140
Thr Val Ser Gly Val Ser Leu Pro Asp Tyr Gly Val Ser Trp Ile Arg
145                 150                 155                 160
Gln Pro Pro Arg Lys Gly Leu Glu Trp Leu Gly Val Ile Trp Gly Ser
                165                 170                 175
Glu Thr Thr Tyr Tyr Asn Ser Ala Leu Lys Ser Arg Leu Thr Ile Ile
            180                 185                 190
Lys Asp Asn Ser Lys Ser Gln Val Phe Leu Lys Met Asn Ser Leu Gln
        195                 200                 205
Thr Asp Asp Thr Ala Ile Tyr Tyr Cys Ala Lys His Tyr Tyr Tyr Gly

210                 215                 220
Gly Ser Tyr Ala Met Asp Tyr Trp Gly Gln Gly Thr Ser Val Thr Val
225                 230                 235                 240
Ser Ser

<210>  19
<211>  732
<212>  DNA
<213>  Artificial Sequence

<220>
<221>  gene
<223>  Nucleotide sequence of CD19-N2 scFv

<400>  19
gacatccaga tgacacagac tacatcctcc ctgtctgcct ctctgggaga cagagtcacc      60
atcagttgca gggcaagtca ggacattagt aaatatttaa attggtatca gcagaaacca     120
gatggaactg ttaaactcct gatctaccat acatcaagat tacactcagg agtcccatca     180
aggttcagtg gcagtgggtc tggaacagat tattctctca ccattagcaa cctggagcaa     240
gaagatattg ccacttactt ttgccaacag ggtaatacgc ttccgtacac gttcggaggg     300
gggactaagt tggaaataac acgggctggt ggcggtggct cgggcggtgg tgggtcgggt     360
ggcggcggat ctgaggtgaa actgcaggag tcaggacctg gcctggtggc gccctcacag     420
agcctgtccg tcacatgcac tgtctcaggg gtctcattac ccgactatgg tgtaagctgg     480
attcgccagc ctccacgaaa gggtctggag tggctgggag taatatgggg tagtgaaacc     540
acatactata attcagctct caaatccaga ctgaccatca tcaaggacaa ctccaagagc     600
caagttttct taaaaatgaa cagtctgcaa actgatgaca cagccattta ctactgtgcc     660
aaacattatt actacggtgg tagctatgct atggactact ggggccaagg aacctcagtc     720
accgtctcct ca                                                         732

<210>  20
<211>  244
<212>  PRT
<213>  Artificial Sequence

<220>
<221>  domain
<223>  Amino acid sequence of CD19-N2 scFv

<400>  20
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Arg Ala Gly Gly Gly
            100                 105                 110
Gly Ser Gly Gly Gly Gly Ser Gly Gly Gly Gly Ser Glu Val Lys Leu
        115                 120                 125
Gln Glu Ser Gly Pro Gly Leu Val Ala Pro Ser Gln Ser Leu Ser Val
    130                 135                 140
Thr Cys Thr Val Ser Gly Val Ser Leu Pro Asp Tyr Gly Val Ser Trp

145                 150                 155                 160
Ile Arg Gln Pro Pro Arg Lys Gly Leu Glu Trp Leu Gly Val Ile Trp
                165                 170                 175
Gly Ser Glu Thr Thr Tyr Tyr Asn Ser Ala Leu Lys Ser Arg Leu Thr
            180                 185                 190
Ile Ile Lys Asp Asn Ser Lys Ser Gln Val Phe Leu Lys Met Asn Ser
        195                 200                 205
Leu Gln Thr Asp Asp Thr Ala Ile Tyr Tyr Cys Ala Lys His Tyr Tyr
    210                 215                 220
Tyr Gly Gly Ser Tyr Ala Met Asp Tyr Trp Gly Gln Gly Thr Ser Val
225                 230                 235                 240
Thr Val Ser Ser

<210>  21
<211>  738
<212>  DNA
<213>  Artificial Sequence

<220>
<221>  gene
<223>  Nucleotide sequence of CD19-N3 scFv

<400>  21
gacatccaga tgacacagac tacatcctcc ctgtctgcct ctctgggaga cagagtcacc      60
atcagttgca gggcaagtca ggacattagt aaatatttaa attggtatca gcagaaacca     120
gatggaactg ttaaactcct gatctaccat acatcaagat tacactcagg agtcccatca     180
aggttcagtg gcagtgggtc tggaacagat tattctctca ccattagcaa cctggagcaa     240
gaagatattg ccacttactt ttgccaacag ggtaatacgc ttccgtacac gttcggaggg     300
gggactaagt tggaaataac acgggcggcc gcaggtggcg gtggctcggg cggtggtggg     360
tcgggtggcg gcggatctga ggtgaaactg caggagtcag gacctggcct ggtggcgccc     420
tcacagagcc tgtccgtcac atgcactgtc tcaggggtct cattacccga ctatggtgta     480
agctggattc gccagcctcc acgaaagggt ctggagtggc tgggagtaat atggggtagt     540
gaaaccacat actataattc agctctcaaa tccagactga ccatcatcaa ggacaactcc     600
aagagccaag ttttcttaaa aatgaacagt ctgcaaactg atgacacagc catttactac     660
tgtgccaaac attattacta cggtggtagc tatgctatgg actactgggg ccaaggaacc     720
tcagtcaccg tctcctca                                                   738

<210>  22
<211>  246
<212>  PRT
<213>  Artificial Sequence

<220>
<221>  domain
<223>  Amino acid sequence of CD19-N3 scFv

<400>  22
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30
Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95

Thr Phe Gly Gly Gly Thr Lys Leu Glu Ile Thr Arg Ala Ala Ala Gly
            100                 105                 110
Gly Gly Gly Ser Gly Gly Gly Gly Ser Gly Gly Gly Gly Ser Glu Val
        115                 120                 125
Lys Leu Gln Glu Ser Gly Pro Gly Leu Val Ala Pro Ser Gln Ser Leu
    130                 135                 140
Ser Val Thr Cys Thr Val Ser Gly Val Ser Leu Pro Asp Tyr Gly Val
145                 150                 155                 160
Ser Trp Ile Arg Gln Pro Pro Arg Lys Gly Leu Glu Trp Leu Gly Val
                165                 170                 175
Ile Trp Gly Ser Glu Thr Thr Tyr Tyr Asn Ser Ala Leu Lys Ser Arg
            180                 185                 190
Leu Thr Ile Ile Lys Asp Asn Ser Lys Ser Gln Val Phe Leu Lys Met
        195                 200                 205
Asn Ser Leu Gln Thr Asp Asp Thr Ala Ile Tyr Tyr Cys Ala Lys His
    210                 215                 220
Tyr Tyr Tyr Gly Gly Ser Tyr Ala Met Asp Tyr Trp Gly Gln Gly Thr
225                 230                 235                 240
Ser Val Thr Val Ser Ser
                245

<210>  23
<211>  765
<212>  DNA
<213>  Artificial Sequence

<220>
<221>  gene
<223>  Nucleotide sequence of CD19-N4 scFv

<400>  23
gacatccaga tgacacagac tacatcctcc ctgtctgcct ctctgggaga cagagtcacc      60
atcagttgca gggcaagtca ggacattagt aaatatttaa attggtatca gcagaaacca     120
gatggaactg ttaaactcct gatctaccat acatcaagat tacactcagg agtcccatca     180
aggttcagtg gcagtgggtc tggaacagat tattctctca ccattagcaa cctggagcaa     240
gaagatattg ccacttactt ttgccaacag ggtaatacgc ttccgtacac gttcggaggg     300
gggactaagt tggaaataac acgggctgat gctgcaccaa ctgtatccat cttcccacca     360
ggtggcggtg gctcgggcgg tggtgggtcg ggtggcggcg gatctgaggt gaaactgcag     420
gagtcaggac ctggcctggt ggcgccctca cagagcctgt ccgtcacatg cactgtctca     480
ggggtctcat tacccgacta tggtgtaagc tggattcgcc agcctccacg aaagggtctg     540
gagtggctgg gagtaatatg gggtagtgaa accacatact ataattcagc tctcaaatcc     600
agactgacca tcatcaagga caactccaag agccaagttt tcttaaaaat gaacagtctg     660
caaactgatg acacagccat ttactactgt gccaaacatt attactacgg tggtagctat     720
gctatggact actggggcca aggaacctca gtcaccgtct cctca                     765

<210>  24
<211>  255
<212>  PRT
<213>  Artificial Sequence

<220>
<221>  domain
<223>  Amino acid sequence of CD19-N4 scFv

<400>  24
Asp Ile Gln Met Thr Gln Thr Thr Ser Ser Leu Ser Ala Ser Leu Gly
1               5                   10                  15
Asp Arg Val Thr Ile Ser Cys Arg Ala Ser Gln Asp Ile Ser Lys Tyr
            20                  25                  30

Leu Asn Trp Tyr Gln Gln Lys Pro Asp Gly Thr Val Lys Leu Leu Ile
        35                  40                  45
Tyr His Thr Ser Arg Leu His Ser Gly Val Pro Ser Arg Phe Ser Gly
    50                  55                  60
Ser Gly Ser Gly Thr Asp Tyr Ser Leu Thr Ile Ser Asn Leu Glu Gln
65                  70                  75                  80
Glu Asp Ile Ala Thr Tyr Phe Cys Gln Gln Gly Asn Thr Leu Pro Tyr
                85                  90                  95
Thr Phe Gly Gly Gly Thr Lys Leu Glu Ile Thr Arg Ala Asp Ala Ala
            100                 105                 110
Pro Thr Val Ser Ile Phe Pro Pro Gly Gly Gly Gly Ser Gly Gly Gly
        115                 120                 125
Gly Ser Gly Gly Gly Gly Ser Glu Val Lys Leu Gln Glu Ser Gly Pro
    130                 135                 140
Gly Leu Val Ala Pro Ser Gln Ser Leu Ser Val Thr Cys Thr Val Ser
145                 150                 155                 160
Gly Val Ser Leu Pro Asp Tyr Gly Val Ser Trp Ile Arg Gln Pro Pro
                165                 170                 175
Arg Lys Gly Leu Glu Trp Leu Gly Val Ile Trp Gly Ser Glu Thr Thr
            180                 185                 190
Tyr Tyr Asn Ser Ala Leu Lys Ser Arg Leu Thr Ile Ile Lys Asp Asn
        195                 200                 205
Ser Lys Ser Gln Val Phe Leu Lys Met Asn Ser Leu Gln Thr Asp Asp
    210                 215                 220
Thr Ala Ile Tyr Tyr Cys Ala Lys His Tyr Tyr Tyr Gly Gly Ser Tyr
225                 230                 235                 240
Ala Met Asp Tyr Trp Gly Gln Gly Thr Ser Val Thr Val Ser Ser
                245                 250                 255

<210>  25
<211>  1464
<212>  DNA
<213>  Artificial Sequence

<220>
<221>  gene
<223>  Nucleotide sequence of CD19 CAR-N1


<400>  25
atggccttac cagtgaccgc cttgctcctg ccgctggcct tgctgctcca cgccgccagg      60
ccggacatcc agatgacaca gactacatcc tccctgtctg cctctctggg agacagagtc     120
accatcagtt gcagggcaag tcaggacatt agtaaatatt taaattggta tcagcagaaa     180
ccagatggaa ctgttaaact cctgatctac catacatcaa gattacactc aggagtccca     240
tcaaggttca gtggcagtgg gtctggaaca gattattctc tcaccattag caacctggag     300
caagaagata ttgccactta cttttgccaa cagggtaata cgcttccgta cacgttcgga     360
ggggggacta agttggaaat aacaggtggc ggtggctcgg gcggtggtgg gtcgggtggc     420
ggcggatctg aggtgaaact gcaggagtca ggacctggcc tggtggcgcc ctcacagagc     480
ctgtccgtca catgcactgt ctcaggggtc tcattacccg actatggtgt aagctggatt     540
cgccagcctc cacgaaaggg tctggagtgg ctgggagtaa tatggggtag tgaaaccaca     600
tactataatt cagctctcaa atccagactg accatcatca aggacaactc caagagccaa     660
gttttcttaa aaatgaacag tctgcaaact gatgacacag ccatttacta ctgtgccaaa     720
cattattact acggtggtag ctatgctatg gactactggg gccaaggaac ctcagtcacc     780
gtctcctcaa ccacgacgcc agcgccgcga ccaccaacac cggcgcccac catcgcgtcg     840
cagcccctgt ccctgcgccc agaggcgtgc cggccagcgg cggggggcgc agtgcacacg     900
agggggctgg acttcgcctg tgatatctac atctgggcgc ccttggccgg gacttgtggg     960
gtccttctcc tgtcactggt tatcaccctt tactgcaaac ggggcagaaa gaaactcctg    1020
tatatattca aacaaccatt tatgagacca gtacaaacta ctcaagagga agatggctgt    1080
agctgccgat ttccagaaga agaagaagga ggatgtgaac tgagagtgaa gttcagcagg    1140
agcgcagacg cccccgcgta ccagcagggc cagaaccagc tctataacga gctcaatcta    1200
ggacgaagag aggagtacga tgttttggac aagagacgtg gccgggaccc tgagatgggg    1260

ggaaagccga gaaggaagaa ccctcaggaa ggcctgtaca atgaactgca gaaagataag 1320
atggcggagg cctacagtga gattgggatg aaaggcgagc gccggagggg caaggggcac 1380
gatggccttt accagggtct cagtacagcc accaaggaca cctacgacgc ccttcacatg 1440
caggccctgc cccctcgctg ataa 1464

<210> 26
<211> 486
<212> PRT
<213> Artificial Sequence

<220>
<221> peptide
<223> Amino acid sequence of CD19 CAR-N1

<400> 26
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Gly Gly Gly Ser Gly Gly Gly Gly Ser Gly Gly Gly Gly Ser Glu
    130                 135                 140
Val Lys Leu Gln Glu Ser Gly Pro Gly Leu Val Ala Pro Ser Gln Ser
145                 150                 155                 160
Leu Ser Val Thr Cys Thr Val Ser Gly Val Ser Leu Pro Asp Tyr Gly
                165                 170                 175
Val Ser Trp Ile Arg Gln Pro Pro Arg Lys Gly Leu Glu Trp Leu Gly
            180                 185                 190
Val Ile Trp Gly Ser Glu Thr Thr Tyr Tyr Asn Ser Ala Leu Lys Ser
        195                 200                 205
Arg Leu Thr Ile Ile Lys Asp Asn Ser Lys Ser Gln Val Phe Leu Lys
    210                 215                 220
Met Asn Ser Leu Gln Thr Asp Asp Thr Ala Ile Tyr Tyr Cys Ala Lys
225                 230                 235                 240
His Tyr Tyr Tyr Gly Gly Ser Tyr Ala Met Asp Tyr Trp Gly Gln Gly
                245                 250                 255
Thr Ser Val Thr Val Ser Ser Thr Thr Thr Pro Ala Pro Arg Pro Pro
            260                 265                 270
Thr Pro Ala Pro Thr Ile Ala Ser Gln Pro Leu Ser Leu Arg Pro Glu
        275                 280                 285
Ala Cys Arg Pro Ala Ala Gly Gly Ala Val His Thr Arg Gly Leu Asp
    290                 295                 300
Phe Ala Cys Asp Ile Tyr Ile Trp Ala Pro Leu Ala Gly Thr Cys Gly
305                 310                 315                 320
Val Leu Leu Leu Ser Leu Val Ile Thr Leu Tyr Cys Lys Arg Gly Arg
                325                 330                 335
Lys Lys Leu Leu Tyr Ile Phe Lys Gln Pro Phe Met Arg Pro Val Gln
            340                 345                 350
Thr Thr Gln Glu Glu Asp Gly Cys Ser Cys Arg Phe Pro Glu Glu Glu

355                 360                 365
Glu Gly Gly Cys Glu Leu Arg Val Lys Phe Ser Arg Ser Ala Asp Ala
    370                 375                 380
Pro Ala Tyr Gln Gln Gly Gln Asn Gln Leu Tyr Asn Glu Leu Asn Leu
385                 390                 395                 400
Gly Arg Arg Glu Glu Tyr Asp Val Leu Asp Lys Arg Arg Gly Arg Asp
                405                 410                 415
Pro Glu Met Gly Gly Lys Pro Arg Arg Lys Asn Pro Gln Glu Gly Leu
            420                 425                 430
Tyr Asn Glu Leu Gln Lys Asp Lys Met Ala Glu Ala Tyr Ser Glu Ile
        435                 440                 445
Gly Met Lys Gly Glu Arg Arg Arg Gly Lys Gly His Asp Gly Leu Tyr
    450                 455                 460
Gln Gly Leu Ser Thr Ala Thr Lys Asp Thr Tyr Asp Ala Leu His Met
465                 470                 475                 480
Gln Ala Leu Pro Pro Arg
                485

<210>  27
<211>  1470
<212>  DNA
<213>  Artificial Sequence

<220>
<221>  gene
<223>  Nucleotide sequence of CD19 CAR-N2


<400>  27
atggccttac cagtgaccgc cttgctcctg ccgctggcct tgctgctcca cgccgccagg      60
ccggacatcc agatgacaca gactacatcc tccctgtctg cctctctggg agacagagtc     120
accatcagtt gcagggcaag tcaggacatt agtaaatatt taaattggta tcagcagaaa     180
ccagatggaa ctgttaaact cctgatctac catacatcaa gattacactc aggagtccca     240
tcaaggttca gtggcagtgg gtctggaaca gattattctc tcaccattag caacctggag     300
caagaagata ttgccactta cttttgccaa cagggtaata cgcttccgta cacgttcgga     360
ggggggacta agttggaaat aacacgggct ggtggcggtg gctcgggcgg tggtgggtcg     420
ggtggcggcg gatctgaggt gaaactgcag gagtcaggac ctggcctggt ggcgccctca     480
cagagcctgt ccgtcacatg cactgtctca ggggtctcat tacccgacta tggtgtaagc     540
tggattcgcc agcctccacg aaagggtctg gagtggctgg gagtaatatg gggtagtgaa     600
accacatact ataattcagc tctcaaatcc agactgacca tcatcaagga caactccaag     660
agccaagttt tcttaaaaat gaacagtctg caaactgatg acacagccat ttactactgt     720
gccaaacatt attactacgg tggtagctat gctatggact actggggcca aggaacctca     780
gtcaccgtct cctcaaccac gacgccagcg ccgcgaccac caacaccggc gcccaccatc     840
gcgtcgcagc ccctgtccct gcgcccagag gcgtgccggc cagcggcggg gggcgcagtg     900
cacacgaggg ggctggactt cgcctgtgat atctacatct gggcgccctt ggccgggact     960
tgtggggtcc ttctcctgtc actggttatc accctttact gcaaacgggg cagaaagaaa    1020
ctcctgtata tattcaaaca accatttatg agaccagtac aaactactca agaggaagat    1080
ggctgtagct gccgatttcc agaagaagaa gaaggaggat gtgaactgag agtgaagttc    1140
agcaggagcg cagacgcccc cgcgtaccag cagggccaga accagctcta taacgagctc    1200
aatctaggac gaagagagga gtacgatgtt ttggacaaga gacgtggccg ggaccctgag    1260
atggggggaa agccgagaag gaagaaccct caggaaggcc tgtacaatga actgcagaaa    1320
gataagatgg cggaggccta cagtgagatt gggatgaaag gcgagcgccg gaggggcaag    1380
gggcacgatg gcctttacca gggtctcagt acagccacca aggacaccta cgacgccctt    1440
cacatgcagg ccctgccccc tcgctgataa                                      1470

<210>  28
<211>  488
<212>  PRT
<213>  Artificial Sequence

<220>

<221>  peptide
<223>  Amino acid sequence of CD19 CAR-N2

<400>  28
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Arg Ala Gly Gly Gly Gly Ser Gly Gly Gly Gly Ser Gly Gly Gly Gly
    130                 135                 140
Ser Glu Val Lys Leu Gln Glu Ser Gly Pro Gly Leu Val Ala Pro Ser
145                 150                 155                 160
Gln Ser Leu Ser Val Thr Cys Thr Val Ser Gly Val Ser Leu Pro Asp
                165                 170                 175
Tyr Gly Val Ser Trp Ile Arg Gln Pro Pro Arg Lys Gly Leu Glu Trp
            180                 185                 190
Leu Gly Val Ile Trp Gly Ser Glu Thr Thr Tyr Tyr Asn Ser Ala Leu
        195                 200                 205
Lys Ser Arg Leu Thr Ile Ile Lys Asp Asn Ser Lys Ser Gln Val Phe
    210                 215                 220
Leu Lys Met Asn Ser Leu Gln Thr Asp Asp Thr Ala Ile Tyr Tyr Cys
225                 230                 235                 240
Ala Lys His Tyr Tyr Tyr Gly Gly Ser Tyr Ala Met Asp Tyr Trp Gly
                245                 250                 255
Gln Gly Thr Ser Val Thr Val Ser Ser Thr Thr Thr Pro Ala Pro Arg
            260                 265                 270
Pro Pro Thr Pro Ala Pro Thr Ile Ala Ser Gln Pro Leu Ser Leu Arg
        275                 280                 285
Pro Glu Ala Cys Arg Pro Ala Ala Gly Gly Ala Val His Thr Arg Gly
    290                 295                 300
Leu Asp Phe Ala Cys Asp Ile Tyr Ile Trp Ala Pro Leu Ala Gly Thr
305                 310                 315                 320
Cys Gly Val Leu Leu Leu Ser Leu Val Ile Thr Leu Tyr Cys Lys Arg
                325                 330                 335
Gly Arg Lys Lys Leu Leu Tyr Ile Phe Lys Gln Pro Phe Met Arg Pro
            340                 345                 350
Val Gln Thr Thr Gln Glu Glu Asp Gly Cys Ser Cys Arg Phe Pro Glu
        355                 360                 365
Glu Glu Glu Gly Gly Cys Glu Leu Arg Val Lys Phe Ser Arg Ser Ala
    370                 375                 380
Asp Ala Pro Ala Tyr Gln Gln Gly Gln Asn Gln Leu Tyr Asn Glu Leu
385                 390                 395                 400
Asn Leu Gly Arg Arg Glu Glu Tyr Asp Val Leu Asp Lys Arg Arg Gly
                405                 410                 415
Arg Asp Pro Glu Met Gly Gly Lys Pro Arg Arg Lys Asn Pro Gln Glu
            420                 425                 430
Gly Leu Tyr Asn Glu Leu Gln Lys Asp Lys Met Ala Glu Ala Tyr Ser
        435                 440                 445
Glu Ile Gly Met Lys Gly Glu Arg Arg Arg Gly Lys Gly His Asp Gly

450                 455                 460
Leu Tyr Gln Gly Leu Ser Thr Ala Thr Lys Asp Thr Tyr Asp Ala Leu
465                 470                 475                 480
His Met Gln Ala Leu Pro Pro Arg
                485

<210>  29
<211>  1476
<212>  DNA
<213>  Artificial Sequence

<220>
<221>  gene
<223>  Nucleotide sequence of CD19 CAR-N3


<400>  29
atggccttac cagtgaccgc cttgctcctg ccgctggcct tgctgctcca cgccgccagg      60
ccggacatcc agatgacaca gactacatcc tccctgtctg cctctctggg agacagagtc     120
accatcagtt gcagggcaag tcaggacatt agtaaatatt taaattggta tcagcagaaa     180
ccagatggaa ctgttaaact cctgatctac catacatcaa gattacactc aggagtccca     240
tcaaggttca gtggcagtgg gtctggaaca gattattctc tcaccattag caacctggag     300
caagaagata ttgccactta cttttgccaa cagggtaata cgcttccgta cacgttcgga     360
ggggggacta agttggaaat aacacgggcg gccgcaggtg gcggtggctc gggcggtggt     420
gggtcgggtg gcggcggatc tgaggtgaaa ctgcaggagt caggacctgg cctggtggcg     480
ccctcacaga gcctgtccgt cacatgcact gtctcagggg tctcattacc cgactatggt     540
gtaagctgga ttcgccagcc tccacgaaag ggtctggagt ggctgggagt aatatggggt     600
agtgaaacca catactataa ttcagctctc aaatccagac tgaccatcat caaggacaac     660
tccaagagcc aagttttctt aaaaatgaac agtctgcaaa ctgatgacac agccatttac     720
tactgtgcca aacattatta ctacggtggt agctatgcta tggactactg gggccaagga     780
acctcagtca ccgtctcctc aaccacgacg ccagcgccgc gaccaccaac accggcgccc     840
accatcgcgt cgcagcccct gtccctgcgc ccagaggcgt gccggccagc ggcggggggc     900
gcagtgcaca cgagggggct ggacttcgcc tgtgatatct acatctgggc gcccttggcc     960
gggacttgtg gggtccttct cctgtcactg gttatcaccc tttactgcaa acggggcaga    1020
aagaaactcc tgtatatatt caaacaacca tttatgagac cagtacaaac tactcaagag    1080
gaagatggct gtagctgccg atttccagaa gaagaagaag gaggatgtga actgagagtg    1140
aagttcagca ggagcgcaga cgcccccgcg taccagcagg gccagaacca gctctataac    1200
gagctcaatc taggacgaag agaggagtac gatgttttgg acaagagacg tggccgggac    1260
cctgagatgg ggggaaagcc gagaaggaag aaccctcagg aaggcctgta caatgaactg    1320
cagaaagata agatggcgga ggcctacagt gagattggga tgaaaggcga gcgccggagg    1380
ggcaaggggc acgatggcct ttaccagggt ctcagtacag ccaccaagga cacctacgac    1440
gcccttcaca tgcaggccct gccccctcgc tgataa                              1476

<210>  30
<211>  490
<212>  PRT
<213>  Artificial Sequence

<220>
<221>  peptide
<223>  Amino acid sequence of CD19 CAR-N3


<400>  30
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr

50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Arg Ala Ala Ala Gly Gly Gly Gly Ser Gly Gly Gly Gly Ser Gly Gly
    130                 135                 140
Gly Gly Ser Glu Val Lys Leu Gln Glu Ser Gly Pro Gly Leu Val Ala
145                 150                 155                 160
Pro Ser Gln Ser Leu Ser Val Thr Cys Thr Val Ser Gly Val Ser Leu
                165                 170                 175
Pro Asp Tyr Gly Val Ser Trp Ile Arg Gln Pro Pro Arg Lys Gly Leu
            180                 185                 190
Glu Trp Leu Gly Val Ile Trp Gly Ser Glu Thr Thr Tyr Tyr Asn Ser
        195                 200                 205
Ala Leu Lys Ser Arg Leu Thr Ile Ile Lys Asp Asn Ser Lys Ser Gln
    210                 215                 220
Val Phe Leu Lys Met Asn Ser Leu Gln Thr Asp Asp Thr Ala Ile Tyr
225                 230                 235                 240
Tyr Cys Ala Lys His Tyr Tyr Tyr Gly Gly Ser Tyr Ala Met Asp Tyr
                245                 250                 255
Trp Gly Gln Gly Thr Ser Val Thr Val Ser Ser Thr Thr Thr Pro Ala
            260                 265                 270
Pro Arg Pro Pro Thr Pro Ala Pro Thr Ile Ala Ser Gln Pro Leu Ser
        275                 280                 285
Leu Arg Pro Glu Ala Cys Arg Pro Ala Ala Gly Gly Ala Val His Thr
    290                 295                 300
Arg Gly Leu Asp Phe Ala Cys Asp Ile Tyr Ile Trp Ala Pro Leu Ala
305                 310                 315                 320
Gly Thr Cys Gly Val Leu Leu Leu Ser Leu Val Ile Thr Leu Tyr Cys
                325                 330                 335
Lys Arg Gly Arg Lys Lys Leu Leu Tyr Ile Phe Lys Gln Pro Phe Met
            340                 345                 350
Arg Pro Val Gln Thr Thr Gln Glu Glu Asp Gly Cys Ser Cys Arg Phe
        355                 360                 365
Pro Glu Glu Glu Glu Gly Gly Cys Glu Leu Arg Val Lys Phe Ser Arg
    370                 375                 380
Ser Ala Asp Ala Pro Ala Tyr Gln Gln Gly Gln Asn Gln Leu Tyr Asn
385                 390                 395                 400
Glu Leu Asn Leu Gly Arg Arg Glu Glu Tyr Asp Val Leu Asp Lys Arg
                405                 410                 415
Arg Gly Arg Asp Pro Glu Met Gly Gly Lys Pro Arg Arg Lys Asn Pro
            420                 425                 430
Gln Glu Gly Leu Tyr Asn Glu Leu Gln Lys Asp Lys Met Ala Glu Ala
        435                 440                 445
Tyr Ser Glu Ile Gly Met Lys Gly Glu Arg Arg Arg Gly Lys Gly His
    450                 455                 460
Asp Gly Leu Tyr Gln Gly Leu Ser Thr Ala Thr Lys Asp Thr Tyr Asp
465                 470                 475                 480
Ala Leu His Met Gln Ala Leu Pro Pro Arg
                485                 490

<210>  31
<211>  1503
<212>  DNA
<213>  Artificial Sequence

<220>
<221>  gene
<223>  Nucleotide sequence of CD19 CAR-N4


<400>  31
atggccttac cagtgaccgc cttgctcctg ccgctggcct tgctgctcca cgccgccagg      60
ccggacatcc agatgacaca gactacatcc tccctgtctg cctctctggg agacagagtc     120
accatcagtt gcagggcaag tcaggacatt agtaaatatt taaattggta tcagcagaaa     180
ccagatggaa ctgttaaact cctgatctac catacatcaa gattacactc aggagtccca     240
tcaaggttca gtggcagtgg gtctggaaca gattattctc tcaccattag caacctggag     300
caagaagata ttgccactta cttttgccaa cagggtaata cgcttccgta cacgttcgga     360
ggggggacta agttggaaat aacacgggct gatgctgcac caactgtatc catcttccca     420
ccaggtggcg gtggctcggg cggtggtggg tcgggtggcg gcggatctga ggtgaaactg     480
caggagtcag gacctggcct ggtggcgccc tcacagagcc tgtccgtcac atgcactgtc     540
tcaggggtct cattacccga ctatggtgta agctggattc gccagcctcc acgaaagggt     600
ctggagtggc tgggagtaat atggggtagt gaaaccacat actataattc agctctcaaa     660
tccagactga ccatcatcaa ggacaactcc aagagccaag ttttcttaaa aatgaacagt     720
ctgcaaactg atgacacagc catttactac tgtgccaaac attattacta cggtggtagc     780
tatgctatgg actactgggg ccaaggaacc tcagtcaccg tctcctcaac cacgacgcca     840
gcgccgcgac caccaacacc ggcgcccacc atcgcgtcgc agcccctgtc cctgcgccca     900
gaggcgtgcc ggccagcggc ggggggcgca gtgcacacga gggggctgga cttcgcctgt     960
gatatctaca tctgggcgcc cttggccggg acttgtgggg tccttctcct gtcactggtt    1020
atcacccttt actgcaaacg gggcagaaag aaactcctgt atatattcaa acaaccattt    1080
atgagaccag tacaaactac tcaagaggaa gatggctgta gctgccgatt tccagaagaa    1140
gaagaaggag gatgtgaact gagagtgaag ttcagcagga gcgcagacgc ccccgcgtac    1200
cagcagggcc agaaccagct ctataacgag ctcaatctag gacgaagaga ggagtacgat    1260
gttttggaca agagacgtgg ccgggaccct gagatggggg gaaagccgag aaggaagaac    1320
cctcaggaag gcctgtacaa tgaactgcag aaagataaga tggcggaggc ctacagtgag    1380
attgggatga aaggcgagcg ccggaggggc aaggggcacg atggccttta ccagggtctc    1440
agtacagcca ccaaggacac ctacgacgcc cttcacatgc aggccctgcc ccctcgctga    1500
taa                                                                  1503

<210>  32
<211>  499
<212>  PRT
<213>  Artificial Sequence

<220>
<221>  peptide
<223>  Amino acid sequence of CD19 CAR-N4


<400>  32
   Met Ala Leu Pro Val Thr Ala Leu Leu Leu Pro Leu Ala Leu Leu Leu
   1               5                   10                  15
   His Ala Ala Arg Pro Asp Ile Gln Met Thr Gln Thr Thr Ser Ser Leu
               20                  25                  30
   Ser Ala Ser Leu Gly Asp Arg Val Thr Ile Ser Cys Arg Ala Ser Gln
           35                  40                  45
   Asp Ile Ser Lys Tyr Leu Asn Trp Tyr Gln Gln Lys Pro Asp Gly Thr
       50                  55                  60
   Val Lys Leu Leu Ile Tyr His Thr Ser Arg Leu His Ser Gly Val Pro
   65                  70                  75                  80
   Ser Arg Phe Ser Gly Ser Gly Ser Gly Thr Asp Tyr Ser Leu Thr Ile
                   85                  90                  95
   Ser Asn Leu Glu Gln Glu Asp Ile Ala Thr Tyr Phe Cys Gln Gln Gly
               100                 105                 110
   Asn Thr Leu Pro Tyr Thr Phe Gly Gly Gly Thr Lys Leu Glu Ile Thr
           115                 120                 125
   Arg Ala Asp Ala Ala Pro Thr Val Ser Ile Phe Pro Pro Gly Gly Gly
   130                 135                 140
   Gly Ser Gly Gly Gly Gly Ser Gly Gly Gly Gly Ser Glu Val Lys Leu
   145                 150                 155                 160
   Gln Glu Ser Gly Pro Gly Leu Val Ala Pro Ser Gln Ser Leu Ser Val
                   165                 170                 175
   Thr Cys Thr Val Ser Gly Val Ser Leu Pro Asp Tyr Gly Val Ser Trp
               180                 185                 190
   Ile Arg Gln Pro Pro Arg Lys Gly Leu Glu Trp Leu Gly Val Ile Trp
           195                 200                 205
   Gly Ser Glu Thr Thr Tyr Tyr Asn Ser Ala Leu Lys Ser Arg Leu Thr
       210                 215                 220
   Ile Ile Lys Asp Asn Ser Lys Ser Gln Val Phe Leu Lys Met Asn Ser
   225                 230                 235                 240
   Leu Gln Thr Asp Asp Thr Ala Ile Tyr Tyr Cys Ala Lys His Tyr Tyr
                   245                 250                 255
   Tyr Gly Gly Ser Tyr Ala Met Asp Tyr Trp Gly Gln Gly Thr Ser Val
               260                 265                 270
   Thr Val Ser Ser Thr Thr Thr Pro Ala Pro Arg Pro Pro Thr Pro Ala
           275                 280                 285
   Pro Thr Ile Ala Ser Gln Pro Leu Ser Leu Arg Pro Glu Ala Cys Arg
       290                 295                 300
   Pro Ala Ala Gly Gly Ala Val His Thr Arg Gly Leu Asp Phe Ala Cys
   305                 310                 315                 320
   Asp Ile Tyr Ile Trp Ala Pro Leu Ala Gly Thr Cys Gly Val Leu Leu
                   325                 330                 335
   Leu Ser Leu Val Ile Thr Leu Tyr Cys Lys Arg Gly Arg Lys Lys Leu
               340                 345                 350
   Leu Tyr Ile Phe Lys Gln Pro Phe Met Arg Pro Val Gln Thr Thr Gln
           355                 360                 365
   Glu Glu Asp Gly Cys Ser Cys Arg Phe Pro Glu Glu Glu Glu Gly Gly
       370                 375                 380
   Cys Glu Leu Arg Val Lys Phe Ser Arg Ser Ala Asp Ala Pro Ala Tyr
   385                 390                 395                 400
   Gln Gln Gly Gln Asn Gln Leu Tyr Asn Glu Leu Asn Leu Gly Arg Arg
                   405                 410                 415
   Glu Glu Tyr Asp Val Leu Asp Lys Arg Arg Gly Arg Asp Pro Glu Met
               420                 425                 430
   Gly Gly Lys Pro Arg Arg Lys Asn Pro Gln Glu Gly Leu Tyr Asn Glu
           435                 440                 445
   Leu Gln Lys Asp Lys Met Ala Glu Ala Tyr Ser Glu Ile Gly Met Lys
       450                 455                 460
   Gly Glu Arg Arg Arg Gly Lys Gly His Asp Gly Leu Tyr Gln Gly Leu
   465                 470                 475                 480
   Ser Thr Ala Thr Lys Asp Thr Tyr Asp Ala Leu His Met Gln Ala Leu
                   485                 490                 495
   Pro Pro Arg

<210>  33
<211>  1461
<212>  DNA
<213>  Artificial Sequence

<220>
<221>  gene
<223>  CD19-CAR nucleotide sequence of the positive control CTL109

<400>  33
atggccttac cagtgaccgc cttgctcctg ccgctggcct tgctgctcca cgccgccagg      60
ccggacatcc agatgacaca gactacatcc tccctgtctg cctctctggg agacagagtc     120

accatcagtt gcagggcaag tcaggacatt agtaaatatt taaattggta tcagcagaaa 180
ccagatggaa ctgttaaact cctgatctac catacatcaa gattacactc aggagtccca 240
tcaaggttca gtggcagtgg gtctggaaca gattattctc tcaccattag caacctggag 300
caagaagata ttgccactta cttttgccaa cagggtaata cgcttccgta cacgttcgga 360
ggggggacca agctggagat cacaggtggc ggtggctcgg gcggtggtgg gtcgggtggc 420
ggcggatctg aggtgaaact gcaggagtca ggacctggcc tggtggcgcc ctcacagagc 480
ctgtccgtca catgcactgt ctcaggggtc tcattacccg actatggtgt aagctggatt 540
cgccagcctc cacgaaaggg tctggagtgg ctgggagtaa tatggggtag tgaaaccaca 600
tactataatt cagctctcaa atccagactg accatcatca aggacaactc caagagccaa 660
gttttcttaa aaatgaacag tctgcaaact gatgacacag ccatttacta ctgtgccaaa 720
cattattact acggtggtag ctatgctatg gactactggg gccaaggaac ctcagtcacc 780
gtctcctcaa ccacgacgcc agcgccgcga ccaccaacac cggcgcccac catcgcgtcg 840
cagcccctgt ccctgcgccc agaggcgtgc cggccagcgg cggggggcgc agtgcacacg 900
agggggctgg acttcgcctg tgatatctac atctgggcgc ccttggccgg gacttgtggg 960
gtccttctcc tgtcactggt tatcaccctt tactgcaaac ggggcagaaa gaaactcctg 1020
tatatattca aacaaccatt tatgagacca gtacaaacta ctcaagagga agatggctgt 1080
agctgccgat ttccagaaga agaagaagga ggatgtgaac tgagagtgaa gttcagcagg 1140
agcgcagacg cccccgcgta caagcagggc cagaaccagc tctataacga gctcaatcta 1200
ggacgaagag aggagtacga tgttttggac aagagacgtg gccgggaccc tgagatgggg 1260
ggaaagccga gaaggaagaa ccctcaggaa ggcctgtaca atgaactgca gaaagataag 1320
atggcggagg cctacagtga gattgggatg aaaggcgagc gccggagggg caaggggcac 1380
gatggccttt accagggtct cagtacagcc accaaggaca cctacgacgc ccttcacatg 1440
caggccctgc cccctcgcta a 1461

<210> 34
<211> 486
<212> PRT
<213> Artificial Sequence

<220>
<221> peptide
<223> CD19-CAR amino acid sequence of the positive control CTL109


<400> 34
Met Ala Leu Pro Val Thr Ala Leu Leu Leu Pro Leu Ala Leu Leu Leu
1               5                   10                  15
His Ala Ala Arg Pro Asp Ile Gln Met Thr Gln Thr Thr Ser Ser Leu
            20                  25                  30
Ser Ala Ser Leu Gly Asp Arg Val Thr Ile Ser Cys Arg Ala Ser Gln
        35                  40                  45
Asp Ile Ser Lys Tyr Leu Asn Trp Tyr Gln Gln Lys Pro Asp Gly Thr
    50                  55                  60
Val Lys Leu Leu Ile Tyr His Thr Ser Arg Leu His Ser Gly Val Pro
65                  70                  75                  80
Ser Arg Phe Ser Gly Ser Gly Ser Gly Thr Asp Tyr Ser Leu Thr Ile
                85                  90                  95
Ser Asn Leu Glu Gln Glu Asp Ile Ala Thr Tyr Phe Cys Gln Gln Gly
            100                 105                 110
Asn Thr Leu Pro Tyr Thr Phe Gly Gly Gly Thr Lys Leu Glu Ile Thr
        115                 120                 125
Gly Gly Gly Gly Ser Gly Gly Gly Gly Ser Gly Gly Gly Gly Ser Glu
    130                 135                 140
Val Lys Leu Gln Glu Ser Gly Pro Gly Leu Val Ala Pro Ser Gln Ser
145                 150                 155                 160
Leu Ser Val Thr Cys Thr Val Ser Gly Val Ser Leu Pro Asp Tyr Gly
                165                 170                 175
Val Ser Trp Ile Arg Gln Pro Pro Arg Lys Gly Leu Glu Trp Leu Gly
            180                 185                 190
Val Ile Trp Gly Ser Glu Thr Thr Tyr Tyr Asn Ser Ala Leu Lys Ser
        195                 200                 205

Arg Leu Thr Ile Ile Lys Asp Asn Ser Lys Ser Gln Val Phe Leu Lys
    210                 215                 220
Met Asn Ser Leu Gln Thr Asp Asp Thr Ala Ile Tyr Tyr Cys Ala Lys
225                 230                 235                 240
His Tyr Tyr Tyr Gly Gly Ser Tyr Ala Met Asp Tyr Trp Gly Gln Gly
                245                 250                 255
Thr Ser Val Thr Val Ser Ser Thr Thr Thr Pro Ala Pro Arg Pro Pro
            260                 265                 270
Thr Pro Ala Pro Thr Ile Ala Ser Gln Pro Leu Ser Leu Arg Pro Glu
        275                 280                 285
Ala Cys Arg Pro Ala Ala Gly Gly Ala Val His Thr Arg Gly Leu Asp
    290                 295                 300
Phe Ala Cys Asp Ile Tyr Ile Trp Ala Pro Leu Ala Gly Thr Cys Gly
305                 310                 315                 320
Val Leu Leu Leu Ser Leu Val Ile Thr Leu Tyr Cys Lys Arg Gly Arg
                325                 330                 335
Lys Lys Leu Leu Tyr Ile Phe Lys Gln Pro Phe Met Arg Pro Val Gln
            340                 345                 350
Thr Thr Gln Glu Glu Asp Gly Cys Ser Cys Arg Phe Pro Glu Glu Glu
        355                 360                 365
Glu Gly Gly Cys Glu Leu Arg Val Lys Phe Ser Arg Ser Ala Asp Ala
    370                 375                 380
Pro Ala Tyr Lys Gln Gly Gln Asn Gln Leu Tyr Asn Glu Leu Asn Leu
385                 390                 395                 400
Gly Arg Arg Glu Glu Tyr Asp Val Leu Asp Lys Arg Arg Gly Arg Asp
                405                 410                 415
Pro Glu Met Gly Gly Lys Pro Arg Arg Lys Asn Pro Gln Glu Gly Leu
            420                 425                 430
Tyr Asn Glu Leu Gln Lys Asp Lys Met Ala Glu Ala Tyr Ser Glu Ile
        435                 440                 445
Gly Met Lys Gly Glu Arg Arg Arg Gly Lys Gly His Asp Gly Leu Tyr
    450                 455                 460
Gln Gly Leu Ser Thr Ala Thr Lys Asp Thr Tyr Asp Ala Leu His Met
465                 470                 475                 480
Gln Ala Leu Pro Pro Arg
                485

<210>  35
<211>  2019
<212>  DNA
<213>  Artificial Sequence

<220>
<221>  gene
<223>  Nucleotide sequence of MSN-CAR (SS1CAR)


<400>  35
atggccttac cagtgaccgc cttgctcctg ccgctggcct tgctgctcca cgccgccagg      60
ccgcaggtac aactgcagca gtctgggcct gagctggaga agcctggcgc ttcagtgaag     120
atatcctgca aggcttctgg ttactcattc actggctaca ccatgaactg ggtgaagcag     180
agccatggaa agagccttga gtggattgga cttattactc cttacaatgg tgcttctagc     240
tacaaccaga agttcagggg caaggccaca ttaactgtag acaagtcatc cagcacagcc     300
tacatggacc tcctcagtct gacatctgaa gactctgcag tctatttctg tgcaaggggg     360
ggttacgacg ggagggggtt tgactactgg ggccaaggga ccacggtcac cgtctcctca     420
ggtggaggcg gttcaggcgg cggtggctct agcggtggtg gatcggacat cgagctcact     480
cagtctccag caatcatgtc tgcatctcca ggggagaagg tcaccatgac ctgcagtgcc     540
agctcaagtg taagttacat gcactggtac cagcagaagt caggcacctc ccccaaaaga     600
tggatttatg acacatccaa actggcttct ggagtcccag gtcgcttcag tggcagtggg     660
tctggaaact cttactctct cacaatcagc agcgtggagg ctgaagatga tgcaacttat     720
tactgccagc agtggagtaa gcaccctctc acgtacggtg ctgggacaaa gttggaaatc     780

aaaagcagcg agagcaagta cggccctccc tgcccccctt gccctgcccc cgagttcctg 840
ggcggaccca gcgtgttcct gttccccccc aagcccaagg acaccctgat gatcagccgg 900
acccccgagg tgacctgtgt ggtggtggac gtgtcccagg aggaccccga ggtccagttc 960
aactggtacg tggacggcgt ggaggtgcac aacgccaaga ccaagccccg ggaggagcag 1020
ttcaatagca cctaccgggt ggtgtccgtg ctgaccgtgc tgcaccagga ctggctgaac 1080
ggcaaggaat acaagtgtaa ggtgtccaac aagggcctgc ccagcagcat cgagaaaacc 1140
atcagcaagg ccaagggcca gcctcgggag ccccaggtgt acaccctgcc ccctagccaa 1200
gaggagatga ccaagaacca ggtgtccctg acctgcctgg tgaagggctt ctaccccagc 1260
gacatcgccg tggagtggga gagcaacggc cagcccgaga acaactacaa gaccaccccc 1320
cctgtgctgg acagcgacgg cagcttcttc ctgtacagcc ggctgaccgt ggacaagagc 1380
cggtggcagg agggcaacgt ctttagctgc tccgtgatgc acgaggccct gcacaaccac 1440
tacacccaga agagcctgag cctgtccctg ggcaagatgt tctgggtgct ggtcgttgtg 1500
ggcggcgtgc tggcctgcta cagcctgctg gtgacagtgg ccttcatcat cttttgggtg 1560
aggagcaagc ggagcagact gctgcacagc gactacatga acatgacccc ccggaggcct 1620
ggccccaccc ggaagcacta ccagccctac gcccctccca gggatttcgc cgcctaccgg 1680
agccgggtga agttcagccg gagcgccgac gcccctgcct accagcaggg ccagaaccag 1740
ctgtacaacg agctgaacct gggccggagg gaggagtacg acgtgctgga caagcggaga 1800
ggccgggacc ctgagatggg cggcaagccc cggagaaaga acccccagga gggcctgtat 1860
aacgaactgc agaaagacaa gatggccgag gcctacagcg agatcggcat gaagggcgag 1920
cggaggcggg gcaagggcca cgacggcctg taccagggcc tgagcaccgc caccaaggat 1980
acctacgacg ccctgcacat gcaggccctg ccccccaga 2019

<210> 36
<211> 673
<212> PRT
<213> Artificial Sequence

<220>
<221> peptide
<223> Amino acid sequence of MSN-CAR (SS1CAR)

<400> 36
Met Ala Leu Pro Val Thr Ala Leu Leu Leu Pro Leu Ala Leu Leu Leu
1               5                   10                  15
His Ala Ala Arg Pro Gln Val Gln Leu Gln Gln Ser Gly Pro Glu Leu
            20                  25                  30
Glu Lys Pro Gly Ala Ser Val Lys Ile Ser Cys Lys Ala Ser Gly Tyr
        35                  40                  45
Ser Phe Thr Gly Tyr Thr Met Asn Trp Val Lys Gln Ser His Gly Lys
    50                  55                  60
Ser Leu Glu Trp Ile Gly Leu Ile Thr Pro Tyr Asn Gly Ala Ser Ser
65                  70                  75                  80
Tyr Asn Gln Lys Phe Arg Gly Lys Ala Thr Leu Thr Val Asp Lys Ser
                85                  90                  95
Ser Ser Thr Ala Tyr Met Asp Leu Leu Ser Leu Thr Ser Glu Asp Ser
            100                 105                 110
Ala Val Tyr Phe Cys Ala Arg Gly Gly Tyr Asp Gly Arg Gly Phe Asp
        115                 120                 125
Tyr Trp Gly Gln Gly Thr Thr Val Thr Val Ser Ser Gly Gly Gly Gly
    130                 135                 140
Ser Gly Gly Gly Gly Ser Ser Gly Gly Gly Ser Asp Ile Glu Leu Thr
145                 150                 155                 160
Gln Ser Pro Ala Ile Met Ser Ala Ser Pro Gly Glu Lys Val Thr Met
                165                 170                 175
Thr Cys Ser Ala Ser Ser Ser Val Ser Tyr Met His Trp Tyr Gln Gln
            180                 185                 190
Lys Ser Gly Thr Ser Pro Lys Arg Trp Ile Tyr Asp Thr Ser Lys Leu
        195                 200                 205
Ala Ser Gly Val Pro Gly Arg Phe Ser Gly Ser Gly Ser Gly Asn Ser
    210                 215                 220

Tyr Ser Leu Thr Ile Ser Ser Val Glu Ala Glu Asp Asp Ala Thr Tyr
225                 230                 235                 240
Tyr Cys Gln Gln Trp Ser Lys His Pro Leu Thr Tyr Gly Ala Gly Thr
                245                 250                 255
Lys Leu Glu Ile Lys Ser Ser Glu Ser Lys Tyr Gly Pro Pro Cys Pro
            260                 265                 270
Pro Cys Pro Ala Pro Glu Phe Leu Gly Gly Pro Ser Val Phe Leu Phe
        275                 280                 285
Pro Pro Lys Pro Lys Asp Thr Leu Met Ile Ser Arg Thr Pro Glu Val
    290                 295                 300
Thr Cys Val Val Val Asp Val Ser Gln Glu Asp Pro Glu Val Gln Phe
305                 310                 315                 320
Asn Trp Tyr Val Asp Gly Val Glu Val His Asn Ala Lys Thr Lys Pro
                325                 330                 335
Arg Glu Glu Gln Phe Asn Ser Thr Tyr Arg Val Val Ser Val Leu Thr
            340                 345                 350
Val Leu His Gln Asp Trp Leu Asn Gly Lys Glu Tyr Lys Cys Lys Val
        355                 360                 365
Ser Asn Lys Gly Leu Pro Ser Ser Ile Glu Lys Thr Ile Ser Lys Ala
    370                 375                 380
Lys Gly Gln Pro Arg Glu Pro Gln Val Tyr Thr Leu Pro Pro Ser Gln
385                 390                 395                 400
Glu Glu Met Thr Lys Asn Gln Val Ser Leu Thr Cys Leu Val Lys Gly
                405                 410                 415
Phe Tyr Pro Ser Asp Ile Ala Val Glu Trp Glu Ser Asn Gly Gln Pro
            420                 425                 430
Glu Asn Asn Tyr Lys Thr Thr Pro Pro Val Leu Asp Ser Asp Gly Ser
        435                 440                 445
Phe Phe Leu Tyr Ser Arg Leu Thr Val Asp Lys Ser Arg Trp Gln Glu
    450                 455                 460
Gly Asn Val Phe Ser Cys Ser Val Met His Glu Ala Leu His Asn His
465                 470                 475                 480
Tyr Thr Gln Lys Ser Leu Ser Leu Ser Leu Gly Lys Met Phe Trp Val
                485                 490                 495
Leu Val Val Val Gly Gly Val Leu Ala Cys Tyr Ser Leu Leu Val Thr
            500                 505                 510
Val Ala Phe Ile Ile Phe Trp Val Arg Ser Lys Arg Ser Arg Leu Leu
        515                 520                 525
His Ser Asp Tyr Met Asn Met Thr Pro Arg Arg Pro Gly Pro Thr Arg
    530                 535                 540
Lys His Tyr Gln Pro Tyr Ala Pro Pro Arg Asp Phe Ala Ala Tyr Arg
545                 550                 555                 560
Ser Arg Val Lys Phe Ser Arg Ser Ala Asp Ala Pro Ala Tyr Gln Gln
                565                 570                 575
Gly Gln Asn Gln Leu Tyr Asn Glu Leu Asn Leu Gly Arg Arg Glu Glu
            580                 585                 590
Tyr Asp Val Leu Asp Lys Arg Arg Gly Arg Asp Pro Glu Met Gly Gly
        595                 600                 605
Lys Pro Arg Arg Lys Asn Pro Gln Glu Gly Leu Tyr Asn Glu Leu Gln
    610                 615                 620
Lys Asp Lys Met Ala Glu Ala Tyr Ser Glu Ile Gly Met Lys Gly Glu
625                 630                 635                 640
Arg Arg Arg Gly Lys Gly His Asp Gly Leu Tyr Gln Gly Leu Ser Thr
                645                 650                 655
Ala Thr Lys Asp Thr Tyr Asp Ala Leu His Met Gln Ala Leu Pro Pro
            660                 665                 670
Arg

<210>  37
<211>  18

<212>  RNA
<213>  Artificial Sequence

<220>
<221>  unsure
<223>  TRAC crRNA-1


<400>  37
caaauguguc acaaagua                                                    18

<210>  38
<211>  18
<212>  RNA
<213>  Artificial Sequence

<220>
<221>  unsure
<223>  TRAC crRNA-2


<400>  38
aaaacugugc uagacaug                                                    18

<210>  39
<211>  18
<212>  RNA
<213>  Artificial Sequence

<220>
<221>  unsure
<223>  TRAC crRNA-3


<400>  39
ucaagagcaa cagugcug                                                    18

<210>  40
<211>  18
<212>  RNA
<213>  Artificial Sequence

<220>
<221>  unsure
<223>  TRAC crRNA-4


<400>  40
caccuucuuc cccagccc                                                    18

<210>  41
<211>  18
<212>  RNA
<213>  Artificial Sequence

<220>
<221>  unsure
<223>  TARC crRNA-5


<400>  41

gaauaaugcu guuguuga                                                    18

<210>  42
<211>  18
<212>  RNA
<213>  Artificial Sequence

<220>
<221>  unsure
<223>  TRAC crRNA-6


<400>  42
gauuuagagu cucucagc                                                    18

<210>  43
<211>  18
<212>  RNA
<213>  Artificial Sequence

<220>
<221>  unsure
<223>  TRAC crRNA-7


<400>  43
acggcagggu caggguuc                                                    18

<210>  44
<211>  18
<212>  RNA
<213>  Artificial Sequence

<220>
<221>  unsure
<223>  TRAC crRNA-8


<400>  44
guuccuguga ugucaagc                                                    18

<210>  45
<211>  18
<212>  RNA
<213>  Artificial Sequence

<220>
<221>  unsure
<223>  TARC crRNA-9


<400>  45
ucaaaaccug ucagugau                                                    18

<210>  46
<211>  18
<212>  RNA
<213>  Artificial Sequence

<220>
<221>  unsure

<223>  TRAC crRNA-10

<400>  46
gaauccuccu ccugaaag                                                    18

<210>  47
<211>  17
<212>  RNA
<213>  Artificial Sequence

<220>
<221>  unsure
<223>  TRAC crRNA-11

<400>  47
gguacacggc aggguca                                                     17

<210>  48
<211>  20
<212>  RNA
<213>  Artificial Sequence

<220>
<221>  unsure
<223>  TRAC crRNA-12

<400>  48
gagaaucaaa aucggugaau                                                  20

<210>  49
<211>  18
<212>  RNA
<213>  Artificial Sequence

<220>
<221>  unsure
<223>  B2M crRNA-13

<400>  49
guagcgcgag cacagcua                                                    18

<210>  50
<211>  20
<212>  RNA
<213>  Artificial Sequence

<220>
<221>  unsure
<223>  PD-1 crRNA-14

<400>  50
cgacuggcca gggcgccugu                                                  20

<210>  51
<211>  18
<212>  RNA

<213>  Artificial Sequence

<220>
<221>  unsure
<223>  PD-1 crRNA-15


<400>  51
gugcuacaac ugggcugg                                                    18

<210>  52
<211>  20
<212>  RNA
<213>  Artificial Sequence

<220>
<221>  unsure
<223>  PD-1 crRNA-16


<400>  52
ggcgcccugg ccagucgucu                                                  20

<210>  53
<211>  80
<212>  RNA
<213>  Artificial Sequence

<220>
<221>  unsure
<223>  tracrRNA sequence corresponding to Cas9 protein


<400>  53
guuuuagagc uagaaauagc aaguuaaaau aaggcuaguc cguuaucaac uugaaaaagu      60
ggcaccgagu cggugcuuuu                                                  80

<210>  54
<211>  1378
<212>  PRT
<213>  Artificial Sequence

<220>
<221>  peptide
<223>  Amino acid sequence of Cas9 protein (including NLS)


<400>  54
Met Asp Lys Lys Tyr Ser Ile Gly Leu Ala Ile Gly Thr Asn Ser Val
1               5                   10                  15
Gly Trp Ala Val Ile Thr Asp Glu Tyr Lys Val Pro Ser Lys Lys Phe
            20                  25                  30
Lys Val Leu Gly Asn Thr Asp Arg His Ser Ile Lys Lys Asn Leu Ile
        35                  40                  45
Gly Ala Leu Leu Phe Asp Ser Gly Glu Thr Ala Glu Ala Thr Arg Leu
    50                  55                  60
Lys Arg Thr Ala Arg Arg Arg Tyr Thr Arg Arg Lys Asn Arg Ile Cys
65                  70                  75                  80
Tyr Leu Gln Glu Ile Phe Ser Asn Glu Met Ala Lys Val Asp Asp Ser
                85                  90                  95
Phe Phe His Arg Leu Glu Glu Ser Phe Leu Val Glu Glu Asp Lys Lys

                100                 105                 110
His Glu Arg His Pro Ile Phe Gly Asn Ile Val Asp Glu Val Ala Tyr
            115                 120                 125
His Glu Lys Tyr Pro Thr Ile Tyr His Leu Arg Lys Lys Leu Val Asp
        130                 135                 140
Ser Thr Asp Lys Ala Asp Leu Arg Leu Ile Tyr Leu Ala Leu Ala His
145                 150                 155                 160
Met Ile Lys Phe Arg Gly His Phe Leu Ile Glu Gly Asp Leu Asn Pro
                165                 170                 175
Asp Asn Ser Asp Val Asp Lys Leu Phe Ile Gln Leu Val Gln Thr Tyr
            180                 185                 190
Asn Gln Leu Phe Glu Glu Asn Pro Ile Asn Ala Ser Gly Val Asp Ala
        195                 200                 205
Lys Ala Ile Leu Ser Ala Arg Leu Ser Lys Ser Arg Arg Leu Glu Asn
    210                 215                 220
Leu Ile Ala Gln Leu Pro Gly Glu Lys Lys Asn Gly Leu Phe Gly Asn
225                 230                 235                 240
Leu Ile Ala Leu Ser Leu Gly Leu Thr Pro Asn Phe Lys Ser Asn Phe
                245                 250                 255
Asp Leu Ala Glu Asp Ala Lys Leu Gln Leu Ser Lys Asp Thr Tyr Asp
            260                 265                 270
Asp Asp Leu Asp Asn Leu Leu Ala Gln Ile Gly Asp Gln Tyr Ala Asp
        275                 280                 285
Leu Phe Leu Ala Ala Lys Asn Leu Ser Asp Ala Ile Leu Leu Ser Asp
    290                 295                 300
Ile Leu Arg Val Asn Thr Glu Ile Thr Lys Ala Pro Leu Ser Ala Ser
305                 310                 315                 320
Met Ile Lys Arg Tyr Asp Glu His His Gln Asp Leu Thr Leu Leu Lys
                325                 330                 335
Ala Leu Val Arg Gln Gln Leu Pro Glu Lys Tyr Lys Glu Ile Phe Phe
            340                 345                 350
Asp Gln Ser Lys Asn Gly Tyr Ala Gly Tyr Ile Asp Gly Gly Ala Ser
        355                 360                 365
Gln Glu Glu Phe Tyr Lys Phe Ile Lys Pro Ile Leu Glu Lys Met Asp
    370                 375                 380
Gly Thr Glu Glu Leu Leu Val Lys Leu Asn Arg Glu Asp Leu Leu Arg
385                 390                 395                 400
Lys Gln Arg Thr Phe Asp Asn Gly Ser Ile Pro His Gln Ile His Leu
                405                 410                 415
Gly Glu Leu His Ala Ile Leu Arg Arg Gln Glu Asp Phe Tyr Pro Phe
            420                 425                 430
Leu Lys Asp Asn Arg Glu Lys Ile Glu Lys Ile Leu Thr Phe Arg Ile
        435                 440                 445
Pro Tyr Tyr Val Gly Pro Leu Ala Arg Gly Asn Ser Arg Phe Ala Trp
    450                 455                 460
Met Thr Arg Lys Ser Glu Glu Thr Ile Thr Pro Trp Asn Phe Glu Glu
465                 470                 475                 480
Val Val Asp Lys Gly Ala Ser Ala Gln Ser Phe Ile Glu Arg Met Thr
                485                 490                 495
Asn Phe Asp Lys Asn Leu Pro Asn Glu Lys Val Leu Pro Lys His Ser
            500                 505                 510
Leu Leu Tyr Glu Tyr Phe Thr Val Tyr Asn Glu Leu Thr Lys Val Lys
        515                 520                 525
Tyr Val Thr Glu Gly Met Arg Lys Pro Ala Phe Leu Ser Gly Glu Gln
    530                 535                 540
Lys Lys Ala Ile Val Asp Leu Leu Phe Lys Thr Asn Arg Lys Val Thr
545                 550                 555                 560
Val Lys Gln Leu Lys Glu Asp Tyr Phe Lys Lys Ile Glu Cys Phe Asp
                565                 570                 575
Ser Val Glu Ile Ser Gly Val Glu Asp Arg Phe Asn Ala Ser Leu Gly
            580                 585                 590

Thr Tyr His Asp Leu Leu Lys Ile Ile Lys Asp Lys Asp Phe Leu Asp
        595                 600                 605
Asn Glu Glu Asn Glu Asp Ile Leu Glu Asp Ile Val Leu Thr Leu Thr
    610                 615                 620
Leu Phe Glu Asp Arg Glu Met Ile Glu Glu Arg Leu Lys Thr Tyr Ala
625                 630                 635                 640
His Leu Phe Asp Asp Lys Val Met Lys Gln Leu Lys Arg Arg Arg Tyr
                645                 650                 655
Thr Gly Trp Gly Arg Leu Ser Arg Lys Leu Ile Asn Gly Ile Arg Asp
            660                 665                 670
Lys Gln Ser Gly Lys Thr Ile Leu Asp Phe Leu Lys Ser Asp Gly Phe
        675                 680                 685
Ala Asn Arg Asn Phe Met Gln Leu Ile His Asp Asp Ser Leu Thr Phe
    690                 695                 700
Lys Glu Asp Ile Gln Lys Ala Gln Val Ser Gly Gln Gly Asp Ser Leu
705                 710                 715                 720
His Glu His Ile Ala Asn Leu Ala Gly Ser Pro Ala Ile Lys Lys Gly
                725                 730                 735
Ile Leu Gln Thr Val Lys Val Val Asp Glu Leu Val Lys Val Met Gly
            740                 745                 750
Arg His Lys Pro Glu Asn Ile Val Ile Glu Met Ala Arg Glu Asn Gln
        755                 760                 765
Thr Thr Gln Lys Gly Gln Lys Asn Ser Arg Glu Arg Met Lys Arg Ile
    770                 775                 780
Glu Glu Gly Ile Lys Glu Leu Gly Ser Gln Ile Leu Lys Glu His Pro
785                 790                 795                 800
Val Glu Asn Thr Gln Leu Gln Asn Glu Lys Leu Tyr Leu Tyr Tyr Leu
                805                 810                 815
Gln Asn Gly Arg Asp Met Tyr Val Asp Gln Glu Leu Asp Ile Asn Arg
            820                 825                 830
Leu Ser Asp Tyr Asp Val Asp His Ile Val Pro Gln Ser Phe Leu Lys
        835                 840                 845
Asp Asp Ser Ile Asp Asn Lys Val Leu Thr Arg Ser Asp Lys Asn Arg
    850                 855                 860
Gly Lys Ser Asp Asn Val Pro Ser Glu Glu Val Val Lys Lys Met Lys
865                 870                 875                 880
Asn Tyr Trp Arg Gln Leu Leu Asn Ala Lys Leu Ile Thr Gln Arg Lys
                885                 890                 895
Phe Asp Asn Leu Thr Lys Ala Glu Arg Gly Gly Leu Ser Glu Leu Asp
            900                 905                 910
Lys Ala Gly Phe Ile Lys Arg Gln Leu Val Glu Thr Arg Gln Ile Thr
        915                 920                 925
Lys His Val Ala Gln Ile Leu Asp Ser Arg Met Asn Thr Lys Tyr Asp
    930                 935                 940
Glu Asn Asp Lys Leu Ile Arg Glu Val Lys Val Ile Thr Leu Lys Ser
945                 950                 955                 960
Lys Leu Val Ser Asp Phe Arg Lys Asp Phe Gln Phe Tyr Lys Val Arg
                965                 970                 975
Glu Ile Asn Asn Tyr His His Ala His Asp Ala Tyr Leu Asn Ala Val
            980                 985                 990
Val Gly Thr Ala Leu Ile Lys Lys  Tyr Pro Lys Leu Glu  Ser Glu Phe
        995                 1000                1005
Val Tyr  Gly Asp Tyr Lys Val  Tyr Asp Val Arg Lys  Met Ile Ala Lys
    1010                1015                1020
Ser  Glu Gln Glu Ile Gly  Lys Ala Thr Ala Lys  Tyr Phe Phe Tyr Ser
1025                1030                1035                1040
Asn Ile Met Asn Phe  Phe Lys Thr Glu Ile  Thr Leu Ala Asn Gly  Glu
                1045                1050                1055
Ile Arg Lys Arg  Pro Leu Ile Glu Thr  Asn Gly Glu Thr Gly  Glu Ile
            1060                1065                1070
Val Trp Asp  Lys Gly Arg Asp Phe  Ala Thr Val Arg Lys  Val Leu Ser

1075                1080                1085
Met Pro Gln Val Asn Ile Val Lys Lys Thr Glu Val Gln Thr Gly Gly
    1090                1095                1100
Phe Ser Lys Glu Ser Ile Leu Pro Lys Arg Asn Ser Asp Lys Leu Ile
1105                1110                1115                1120
Ala Arg Lys Lys Asp Trp Asp Pro Lys Lys Tyr Gly Gly Phe Asp Ser
                1125                1130                1135
Pro Thr Val Ala Tyr Ser Val Leu Val Val Ala Lys Val Glu Lys Gly
            1140                1145                1150
Lys Ser Lys Lys Leu Lys Ser Val Lys Glu Leu Leu Gly Ile Thr Ile
        1155                1160                1165
Met Glu Arg Ser Ser Phe Glu Lys Asn Pro Ile Asp Phe Leu Glu Ala
    1170                1175                1180
Lys Gly Tyr Lys Glu Val Lys Lys Asp Leu Ile Ile Lys Leu Pro Lys
1185                1190                1195                1200
Tyr Ser Leu Phe Glu Leu Glu Asn Gly Arg Lys Arg Met Leu Ala Ser
                1205                1210                1215
Ala Gly Glu Leu Gln Lys Gly Asn Glu Leu Ala Leu Pro Ser Lys Tyr
            1220                1225                1230
Val Asn Phe Leu Tyr Leu Ala Ser His Tyr Glu Lys Leu Lys Gly Ser
        1235                1240                1245
Pro Glu Asp Asn Glu Gln Lys Gln Leu Phe Val Glu Gln His Lys His
    1250                1255                1260
Tyr Leu Asp Glu Ile Ile Glu Gln Ile Ser Glu Phe Ser Lys Arg Val
1265                1270                1275                1280
Ile Leu Ala Asp Ala Asn Leu Asp Lys Val Leu Ser Ala Tyr Asn Lys
                1285                1290                1295
His Arg Asp Lys Pro Ile Arg Glu Gln Ala Glu Asn Ile Ile His Leu
            1300                1305                1310
Phe Thr Leu Thr Asn Leu Gly Ala Pro Ala Ala Phe Lys Tyr Phe Asp
        1315                1320                1325
Thr Thr Ile Asp Arg Lys Arg Tyr Thr Ser Thr Lys Glu Val Leu Asp
    1330                1335                1340
Ala Thr Leu Ile His Gln Ser Ile Thr Gly Leu Tyr Glu Thr Arg Ile
1345                1350                1355                1360
Asp Leu Ser Gln Leu Gly Gly Asp Pro Lys Lys Lys Arg Lys Val Met
                1365                1370                1375
Asp Lys


<210>  55
<211>  127
<212>  DNA
<213>  Artificial Sequence

<220>
<221>  unsure
<223>  Exemplary N-oligo sequence


<400>  55
tcatgtggtc ggggtagcgg ctgaagcact gcacgccgta cgtcagggtg gtcacgaggg      60
tgggccaggg cacgggcagc ttgccggtgg tgcagatgaa cttcagggtc agcttgccgt     120
aggtggc                                                               127

<210>  56
<211>  336
<212>  DNA
<213>  Artificial Sequence

<220>

<221>  gene
<223>  Nucleotide sequence of CD3-zeta intracellular region

<400>  56
agagtgaagt tcagcaggag cgcagacgcc cccgcgtacc agcagggcca gaaccagctc      60
tataacgagc tcaatctagg acgaagagag gagtacgatg ttttggacaa gagacgtggc     120
cgggaccctg agatgggggg aaagccgaga aggaagaacc ctcaggaagg cctgtacaat     180
gaactgcaga aagataagat ggcggaggcc tacagtgaga ttgggatgaa aggcgagcgc     240
cggaggggca aggggcacga tggcctttac cagggtctca gtacagccac caaggacacc     300
tacgacgccc ttcacatgca ggccctgccc cctcgc                               346

<210>  57
<211>  112
<212>  PRT
<213>  Artificial Sequence

<220>
<221>  peptide
<223>  Amino acid sequence of CD3-zeta intracellular region

<400>  57
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110

## Description

### FIELD OF THE INVENTION

The present disclosure belongs to the field of biomedicine. In particular, the present disclosure relates to a chimeric antigen receptor, cells comprising the chimeric antigen receptor, and uses thereof.

### BACKGROUND OF THE INVENTION

The statements herein merely provide background information related to the present invention and do not necessarily constitute the prior art.

Lymphatic malignancies, including lymphocytic leukemia and lymphoma, are tumors that occur on lymphocytes such as B cells, T cells, and NK cells. At present, there are many difficulties in the treatment, especially for the recurrent and refractory diseases that are often encountered in clinical practice. In the past 10 years, great progress has been made in the clinical treatment of lymphatic tumors. Anti-CD20 monoclonal antibodies have been widely used in CD20-positive B cell non-Hodgkin's lymphoma, and have achieved good curative effect. They have become clinical first-line medication. However, since CD20 is not expressed on the cells of some B lymphoma, acute and chronic B lymphoblastic leukemia, anti-CD20 antibodies such as rituximab have no obvious therapeutic effect on them. There is an urgent need for a new therapy to improve the cure rate of lymphoma and acute and chronic lymphocytic leukemia.

Chimeric antigen receptor T cells (CAR-T or CART) are T lymphocytes expressing specific CAR through genetic modification, and such cells can specifically recognize the target antigen and kill the target cells. CAR-T cells have high affinity to specific tumor antigen, and thereby can efficiently kill the tumor cells expressing the antigen. CD 19 is specifically expressed on the surface of B lymphocytes at different stages of differentiation, and both B cell lymphoma and B lymphocyte leukemia express CD 19 antigen. Therefore, construction of chimeric antigen receptor CART cells recognizing CD 19 can achieve the purpose of effectively treating B lymphocytic tumors.

CD19-CART cells can recognize the specific CD19 target of B lymphocytic leukemia and attack the B lymphocytes expressing CD 19 antigen by releasing cytokines such as perforin and granzyme, thereby promote to eliminate malignant lymphocytes from the organism. Sloan-Kettering Cancer Center in the United States has applied autologous 19-28zCAR-T technology in the treatment of refractory and relapsed acute B-cell lymphocytic leukemia (B-ALL), 14 of 16 patients achieved complete remission (CR), and the therapeutic effect was also found in Philadelphia chromosome-positive acute lymphocytic leukemia (Ph+ALL). Treatment with CART also creates prerequisites for allogeneic hematopoietic stem cell transplantation. The University of Pennsylvania also reported the results of the treatment of B-cell tumors with 19-CD137zCART, 27 out of 30 cases of refractory and relapsed B-ALL achieved CR, the rate of 6-month disease-free survival was 67%, and the overall survival rate reached 78%. Currently, Novartis, co-working with the University of Pennsylvania, received a marketing approval of the first CART cell as therapeutic drug for relapsed/refractory ALL in children, and thereafter Kite acquired a marketing approval of the second CAR-T as drug for non-Hodgkin's lymphoma.

Currently the approved CART products are produced by autologous cells, and have disadvantages of: long production cycle, high production cost, and qualified CAR-T cells cannot be produced in some patients. Such disadvantages make this technology incapable of being widely applied in patients. The TCR gene on the surface of T cells was knocked out in universal CAR-T cells (UCARTs), thereby eliminating or greatly reducing the GvHD efficiency. In addition, B2M-knockout can reduce the rejection of host to allogeneic cells. Meanwhile, allogeneic UCARTs are characterized in being ready-to-use and capable of being re-injected to the patients at a fixed dose, which avoid such a situation wherein the patients' T cells are unable to be expanded or prepared in time. Further, the large-scale preparation can reduce the production cost and is suitable for large-scale applications.

WO2014186585A2 and WO2016057821A2 patent application relate to a method for knocking out endogenous genes; WO2009091826, WO2012079000A1, WO2015187528, WO2015158671, WO2016014789, WO2016014576, WO2017049166 and WO2017173349 relate to the preparation and application of CAR-T cells; WO2015136001, WO2015140268, WO2015158671, WO2015193406 and WO2017032777 relate to the preparation and application of UCARTs. However, currently only the UCARTs from Cellectis SA, Pfizer Inc. and Shanghai BIORAY Inc. are at clinical research, Phase I stage, and there is no commercially available UCART cell as therapeutic drug. Therefore, there is a need to continuously explore new UCART cell as therapeutic drug.

### SUMMARY OF THE INVENTION

The purpose of the present disclosure is to overcome the problems in immunotherapy of the prior art, and to provide a genetically modified T cell comprising a nucleic acid encoding a chimeric antigen receptor that binds to CD19, and the endogenous genes TARC and B2M are knocked out in genetically modified T cell by CRISPR/Cas9 gene editing technology. Furthermore, the present disclosure also provides a crRNA with novel sequence for knocking out the endogenous genes TARC, B2M and PD-1, and provides use of gene knockout T cells obtained according to the methods of the present disclosure in the treatment or prevention of CD19-mediated diseases.

Some embodiments of the present disclosure provide a TCR- and PD-1- or B2M-double negative T cell and a method of constructing the same.

Further embodiments of the present disclosure provide a TCR-, B2M- and PD-1- triple negative T cell and a method of constructing the same.

Further, the above-mentioned TCR-negative T cells, TCR- and PD-1- or B2M-double-negative T cells and TCR/B2M/PD-1 triple negative T cells are sorted by magnetic beads, and are used for adoptive cell immunotherapy of tumors and the like.

In some embodiments, provided is a method of knocking out one or more target genes in T cells in vitro, the method comprises the steps of:
1) sgRNA(s) targeting one or more target genes in the T cells is/are contacted respectively with Cas9 protein to form protein RNA complex(s) (RNP(s));
2) the RNP(s) is/are mixed with oligo-deoxyribonucleic acid (N-oligo) or fish sperm DNA fragment; and used to transform the T cells, wherein the sgRNA(s) direct the Cas9 protein to a target sequence of the corresponding target gene and to hybridize with the target sequence, wherein the target gene is cleaved, and wherein the cleavage efficiency of the target gene is greater than 75%.

In some embodiments, the target gene is one or more or any combination selected from the group consisting of TRAC, TRBC, B2M and PD1 genes, and the sgRNA(s) target the coding sequence or the expression-regulating sequence of the target gene.

Further, the sgRNA(s) is/are formed by linking, from 5' to 3', a crRNA to a tracrRNA corresponding to the Cas9 protein, the crRNA targets the endogenous gene(s) and is 17 nt, 18 nt, 19 nt or 20 nt in length, preferably the crRNA is 17nt in length.

In some embodiments, the oligodeoxyribonucleic acid is a double-stranded DNA with 100 bp, 250 bp, or any between 100bp to 250 bp in length, or a single-stranded DNA with 100 nt, 250 nt, or any between 100nt to 250 nt in length. Preferably, the sequence of the oligodeoxyribonucleic acid is shown in SEQ ID NO: 55.

In some embodiments, the crRNA(s) that target the TRAC gene is (are) any one or more crRNAs selected from the group consisting of SEQ ID NOs: 37, 38, 39, 40, 41, 42, 43, 44, 45, 46, 47 and 48, preferably the crRNA is shown in SEQ ID NO: 37; the sequence of crRNA that targets the B2M gene is shown in SEQ ID NO: 49; and the crRNA(s) that target the PD-1 gene is (are) any one or more selected from the group consisting of SEQ ID NOs: 50, 51 and 52, preferably the crRNA is shown in SEQ ID NO: 52.

In some embodiments, the Cas9 protein is *Streptococcus Pyogenes-derived* Cas9 protein, the amino acid sequence of which is shown in SEQ ID NO: 54, and the tracrRNA sequence corresponding to the Cas9 protein is shown in SEQ ID NO: 53.

In some embodiments, the T cell is selected from the group consisting of helper T cell, cytotoxic T cell, memory T cell, regulatory T cell, natural killer T cell, γδ T cell, CAR-T cell, and TCR-T cell.

In another aspect, the present disclosure also provides a T cell in which the target gene was knocked out, obtained according to the above method.

In another aspect, the disclosure also provides crRNAs for knocking out the TRAC gene, the crRNA targets the coding sequence or expression-regulating sequence of human TRAC gene and is selected from the group consisting of SEQ ID NOs: 37, 38, 39, 40, 41, 42, 43, 44, 45, 46, 47 and 48, preferably SEQ ID NO: 37.

In another aspect, the present disclosure also provides a crRNA for knocking out the B2M gene, the crRNA targets the coding sequence or expression-regulating sequence of human B2M gene and the sequence of the crRNA is shown in SEQ ID NO: 49.

In another aspect, the present disclosure also provides crRNAs for knocking out the PD-1 gene, the crRNA targets the coding sequence or expression-regulating sequence of human PD-1 gene and is selected from the group consisting of SEQ ID NOs: 50, 51 and 52, preferably SEQ ID NO: 52.

In another aspect, the present disclosure provides a kit for gene knockout, comprising sgRNA(s), Cas9 protein, and oligodeoxyribonucleic acid or fish sperm DNA, wherein the sgRNA(s) consist of the above-described one or more crRNAs separately linked to tracrRNA corresponding to the Cas9 protein.

In some embodiments of the kit for gene knockout, the oligodeoxyribonucleic acid is a double-stranded DNA with 100 bp, 250 bp, or any between 100bp to 250 bp in length, or a single-stranded DNA with 100 nt, 250 nt, or any between 100nt to 250 nt in length. Preferably, the sequence of the oligodeoxyribonucleic acid is shown in SEQ ID NO: 55.

In some embodiments of the kit for gene knockout, the Cas9 protein is Streptococcus Pyogenes-derived Cas9 protein, the amino acid sequence of which is shown in SEQ ID NO: 54, and the tracrRNA sequence corresponding to the Cas9 protein is shown in SEQ ID NO: 53.

In some embodiments, the disclosure provides use of the gene knockout T cells of the present disclosure in the preparation of an anti-tumor medicament.

In some embodiments, the present disclosure also provides use of the gene knockout T cells of the present disclosure in the preparation of a medicament for the prevention/treatment of infectious diseases caused by virus or bacteria.

In some embodiments, TCR, B2M or PD-1 are effectively knocked out using the designed crRNAs and method. The *in vitro* killing activity of TCR- and B2M- and/or PD-1-knockout CART cells is not affected by the gene knockout of TCR, B2M and/or PD-1.

The disclosure provides an isolated chimeric antigen receptor (CAR) comprising a CD19 antigen binding domain, a co-stimulatory signaling region, and a CD3ζ signaling domain, wherein the CD19 antigen binding domain comprises the amino acid sequence selected from the group consisting of SEQ ID NO: 18, SEQ ID NO: 20, SEQ ID NO: 22 and SEQ ID NO: 24, preferably comprises the amino acid sequence of SEQ ID NO: 20.

In some embodiments, the co-stimulatory signaling region comprises an intracellular domain of a co-stimulatory molecule selected from the group consisting of CD27, CD28, 4-1BB, OX40, CD30, CD40, PD-1, ICOS, lymphocyte function associated antigen-1 (LFA-1), CD2, CD7, LIGHT, NKG2C, B7-H3, and any combination thereof, preferably the 4-1BB co-stimulatory signaling region of SEQ ID NO: 12.

In some embodiments, the CD3ζ signaling domain comprises the amino acid sequence shown in SEQ ID NO: 14 or SEQ ID NO: 57.

In some embodiments, the CAR of the present disclosure further comprises an extracellular hinge domain, wherein the extracellular hinge domain comprises human CD8α leading signal region as shown in SEQ ID NO: 6 and human CD8α hinge region as shown in SEQ ID NO: 8.

In some embodiments, the CAR of the present disclosure further comprises CD8α transmembrane domain as shown in SEQ ID NO: 10.

In some embodiments, the CAR of the present disclosure comprises the amino acid sequence shown in SEQ ID NO:26, SEQ ID NO:28, SEQ ID NO:30 or SEQ ID NO:32, preferably SEQ ID NO:28.

The present disclosure further provides a series of nucleic acid molecules encoding the CARs as described above.

In some embodiments, the nucleic acid molecule comprises the nucleic acid sequence shown in SEQ ID NO: 17, SEQ ID NO: 19, SEQ ID NO: 21, or SEQ ID NO: 23.

In some embodiments, the nucleic acid molecule comprises a nucleic acid sequence encoding co-stimulatory signal transduction region and/or a nucleic acid sequence encoding CD3ζ signaling domain, preferably, the nucleic acid sequence encoding co-stimulatory signal transduction region is shown in SEQ ID NO: 11, and the nucleic acid sequence encoding CD3ζ signaling domain is shown in SEQ ID NO: 13 or SEQ ID NO: 56.

In some embodiments, the nucleic acid molecule further comprises a nucleic acid sequence encoding extracellular hinge domain, preferably the nucleic acid sequence encoding extracellular hinge domain comprises human CD8α leading signal region shown in SEQ ID NO: 5 and human CD8α hinge region shown in SEQ ID NO: 7.

In some embodiments, the nucleic acid molecule further comprises a CD8α transmembrane domain shown in SEQ ID NO: 9.

In some embodiments, the nucleic acid molecule of the present disclosure encodes a CAR, wherein the CAR comprises the amino acid sequence shown in SEQ ID NO: 26, SEQ ID NO: 28, SEQ ID NO: 30, or SEQ ID NO: 32, preferably, SEQ ID NO:28.

In one embodiment, the nucleic acid molecule of the present disclosure encodes a CAR, wherein the nucleic acid molecule comprises the nucleic acid sequence shown in SEQ ID NO: 25, SEQ ID NO: 27, SEQ ID NO: 29 or SEQ ID NO: 31, preferably SEQ ID NO: 27.

The present disclosure further provides a vector comprising a nucleic acid sequence encoding the above CAR.

In some embodiments, the vector described in the present disclosure is selected from the group consisting of DNA, RNA, plasmid, lentiviral vector, adenoviral vector, and retroviral vector, preferably lentiviral vector.

In some embodiments, the vector of the present disclosure further comprises a promoter, preferably comprises EF-1 promoter shown in SEQ ID NO: 4.

The disclosure further provides some T cells comprising a nucleic acid sequence encoding CAR.

The present disclosure further provides a method for generating T cells comprising a nucleic acid sequence encoding CAR, the method comprises the step of introducing a nucleic acid encoding chimeric antigen receptor (CAR) into the T cells.

The present disclosure further provides some compositions comprising one or more selected from the group consisting of:
(i) an isolated CAR as described above,
(ii) a nucleic acid molecule encoding the CAR as described above,
(iii) a vector comprising the nucleic acid molecule encoding the CAR as described above, and
(iv) modified T cells comprising the CAR as described above.

The present disclosure further provides some modified T cells comprising:
a nucleic acid capable of down-regulating gene expression of an endogenous gene, the endogenous gene is one or more or any combination selected from the group consisting of TRAC, B2M and PD-1; and
a nucleic acid encoding chimeric antigen receptor (CAR), the CAR comprises a CD19 antigen binding domain, a co-stimulatory signaling region and a CD3ζ signaling domain, wherein the CD19 antigen binding domain comprises the amino acid sequence shown in SEQ ID NO: 18, SEQ ID NO: 20, SEQ ID NO: 22 or SEQ ID NO: 24.

In some embodiments of the modified T cells of the present disclosure, the nucleic acid capable of down-regulating the expression of an endogenous gene in T cell is selected from the group consisting of antisense RNA, antigomer RNA, siRNA, shRNA, and CRISPR-Cas9 systems, preferably CRISPR-Cas9 system.

In some embodiments, the Cas9 protein is a Cas9 from *Streptococcus Pyogenes,* the amino acid sequence of which is shown in SEQ ID NO:54, and the corresponding tracrRNA sequence is shown in SEQ ID NO:55.

In some embodiments, the CRISPR-Cas9 system further comprises a sgRNA that targets the coding sequence of an endogenous gene or the expression-regulation sequence of the endogenous gene, wherein the sgRNA is/are formed by linking, from 5' to 3', a crRNA targeting the endogenous gene(s) with 17 nt, 18 nt, 19 nt or 20 nt in length to a tracrRNA corresponding to the Cas9 protein.

In one embodiment of the modified T cell of the present disclosure, the endogenous gene is selected from the group consisting of TRAC and B2M.

In some embodiments of the modified T cells of the present disclosure, the crRNA that targets the endogenous gene TRAC is any one or more crRNAs selected from the group consisting of SEQ ID NOs: 37, 38, 39, 40, 41, 42, 43, 44, 45, 46, 47 and 48, preferably SEQ ID NO: 47; the crRNA that targets the endogenous gene B2M is shown in SEQ ID NO: 49; the crRNA that targets the endogenous gene PD-1 is shown in SEQ ID NO: 50, 51 or 52, preferably SEQ ID NO: 52.

In some embodiments of the modified T cells of the present disclosure, the co-stimulatory signaling region is a 4-1BB co-stimulatory signaling region, the amino acid sequence of which is shown in SEQ ID NO: 12.

In some embodiments of the modified T cells of the present disclosure, the CD3ζ signaling domain comprises the amino acid sequence shown in SEQ ID NO: 14.

In some embodiments of the modified T cells of the present disclosure, the CAR further comprises an extracellular hinge domain, wherein the extracellular hinge domain comprises the human CD8α leading signal region shown in SEQ ID NO:6 and the human CD8α hinge region shown in SEQ ID NO: 8.

In some embodiments of the modified T cells of the present disclosure, the CAR further comprises the CD8α transmembrane domain shown in SEQ ID NO: 10.

In some embodiments of the modified T cells of the present disclosure, the CAR comprises the amino acid sequence shown in SEQ ID NO: 26, SEQ ID NO: 28, SEQ ID NO: 30 or SEQ ID NO: 32, preferably, comprises the amino acid sequence shown in SEQ ID NO: 28.

The disclosure further provides some modified T cells, comprising:
nucleic acids capable of down-regulating gene expression of endogenous gene TRAC and B2M in the T cells, wherein the crRNA which down-regulates endogenous gene TRAC is shown in SEQ ID NOs: 37, 38, 39, 40, 41, 42, 43, 44, 45, 46, 47 or 48, preferably SEQ ID NO: 47, the crRNA which down-regulates the endogenous gene B2M is shown in SEQ ID NO: 49, and the crRNA that targets the endogenous gene PD-1 is shown in SEQ ID NO: 50, 51 or 52, preferably SEQ ID NO: 52; and
a nucleic acid encoding chimeric antigen receptor (CAR), wherein the CAR comprises the amino acid sequence shown in SEQ ID NO: 26, SEQ ID NO: 28, SEQ ID NO: 30 or SEQ ID NO: 32, preferably comprises the amino acid sequence shown in SEQ ID NO: 28.

The present disclosure further provides some modified T cells, comprising:
nucleic acids capable of down-regulating gene expression of endogenous gene TRAC and B2M in the T cells, wherein the crRNA which down-regulates endogenous gene TRAC in the T cells is shown in SEQ ID NO: 47, the crRNA which down-regulates endogenous gene B2M is shown in SEQ ID NO: 49, the crRNA that down-regulates endogenous gene PD-1 is shown in SEQ ID NO: 52; and the chimeric antigen receptor comprises the amino acid sequence shown in SEQ ID NO: 26, SEQ ID NO: 28, SEQ ID NO: 30 or SEQ ID NO: 32, preferably comprises the amino acid sequence shown in SEQ ID NO: 28; most preferably the modified T cells are UCART19^{TCR-/-} (single knockout: TCR knockout) or UCART19^{TCR-1-B2M-/-} (double knockout: TCR and B2M knockout) or UCART19^{TCR-/-B2M-/-PD-1-/-} (triple knockout: TCR, B2M and PD-1 knockout).

The present disclosure further provides a pharmaceutical composition comprising the above modified T cells.

The present disclosure further provides a method for preparing the above modified T cells, comprising:
(1) introducing a nucleic acid encoding chimeric antigen receptor (CAR) into the T cells;
(2) introducing a nucleic acid of sgRNA into the T cells by CRISPR-Cas9 system, said sgRNA is capable of down-regulating the expression of an endogenous target gene in the T cells, the endogenous target gene is selected from the group consisting of TARC and B2M.

In some embodiments of the method for preparing modified T cells of the present disclosure, the CAR comprises the amino acid sequence shown in SEQ ID NO:26, SEQ ID NO:28, SEQ ID NO:30 or SEQ ID NO:32.

In some embodiments of the method for preparing modified T cells of the present disclosure, the crRNA that targets the endogenous gene TRAC is any one or more or any combination selected from the group consisting of SEQ ID NOs: 37, 38, 39, 40, 41, 42, 43, 44, 45, 46, 47 and 48, preferably SEQ ID NO: 47; the crRNA that targets endogenous gene B2M is shown in SEQ ID NO: 49.

In some embodiments of the method for preparing modified T cells, the T cells are obtained from peripheral blood mononuclear cells, cord blood cells, purified T cell population or T cell lines.

In some embodiments of the method for preparing modified T cells of the present disclosure, the method further comprises expanding the T cells.

In some embodiments of the method for preparing modified T cells of the present disclosure, the step of expanding the T cell comprises stimulating the expanded T cell population with at least one molecule or cytokine selected from the group consisting of CD3, CD27, CD28, CD83, CD86, CD127, 4-1BBL, IL2, IL21, IL-15, IL-7, PD1-CD28 and PD-1.

In some embodiments, the method for preparing modified T cells of the disclosure further comprises cryopreserving the T cells.

In some embodiments, the method for preparing modified T cells of the present disclosure further comprises the step of thawing the cryopreserved T cells and then introducing the nucleic acid into the T cells.

In some embodiments of the method for preparing modified T cells of the present disclosure, wherein introduction of the nucleic acid is selected from the group consisting of transduction of the expanded T cells, transfection of the expanded T cells, and electroporation of the expanded T cells.

In some embodiments, the pharmaceutical composition of the present disclosure further comprises a pharmaceutically acceptable carrier, diluent or excipient.

In some embodiments, the pharmaceutical composition of the present disclosure further comprises a buffer.

In some embodiments of the pharmaceutical composition of the present disclosure, the buffer is neutral buffered saline or phosphate buffered saline.

In some embodiments, the pharmaceutical composition of the present disclosure further comprises an injectable freezing medium.

In some embodiments of the pharmaceutical composition of the present disclosure, the injectable freezing medium comprises plasmalyte-A, dextrose, NaCl, DMSO, dextran, and human serum albumin.

In some embodiments, the pharmaceutical composition of the present disclosure further comprises one or more cytokines.

The present disclosure further provides the use of the nucleic acid molecule encoding CAR, the vector comprising the nucleic acid molecule encoding CAR, the T cells comprising CAR, the T cells comprising nucleic acid(s) capable of down-regulating gene expression of the endogenous gene TRAC and B2M and a nucleic acid encoding CAR, or the use of the composition comprising the above components in the manufacture of a medicament for the treatment or prevention of a CD19-mediated disease.

The present disclosure further provides the nucleic acid molecule encoding CAR, the vector comprising the nucleic acid molecule encoding CAR, the T cells comprising CAR, the T cells comprising nucleic acid(s) capable of down-regulating gene expression of the endogenous gene TRAC and B2M and a nucleic acid encoding CAR, or the composition comprising the above components, for use in the treatment or prevention of a CD19-mediated disease.

The present disclosure further provides a method of treating or preventing a CD19-mediated disease, the method comprises administering to a subject an effective amount of the nucleic acid molecule encoding CAR, the vector comprising the nucleic acid molecule encoding CAR, the T cells comprising CAR, the T cells comprising nucleic acid(s) capable of down-regulating gene expression of the endogenous gene TRAC and B2M and a nucleic acid encoding CAR, or the composition comprising the above components.

In other embodiments, the above method comprises administering to the subject effect dose of cells which have been genetically modified to express CAR or effect dose of cells comprising nucleic acid(s) capable of down-regulating gene expression of the endogenous gene TRAC and B2M and a nucleic acid encoding CAR, wherein the CAR comprises the amino acid sequence shown in SEQ ID NO: 26, SEQ ID NO: 28, SEQ ID NO: 30 or SEQ ID NO: 32, preferably comprises the amino acid sequence shown in SEQ ID NO: 28, wherein the crRNA that down-regulates the endogenous gene TRAC is any one or more any combination selected from the group consisting of SEQ ID NO: 37, 37, 38, 39, 40, 41, 42, 43, 44, 45, 46, 47 and 48, preferably SEQ ID NO: 47; wherein the crRNA that down-regulates the endogenous gene B2M is shown in SEQ ID NO: 49, and wherein the crRNA that targets the endogenous gene PD-1 is anyone or more selected from the group consisting of SEQ ID NO: 50, 51 and 52, preferably SEQ ID NO: 52.

In one embodiment, the CD19 mediated disease is selected from the group consisting of cancer, infectious disease caused by virus or bacteria, and autoimmune disease, preferably cancer, more preferably breast cancer, prostate cancer, ovarian cancer, cervical cancer, skin cancer, pancreatic cancer, colorectal cancer, kidney cancer, liver cancer, brain cancer, hematological cancer, lung cancer, and thyroid cancer, most preferred hematological cancer.

In one embodiment, the hematological cancer is selected from the group consisting of leukemia, including acute leukemia, such as acute lymphocytic leukemia, acute myelocytic leukemia, acute myelogenous leukemia and myeloblastic leukemia, promyelocytic leukemia, myelomonocytic leukemia, monocytic leukemia and erythroleukemia; and chronic leukemia, such as chronic myelocytic (granulocytic) leukemia, chronic myelogenous leukemia and chronic lymphocytic leukemia and refractory CD19⁺ leukemia and lymphoma; polycythemia vera, lymphoma, mantle cell lymphoma, diffuse large B-cell lymphoma, Hodgkin's disease, non-Hodgkin's lymphoma, multiple myeloma, Waldenstrom's macroglobulinaemia, heavy chain disease, myelodysplastic syndrome, hairy cell leukemia and myelodysplasia; preferably acute lymphocytic leukemia or chronic lymphocytic leukemia.

### DESCRIPTION OF THE DRAWINGS

Figure 1: Identification of the purity of the screened UCART19 cells.
Figure 2: Transfection efficiency of lentivirus.
Figure 3: Comparison of knockout efficiency of different delivery systems. The results show that RNP delivery method has the highest gene knockout efficiency in Jurkat cells.
Figure 4A-4C: Effects of N-oligo and fish sperm DNA on gene knockout efficiency by CRISPR-Cas9 system in T cells. Figure 4A shows comparison of gene knockout efficiency in T cells; Figure 4B shows comparison of gene knockout efficiency in CAR-T cells; Figure 4C shows the effect of fish sperm DNA fragments on gene knockout efficiency in T cells.
Figure 5: Determination of B2M knockout efficiency in T cells. The results show that B2M knockout efficiency was up to 81.7%.
Figure 6: Determination of PD-1 gene knockout efficiency in T cells. The results show that all the three crRNAs significantly knocked down PD-1.
Figure 7A-7B: Analysis of gene mutations resulting from RNP and N-Oligo or fish sperm DNA. Fig. 7A shows analysis result for TRAC, and Fig. 7B shows analysis result for B2M.
Figures 8A-8C: Analysis of off-target rate with RNP. Fig. 8A shows the analysis results of the TRAC gene off-target rate; Fig. 8B shows the analysis results of the B2M gene off-target rate; and Fig. 8C shows analysis results of the PD-1 gene off-target rate.
Figure 9A-9B: Analysis of CD25 and CD69 activation in TRAC knockout T cells. Figure 9A shows comparison of CD69 activation; Figure 9B shows comparison of CD25 activation.
Figure 10: Killing effect of CART19 on CD19 positive cells K562-CD19 and K562.
Figure 11: Killing effect of CART19 on Raji tumor cells.
Figure 12A-12B: Cytokine release during killing of Raji and Daudi tumor cells by CART19-N2.
Figure 13: Comparison of the killing ability of CART19 and UCART19 cells on tumor target cells. Figure 13A shows the killing effect on Dudi cells; Figure 13B shows the killing effect on Raji cells; and Figure 13C shows the killing effect on Nalm6 cells.
Figure 14A to 14C: The results of measurement of the expression level of cell surface CD107a in the process of *in vitro* killing target cells by CART19 and UCART19 cells. Fig. 14A shows the expression level of cell surface CD107a in the process of killing Daudi cells; Fig. 14B shows the expression level of cell surface CD107a in the process of killing Raji cells; and Fig. 14C shows the expression level of cell surface CD107a in the process of killing Nalm6 cells.
Figure 15A-15D: Analysis of antitumor activity of CART19 cells in mouse in vivo. Fig. 15A shows a flow chart of establishment of NOG mouse model and injection of CART19 cells; Fig. 15B shows the results of pictures 5 weeks after injection; Fig. 15C shows the statistical results of bioluminescence intensity in mice; Fig. 15D shows the overall survival of CART19 cells in mouse.
Figure 16A-16B: Analysis results of antitumor activity in mouse of K562 cells-stimulated CART19 and UCART19 cells. Figure 16A shows the tumor burden in mouse after CART19 and UCART19 without or with secondary stimulation with K562-CD19 cells were injected into NOG mouse model; and Figure 16B shows the survival rates of NOG mouse model injected with CART19 and UCART19 without or with secondary stimulation with K562-CD19 cells.
Figure 17A-17B: Changes in the number of human T cells in peripheral blood of mice within 3 weeks after CART injection. Figure 17A shows the proliferation profile of negative control CART-MSN cells in mice; and Figure 17B shows the proliferation profile of CART19-N2 cells in mice.
Figure 18A-18D: Survival rate, body weight change, and proliferation profile of human T cells in mice after injection of T-mock cells and T-TCR-cells. Figure 18A shows the survival rate of mice after injection of different cells; Figure 18B shows body weight changes of mice; Figure 18C shows the proportion of CD45⁺ cells in mice after injection of CTL-019 cells; and Figure 18D shows the proportion of CD45⁺ cells in mice after injection of CTL-019TCR^{-/-} cells.

### DETAILED DESCRIPTION OF THE DISCLOSURE

The present disclosure provides genetically modified UCART cells that can be used among different individuals, the genetically modified UCART cells have ability of specifically killing CD19 positive cells and tumor target cells *in vitro* and *in vivo,* and greatly reduce GvHD effects and allogeneic rejection.

### Terminology

In order to make the present disclosure more easily understood, certain technical and scientific terms are specifically defined below. Unless otherwise defined explicitly herein, all other technical and scientific terms used herein have the meaning commonly understood by one of ordinary skill in the art to which this disclosure belongs.

As used herein, the term "contacting" (i.e., contacting polynucleotide sequences with clustered regularly interspaced short palindromic repeats-related (Cas) protein and/or ribonucleic acids) is intended to include *in vitro* incubating the Cas protein and/or RNA, or *ex vivo* contact with cells. The step of contacting the polynucleotide sequences of the target genes with the Cas protein and/or ribonucleic acids disclosed herein can be carried out in any suitable manner. For example, the cells can be treated in the form of adherent or suspension culture. Cells contacted with the Cas protein and/or ribonucleic acids as disclosed herein can also be simultaneously or subsequently contacted with another agent, such as growth factor or other differentiation agent or environment, to stabilize or further differentiate the cells.

When applied to an isolated cell, the term "treating" includes subjecting the cell to any type of process or condition, or performing any type of operation or procedure on the cell. When applied to a subject, the term refers to providing the cell, in which the polynucleotide sequences of the target genes have been altered *ex vivo* according to the methods described herein, to an individual. The individual is typically ill or injured, or is at an increased risk of illness relative to the average member of the population and requires such attention, care or management.

The term "treating" as used herein, refers to administering to a subject an effective amount of cells in which the polynucleotide sequences of the target genes have been altered *ex vivo* according to the methods described herein, such that at least one symptom of the disease from which the subject suffered is alleviated or the disease is improved, for example, beneficial or desired clinical outcomes. For the purposes of the present disclosure, beneficial or desired clinical outcomes include, but are not limited to, alleviation of one or more symptoms, reduction of the disease level, stabilization of the disease state (i.e., no deterioration), delay or retardation in disease progression, improvement or mitigation of the disease state, and remission (whether partial or complete remission), whether detectable or undetectable. Treatment may mean prolonging survival as compared to the expected survival in the absence of treatment. Thus, those skilled in the art recognize that treatment may improve disease conditions, but may not be a complete cure for the disease. As used herein, the term "treatment" includes prophylaxis. Alternatively, treatment is "effective" in case that the progression of the disease is reduced or ceased. "Treatment" can also mean prolonging survival as compared to the expected survival in the absence of treatment. Patients in need of treatment include those who have been diagnosed having disease associated with the expression of polynucleotide sequences, and those who may develop such disease due to genetic susceptibility or other factors.

As used herein, "mutant cell" refers to a cell that differs from its original genotype. In some examples, "mutant cell" exhibits a mutant phenotype, for example, when a functionally normal gene is altered using the CRISPR/Cas system of the present disclosure. In other examples "mutant cell" exhibits a wild-type phenotype, for example, when a mutant genotype is corrected using the CRISPR/Cas system of the present disclosure. In some embodiments, the polynucleotide sequence of a target gene in a cell is altered to correct or repair the gene mutation (e.g., to restore the normal genotype of the cell). In some embodiments, the polynucleotide sequence of a target gene in a cell is altered to induce a genetic mutation (e.g., to disrupt the function of a gene or genomic element).

In some embodiments, the alteration is an indel. "Indel" as used herein refers to mutations resulting from insertions, deletions, or a combination thereof. As will be understood by those of skill in the art, an indel in a coding region of a genomic sequence will result in frameshift mutations unless the length of the indel is a multiple of three. In some embodiments, alteration is point mutation. "Point mutation" as used herein refers to a substitution that replaces one of the nucleotide. The CRISPR/Cas system of the present disclosure can be used to induce an indel of any length or a point mutations in a target polynucleotide sequences.

"Oligodeoxyribonucleic acid" or "N-oligo" refers to a deoxyribonucleic acid fragment with random sequence which is transformed into a cell together with RNP when gene knockout is performed with RNP delivery system. Preferably it refers to a double-stranded DNA with 100-250 bp in length, or a single-stranded DNA with 100-250 nt in length.

"Fish sperm DNA fragment" refer to small molecule fragment produced by mechanically shearing solution containing salmon sperm DNA. For example, 1% salmon sperm DNA solution is repeatedly aspirated with a 7-gauge needle to cut DNAs into small molecules, aliquoted and stored.

"Knockout" as used herein includes deleting all or part of target polynucleotide sequence in a way that interferes with the function of the target polynucleotides. For example, a knockout can be achieved by altering of a target polynucleotide sequence, and the alteration is performed by inducing indel in the target polynucleotide sequence in a functional domain (e.g., DNA binding domain). Based on the details described herein, one of skill in the art will readily understand how to use the CRISPR/Cas system of the present disclosure to knock out a target polynucleotide or a portion thereof.

In some embodiments, cleavage of the target gene results in decreased expression of the target gene. The term "decrease" is used herein generally to mean decreasing by a statistically significant amount. However, to avoid confusion, " decrease" means a decrease of at least 10% compared to a reference level, such as a decrease of at least about 20%, or at least about 30%, or at least about 40%, or at least about 50%, or at least about 60%, or at least about 70%, or at least about 75%, or at least about 80%, or at least about 90%, or a decrease of up to (and including) 100% compared to a reference level (i.e., a level corresponding to the absence of expression, when compared to the reference sample), or a decrease of any between 10% and 100%.

The term "statistically significant" or "significantly" refers to statistical significance and generally means a two standard deviations (2SD) less or below the normal marker concentration. The term means statistical evidence of the presence of difference. It is defined as the probability of making a decision to reject a hypothesis when the hypothesis is actually true. The decision is often expressed as p value.

In some embodiments, cleavage of a target gene is cleavage of a homozygous target gene. In some embodiments, cleavage of a target gene is cleavage of a heterozygous target gene.

The term "Cas9 protein" (also known as CRISPR-related endonuclease Cas9/Csnl) is a polypeptide comprising 1368 amino acids. An exemplary amino acid sequence of the Cas9 protein is shown in SEQ ID NO: 53. Cas9 contains two endonuclease domains, RuvC-like domain (residues 7-22, 759-766, and 982-989), which cleaves target DNA non-complementary to crRNA, and HNH nuclease domain (residues 810-872), which cleaves target DNA complementary to the crRNA.

The term "T cell receptor (TCR)" is a heterodimeric protein receptor for a specific antigenic peptide presented by the major histocompatibility complex (MHC). In the immune system, the binding of the antigen-specific TCR to the pMHC complex triggers direct physical contact between T cells and antigen presenting cells (APCs), and then T cells interact with other cell membrane surface molecules of APC, which causes a series of subsequent cell signaling and other physiological responses that allow different antigen-specific T cells to exert immune effects on their target cells.

TCR is a glycoprotein on the cell membrane surface in the form of a heterodimer formed by α chain/β chain or γ chain/δ chain. The TCR heterodimer in 95% of T cells consists of α and β chain, while 5% of T cells have TCR consisting of γ and δ chain. The native αβ heterodimeric TCR has α chain and β chain, and the α chain and the β chain constitute a subunit of the αβ heterodimeric TCR. Broadly, the α chain and the β chain comprise variable regions, a linker region and constant regions, and the β chain typically further contains a short diversified region between the variable region and the linker region, but this diversified region is often considered as a portion of the linker region. Each variable region comprises three CDRs (complementarity determining regions), CDR1, CDR2 and CDR3, which are interspersed in framework regions. The CDR regions determine the binding of the TCR to the pMHC complex, wherein the CDR3 is recombinantly composed of the variable regions and the linker region, and is referred to as the hypervariable region. It is generally considered that each of α and β chains of TCR has two "domains", namely a variable domain and a constant domain, and the variable domain consists of variable region linked to the linker region. The sequence of the TCR constant domain can be found in the public database of the International Immunogenetics Information System (IMGT). For example, the constant domain sequence of the TCR molecule α chain is "TRAC*01", and the constant domain sequence of the TCR molecule β chain is "TRBC1*01" or "TRBC2*01". In addition, the α and β chains of TCR also contain transmembrane region and a very short cytoplasmic region.

"B2M", also known as β-2 microglobulin, is the light chain of MHC class I molecules and is therefore an indispensable part of the major histocompatibility complex. In human, B2M is encoded by the b2m gene which is located on chromosome 15 and opposite to other MHC genes (as a cluster of genes) located on chromosome 6. The human protein consists of 119 amino acids and has a molecular weight of 11,800 Daltons. β-2 microglobulin deficient murine model has demonstrated that B2M is essential for MHC class I expression on cell surface and for stability of peptide binding cleft.

"PD-1" or "PD1" is a 50-55 kDa type I transmembrane receptor, which was originally identified in T cell line that underwent activation-induced apoptosis. PD-1 is expressed on T cells, B cells and macrophages. The PD-1 ligands are members of B7 family, PD-L1 (B7-H1) and PD-L2 (B7-DC).

PD-1 is a member of the immunoglobulin (Ig) superfamily and contains a single IgV-like domain in its extracellular region. The PD-1 cytoplasmic domain contains two tyrosine amino acids, of which the tyrosine much closer to the membrane (VAYEEL in mouse PD-1) is located within ITIM (an inhibitory motif of the immunoreceptor tyrosine). The presence of ITIM on PD-1 indicates that this molecule functions by recruiting cytosolic phosphatase to attenuate the signaling of antigen receptors. The human and murine PD-1 proteins share approximately 60% amino acid identity, with four conserved potential N-glycosylation sites and residues defining the Ig-V domain. The ITIM in cytoplasmic region and the ITIM-like motif around the carboxy terminal tyrosine (human and mouse TEYATI) are also conserved between human and murine orthologues.

The term "antibody" refers to an immunoglobulin molecule that specifically binds to an antigen. An antibody may be a complete immunoglobulin derived from natural source or derived from recombinant source, and may be an immunoreactive portion of a complete immunoglobulin. Antibodies are typically tetramers of immunoglobulin molecule. The antibodies of the present disclosure may exist in a variety of forms, including polyclonal antibodies, monoclonal antibodies, Fv, Fab, and F(ab)2, as well as single chain antibodies and humanized antibodies (Harlow et al, 1999, In: Using Antibodies: A Laboratory Manual, Cold Spring Harbor Laboratory Press, NY; Harlow et al, 1989, In: Antibodies: A Laboratory Manual, Cold Spring Harbor, New York; Houston et al, 1988, Proc. Natl. Acad. Sci. USA 85: 5879-5883; Bird et al., 1988, Science 242: 423-426).

The term "antibody fragment" as used herein refers to a portion of an intact antibody and refers to the antigenic determinant variable region of an intact antibody. Examples of antibody fragments include, but are not limited to, Fab, Fab', F(ab')2 and Fv fragments, linear antibodies formed from antibody fragments, scFv antibodies, and multispecific antibodies.

The term "antibody heavy chain" as used herein refers to the larger chain of the two types of polypeptide chains present in all antibody molecules in their naturally occurring conformation.

The term "antibody light chain" as used herein refers to the smaller chain of the two types of polypeptide chains present in all antibody molecules in their naturally occurring conformation, and the κ and λ light chains are referred to as the two major isoforms of the antibody light chain.

The term "synthetic antibody" as used herein means an antibody produced by recombinant DNA techniques, such as, for example, an antibody expressed by a phage. The term should also be interpreted to mean an antibody that has been produced by synthesis mediated by synthetic DNA molecule (which encodes the antibody and the DNA molecule expresses the antibody protein or the amino acid sequence defining the antibody), wherein the DNA or amino acid sequence has been obtained by technology of synthesis of DNA or amino acid sequence available and well-known in the art.

The term "antigen" or "Ag" as used herein is defined as a molecule that elicits an immune response that can be involved in antibody production, or activation of specific immunocompetent cells. Those skilled in the art will appreciate that any macromolecule, including all proteins or peptides, can be used as an antigen. Furthermore, the antigen can be derived from recombinant or genomic DNA. One skilled in the art will appreciate any DNA, including nucleotide sequence or partial nucleotide sequence encoding a protein that elicits an immune response, encodes the term "antigen" as used herein. Furthermore, it will be understood by those skilled in the art that the antigen is not necessary to be individually encoded by full length nucleotide sequence of the gene. It will be readily apparent that the present disclosure includes, but is not limited to, use of partial nucleotide sequences of more than one gene, and these nucleotide sequences are arranged in different combinations to elicit a desired immune response. Furthermore, it will be understood by those skilled in the art that the antigen does not have to be encoded by a "gene" at all, and the antigen can be produced, synthesized or derived from a biological sample. Such biological samples can include, but are not limited to, tissue samples, tumor samples, cells, or biological fluids.

The term "autoantigen" means any autoantigen that is recognized by the immune system as foreign. Autoantigens include, but are not limited to, cellular proteins, phosphoproteins, cell surface proteins, cellular lipids, nucleic acids, glycoproteins, including cell surface receptors.

The term "chimeric antigen receptor" or "CAR" as used herein refers to an artificial T cell receptor engineered to be expressed on an immune effector cell and specifically bind to an antigen. CAR can be used as a therapy by adoptive cell transfer. T cells are removed from a patient and modified in a way that they can express a receptor specific for a particular antigen. CAR may also include an intracellular activation domain, a transmembrane domain, and an extracellular domain, including a tumor associated antigen binding region. In some aspects, the CAR comprises a single-chain variable fragment (scFv)-derived monoclonal antibody fused to a CDS-ζ transmembrane and intracellular domain. The designed specificity of the CAR can be derived from the ligand of the receptor (e.g., a peptide). In some embodiments, the CAR can target cancer by redirecting the specificity of T cells to express a CAR specific for a tumor associated antigen.

The term "anti-tumor effect" as used herein refers to a biological effect which may be clearly indicated by a decrease in tumor volume, a decrease in the number of tumor cells, a decrease in the number of metastases, an increase in life expectancy, or various physiological symptom associated with a cancerous condition. "Anti-tumor effect" can also be shown by the ability of the disclosed peptides, polynucleotides, cells and antibodies to prevent tumors.

The term "autoimmune disease" as used herein is defined as a disorder resulting from an autoimmune response. Autoimmune diseases are the result of inappropriate and excessive responses to autoantigens. Examples of autoimmune diseases include, but are not limited to, Addison's disease, alopecia areata, ankylosing spondylitis, autoimmune hepatitis, autoimmune mumps, Crohn's disease, diabetes (type 1), dystrophic bullous epidermis palliative, epididymitis, glomerulonephritis, Graves' disease, Guillain-Barre syndrome, Hashimoto's disease, hemolytic anemia, systemic lupus erythematosus, multiple sclerosis, myasthenia gravis, Pemphigus vulgaris, psoriasis, rheumatic fever, rheumatoid arthritis, sarcoidosis, scleroderma, Sjogren's syndrome, spondyloarthropathy, thyroiditis, vasculitis, vitiligo, myxedema, malignancy anemia, ulcerative colitis, etc.

The term "co-stimulatory ligand" as used herein includes a molecule on antigen presenting cells (e.g., APCs, dendritic cells, B cells, etc.) that specifically binds to an associated co-stimulatory molecule on T cell, thereby said co-stimulatory ligand provides, not only the primary signal (by binding the TCR/CD3 complex to the peptide-loaded MHC molecule), but also a signal that mediates T cell response, and said T cell response includes but not limited to proliferation, activation, differentiation, and the like. Co-stimulatory ligands can include, but are not limited to, CD7, B7-1(CD80), B7-2(CD86), PD-L1, PD-L2, 4-1BBL, OX40L, inducible co-stimulatory ligands (ICOS-L), intercellular adhesion molecule (ICAM), CD30L, CD40, CD70, CD83, HLA-G, MICA, MICB, HVEM, lymphotoxin β receptor, 3/TR6, ILT3, ILT4, HVEM, agonists or antibodies of Toll ligand-binding receptor and ligands specifically binding to B7-H3. Co-stimulatory ligands also include, inter alia, antibodies that specifically bind to co-stimulatory molecules present on T cells, said co-stimulatory molecules including but not limited to CD27, CD28, 4-1BB, OX40, CD30, CD40, PD-1, ICOS, lymphocyte function associated antigen-1 (LFA-1), CD2, CD7, LIGHT, NKG2C, B7-H3 and ligands that specifically bind to CD83.

"Co-stimulatory molecule" refers to an associated binding partner on T cells that specifically binds to a co-stimulatory ligand, thereby mediating co-stimulatory response of T cells, such as, but not limited to, proliferation. The co-stimulatory molecules include but are not limited to MHC Class I molecules, BTLA and Toll ligand receptors.

As used herein, "co-stimulatory signal" refers to a signal that associates with a primary signal, such as TCR/CD3 binding, and results in T cell proliferation and/or up- or down-regulation of key molecules.

As used herein, the term "autologous" refers to any substance derived from the same individual, which is subsequently reintroduced into the individual.

"Allogeneic" refers to a graft derived from a different animal of the same species.

"Xenogeneic" refers to a graft derived from an animal of a different species.

The term "cleavage" refers to breakage of a covalent bond, for example, in the backbone of a nucleic acid molecule. Cleavage can be initiated by a variety of methods including, but are not limited to, enzymatic cleavage or chemical hydrolysis of phosphodiester bonds. Cleavage is possible for both single-strand and double-strand. A double-strand may be cleaved by cleavage events of the two different single-strands. DNA cleavage can result in blunt ends or staggered ends. In certain embodiments, a fusion polypeptide can be used to target a cleaved double stranded DNA.

The term "CRISPR/CAS", "clustered regular interspaced short palindromic repeats system" or "CRISPR" refers to a DNA locus comprising a short repeat of base sequence. Each repeat is followed by a short segment of spacer DNA that was previously exposed to virus. Bacteria and archaea have evolved an adaptive immune defense known as CRISPR-CRISPR-associated (Cas) system, which uses short RNA to direct the degradation of exogenous nucleic acids. In bacteria, the CRISPR system provides acquired immunity against invading foreign DNAs via RNA-guided DNA cleavage.

In Type II CRISPR/Cas system, "spacer region", a short segment of exogenous DNA, is integrated into the CRISPR genomic locus and transcribed and processed into short CRISPR RNA (crRNA). These crRNAs were annealed with transactivated crRNAs (tracrRNAs) and pathogenic DNA was sequence-specifically cleaved and silenced under the direction of Cas protein. Recent work has shown that a "seed" sequence within the crRNA and Protospacer Adjacent Motif (PAM) sequence containing a conserved dinucleotide upstream of the crRNA-binding region are required for Cas9 protein to recognize the target.

In order to direct Cas9 to cleave the sequence of interest, crRNA-tracrRNA fusion transcript can be designed based on human U6 polymerase III promoter, hereinafter referred to as "guide RNA" or "sgRNA". CRISPR/CAS-mediated genome editing and regulation highlights its transformative potential for basic science, cell engineering and therapy.

The term "CRISPRi" refers to a CRISPR system for sequence-specifically repressing or inhibiting gene repression, such as at the transcriptional level.

As used herein, the term "exogenous" refers to any substance introduced into an organism, cell, tissue or system, or produced outside of the organism, cell, tissue or system.

As used herein, "endogenous" or "endogenic" refers to any substance derived from an organism, cell, tissue or system, or produced within the organism, cell, tissue or system.

The term "downregulation" as used herein refers to the reduction or elimination of gene expression of one or more genes.

The term "expansion" as used herein refers to an increase in the number, such as an increase in the number of T cells. In one embodiment, the number of *ex vivo* expanded T cells is increased relative to the number originally present in the culture. In another embodiment, the number of *ex vivo* expanded T cells is increased relative to the number of other cell types in the culture.

The term *"ex vivo"* as used herein refers to cells which have been removed from a living organism (e.g., human) and are propagated outside the organism (e.g., in a culture dish, test tube, or bioreactor).

The term "expression" as used herein is defined as the transcription and/or translation of particular nucleotide sequence driven by its promoter.

A "vector" is a composition of matter, it includes an isolated nucleic acid, and can be used to deliver the isolated nucleic acid into the cell. Numerous vectors are known in the art including, but not limited to, linear polynucleotides, polynucleotides associated with ionic or amphiphilic compounds, plasmids, and viruses. Thus, the term "vector" includes autonomously replicating plasmids or viruses. The term should also be interpreted to include non-plasmid and non-viral compounds that facilitate the transfer of nucleic acids into cells, such as, for example, polylysine compounds, liposomes, and the like. Examples of viral vectors include, but are not limited to, Sendai viral vectors, adenoviral vectors, adeno-associated viral vectors, retroviral vectors, lentiviral vectors, and the like.

"Expression vector" refers to a vector comprising a recombinant polynucleotide comprising an expression control sequence operably linked to a nucleotide sequence to be expressed. Expression vectors include sufficient cis-acting elements for expression; other elements for expression can be supplied by host cells or supplied in an *in vitro* expression system. Expression vectors include all those incorporating recombinant polynucleotides known in the art, such as cosmids, plasmids (e.g., naked or contained in liposomes), and viruses (e.g., Sendai virus, lentivirus, retrovirus, adenovirus and adeno-associated virus).

As used herein, "homologous" refers to the sequence identity of the subunits between two polymer molecules, for example, two nucleic acid molecules, such as two DNA molecules or two RNA molecules, or two polypeptide molecules. When the subunit positions in both molecules are occupied by the same monomeric subunit, for example, if the position in each of the two DNA molecules is occupied by adenine, they are homologous at that position. The homology between two sequences is a direct function of the number of matching or homologous positions; for example, if half positions in the two sequences (for example, five positions in a polymer with ten subunits in length) are homologous, the two sequences are 50% homologous; if 90% of the positions (e.g., 9 out of 10) are matched or homologous, the two sequences are 90% homologous.

As used herein, "identity" refers to the sequence identity of the subunit between two polymer molecules, particularly between two amino acid molecules, for example, between two polypeptide molecules. When two amino acid sequences share the same residue at the same position, for example, if the position in each of the two polypeptide molecules is occupied by arginine, they are identical at that position. In alignment, the identity or extent of two amino acid sequences having the same residue at the same position is often expressed as a percentage. The identity between two sequences is a direct function of the number of matching or homologous positions; for example, if half positions in the two sequences (for example, five positions in a polymer with ten amino acids in length) are homologous, the two sequences are 50% identical; if 90% of the positions (e.g., 9 out of 10) are matched or homologous, the two sequences are 90% identical.

The term "immunoglobulin" or "Ig" as used herein is defined as a class of proteins that function as antibodies. Antibodies expressed by B cells are sometimes referred to as BCR (B cell receptor) or antigen receptor. The five members included in such proteins are IgA, IgG, IgM, IgD, and IgE. IgA is a primary antibody present in body secretion such as saliva, tears, milk, gastrointestinal secretions, and mucus secretions of the respiratory and genitourinary tract. IgG is the most common circulating antibody. IgM is the major immunoglobulin produced in the primary immune response in most subjects. It is the most effective immunoglobulin in agglutination, complement binding and other antibody responses and is important in defensing bacteria and viruses. IgD is an immunoglobulin that does not have the well-known antibody function, but it can act as an antigen receptor. IgE is an immunoglobulin that mediates rapid allergic reaction by initiating release of mediators from mast cells and basophilic granulocyte, upon exposure to allergens.

The term "immune response" as used herein is defined as a cellular response to an antigen that occurs when lymphocytes recognize an antigen molecule as a foreigner and induce the formation of an antibody and/or the lymphocytes are activated to remove the antigen.

"Isolated" means being altered or removed from natural state. For example, a nucleic acid or peptide naturally present in a living animal is not "isolated", but the same nucleic acid or peptide that is partially or completely separated from the coexisting material in its natural state is deemed as "isolated." The isolated nucleic acid or protein may be present in substantially purified form, or may be present in a non-native environment, such as, for example, in host cells.

The term "knockdown" as used herein refers to a decrease in gene expression of one or more genes.

The term "knockout" as used herein refers to ablation of gene expression of one or more genes.

"Lentivirus" as used herein refers to a genus of the retroviridae. In retroviruses, lentivirus is the only virus that is capable of infecting non-dividing cells, such as HIV, S1V and FIV; They can transfer significant amount of genetic information into the DNA of host cells, hence they are the most efficient means of gene-delivery vectors. Vectors derived from lentivirus provide a means to accomplish significant levels of gene transfer *in vivo.*

The term "modified" as used herein means an altered state or structure of a molecule or cell of the present disclosure. Molecules can be modified in various ways, including chemical, structural and functional modification. Cells can be modified by the introduction of nucleic acids.

The term "regulation" as used herein means to mediate a detectable increase or decrease of the response level in a subject, compared to the response level in a subject without the treatment or not administrated with a compound, and/or compared to the response level in a subject without treatment but comparable in other aspects. The term includes disturbing and/or affecting a natural signal or response, thereby mediating a beneficial therapeutic response in a subject, preferably a human.

Unless otherwise specified, "nucleotide sequences encoding an amino acid sequence" include all nucleotide sequences which encode the same amino acid sequence, due to the degeneracy. The phrase "nucleotide sequences encoding a protein or RNA" may also include intron(s), to an extent that the nucleotide sequence encoding the protein may comprise (one or more) intron(s) in particular form.

The term "operably linked" refers to a functional linkage between a regulatory sequence and a heterologous nucleic acid sequence, which results in expression of the heterologous nucleic acid sequence. For example, when the first nucleic acid sequence is in a functional relationship with the second nucleic acid sequence, the first nucleic acid sequence is operably linked to the second nucleic acid sequence. For example, a promoter is operably linked to a coding sequence if the promoter affects the transcription or expression of the coding sequence. Typically, operably linked DNA sequence is adjacent to each other and, where necessary, two protein coding regions are ligated in the same reading frame.

The term "overexpressed" tumor antigen or "overexpression" of tumor antigen is intended to indicate abnormal expression level of a tumor antigen from a diseased region (such as cells from a solid tumor in a particular tissue or organ of a patient), relative to the expression level of a normal cell from the tissue or organ. Patients with solid tumors or hematological malignancies characterized by overexpressed tumor antigen can be determined by standard assays known in the art.

As used herein, the terms "peptide", "polypeptide" and "protein" are used interchangeably and refer to a compound consisting of amino acid residues covalently linked by peptide bonds. The protein or peptide must contain at least two amino acids, and there is no limitation to the maximum number of amino acids constituting a protein or peptide sequence. Polypeptide include any peptide or protein comprising two or more amino acids connected to each other by peptide bond. As used herein, the term refers to both short chains (which are also commonly referred to in the art as, for example, peptide, oligopeptide, and oligomer); and longer chains (which are commonly referred to in the art as proteins), which has many types. "Polypeptide" includes, for example, biologically active fragment, substantially homologous polypeptide, oligopeptide, homodimer, heterodimer, variant of polypeptide, modified polypeptide, derivative, analog, fusion protein, and the like. Polypeptide includes natural peptide, recombinant peptide, synthetic peptide, or a combination thereof.

The term "promoter" as used herein is defined as a DNA sequence, which is necessary for initiating the specific transcription of a polynucleotide sequence, and is recognized by synthetic machine of a cell, or is introduced by synthetic machinery.

As used herein, the term "promoter/regulatory sequence" means a nucleic acid sequence required for expression of a gene product operably linked to the promoter/regulatory sequence. In some examples, the sequence may be a core promoter sequence, and in other examples, the sequence may also include enhancer sequence and other regulatory elements required for expression of the gene product. For example, a promoter/regulatory sequence can be a sequence that expresses a gene product in a tissue-specific manner.

"Signaling pathway" refers to the biochemical relationship among a variety of signaling molecules which function in transferring a signal from one part of a cell to another part of the cell.

"Cell surface receptors" include complexes of a molecule with other molecule(s), and they are capable of receiving and transmitting signals across the plasma membrane of a cell.

The term "specifically binds to" as used herein with respect to an antibody means that the antibody recognizes a specific antigen but does not substantially recognize or bind to other molecules in the sample. For example, an antibody that specifically binds to an antigen from one species can also bind to an antigen from one or more species. However, such cross-species reactivity *per se* does not change the specificity of class of the antibody. In some cases, an antibody that specifically binds to an antigen can also bind to an antigen of a different allelic form. However, such cross-reactivity *per se* does not change the specificity of the class of the antibody. In some instances, the terms "specifically bind to" or "specifically bind" may refer to the interaction of an antibody, protein or peptide with a second chemical species, meaning that the interaction is dependent on the presence of a particular structure of the chemical species (e.g., an antigen determinant or epitope); for example, an antibody recognizes and binds to a particular protein structure, rather than generally recognizes and binds to the protein. If an antibody is specific for epitope "A", the presence of a molecule comprising epitope A (or free unlabeled A) in the reaction comprising the labeled "A" and the antibody will reduce the amount of labeled A bound to the antibody.

"Single-chain antibody" refers to an antibody formed by recombinant DNA techniques in which immunoglobulin heavy and light chain fragments are linked to the Fv region via an engineered span of amino acids. A variety of methods for generating single-chain antibodies are known, and are included in U.S. Patent No. 4,694,778; Bird (1988) Science 242: 423-442; Huston et al. (1988) Proc. Natl. Acad. Sci. USA 85: 5879-5883; Ward et al (1989) Nature 334: 54454; Skerra et al (1988) Science 242: 1038-1041.

The term "stimulation" means mediating signal transduction event by binding to a stimulatory molecule (e.g., TCR/CD3 complex) and its associated ligand, for example, but not limited to, the first response induced by signal transduction via the TCR/CD3 complex. Stimulation can mediate altered expression of certain molecules, for example, down-regulation of TGF-β, and/or recombination of cytoskeletal structures.

"Stimulatory molecule", as a term used herein, means a molecule on T cells that specifically binds to an associated stimulatory ligand present on an antigen presenting cell.

As used herein, "stimulatory ligand" refers to the following ligand: when present on antigen presenting cells (e.g., aAPC, dendritic cells, B-cells, etc.), it can specifically bind to the associated binding partner on T cells (referred to as "stimulatory molecule" herein), thereby mediates the primary response of T cells, including but not limited to activation, initiation of an immune response, proliferation, and the like. Stimulatory ligands are well known in the art and include, inter alia, MHC class I molecules: loaded with peptide, anti-CD3 antibody, super-agonist anti-CD28 antibody and super-agonist anti-CD2 antibody.

The term "subject" is intended to include living organisms (e.g., mammals) in which an immune response can be elicited. The "subject" or "patient" as used therein may be human or non-human mammals. Non-human mammals include, for example, domestic animals and pets, such as sheep, bovine, porcine, canine, feline, and murine. Preferably, the subject is human.

"Substantially purified" cells as used herein are cells that are substantially free of other cell types. Substantially purified cells also refers to the cells that have been separated from other cell types which are normally associated with the cells in their naturally occurring state. In some cases, a substantially purified population of cells refers to a homogeneous population of cells. In other instances, the term simply refers to cells that have been separated from other cells that are normally associated with the cells in their native state. In some embodiments, the cells are cultured *in vitro.* In other embodiments, the cells are not cultured *in vitro.*

"Target site" or "target sequence" refers to genomic nucleic acid sequences, which define a portion of nucleic acid that can specifically bind to a binding molecule under conditions sufficiently for binding.

The term "therapeutic" as used herein means treatment and/or prevention. Therapeutic effects are obtained by suppression, alleviation or eradication of the disease state.

The term "transfected" or "transformed" or "transduced" as used herein refers to a process by which an exogenous nucleic acid is transferred or introduced into a host cell. "Transfected" or "transformed" or "transduced" cells are those which have been transfected, transformed or transduced with exogenous nucleic acid(s). Cells include primary subject cells and their progeny.

The phrase "under transcriptional control" or "operably linked to" as used herein means that the promoter is in the correct position and orientation relative to the polynucleotide, thereby it can control the initiation of transcription by RNA polymerase and the expression of the polynucleotide.

The term "effective amount" or "therapeutically effective amount" refers to an amount of a subject compound that will elicit a biological or medical response in a tissue, system or subject which a researcher, veterinarian, doctor or other clinician is looking for. The term "therapeutically effective amount" includes an amount of a compound which, when administered, is sufficient to prevent the development of one or more of the signs or symptoms of the disorder or disease, or to some extent alleviate one or more of the signs or symptoms of the disorder or disease being treated. The therapeutically effective amount will vary depending on the compound, the disease and its severity, and the age, weight of the subject to be treated, etc.

### DETAILED DESCRIPTION OF THE DISCLOSURE

### Examples

The present disclosure is further illustrated in detail by the following examples. These examples are for illustrative purposes only and are not intended to limit the scope of the disclosure.

In the examples of the present disclosure, where specific conditions are not described, the experiments are generally conducted under conventional conditions; or under conditions proposed by the material or product manufacturers. Where the source of the reagents is not specifically given, the reagents are the commercially available conventional reagents.

### Example 1. Construction of pLVX-EF1-CD19 CAR lentiviral vector

(1) Gene sequence information of human CD8α leading signal region (SEQ ID NO: 5), human CD8α hinge region (SEQ ID NO: 7), human CD8α transmembrane region (SEQ ID NO: 9), human 4-1BB intracellular region (SEQ ID NO: 11) and human CD3ξ intracellular region (SEQ ID NO: 13) was obtained from the NCBI website database; CD19scFv was derived from FMC63 antibody (See Mol Immunol. 1997; 34: 1157-1165.), and the nucleic acid sequences thereof are shown in CD19-N1 scFv (SEQ ID NO: 17), CD19-N2 scFv (SEQ ID NO: 19), CD19-N3 scFv (SEQ ID NO: 21) and CD19-N4 scFv (SEQ ID NO: 23), respectively.
(2) The nucleotides with the above sequences were synthesized by Nanjing Genscript Biotech Co., Ltd., and the restriction enzyme sites were added to both ends to obtain a complete CD19-CAR gene sequence CD19 CAR-N1 (SEQ ID NO: 25), CD19 CAR-N2 (SEQ ID NO: 27), CD19 CAR-N3 (SEQ ID NO: 29), CD19 CAR-N4 (SEQ ID NO: 31). The CD19-CAR structure, from 5' to 3', successively consists of CD19scFv, hinge structure, transmembrane structure, 4-1BB and CD3ζ.
(3) pLVX-EF1-MSC plasmid obtained
   The CMV promoter was excised from pLVX-CMV-MCS (Clontech, pLVX-IRES-ZsGreenl, Cat. No. 632187) and the EF1 promoter (SEQ ID NO: 4) was excised from pCDH-EF1-MCS (purchased from System Biosciences, Cat. No. CD530A- 2) with ClaI and EcoRI endonucleases. The pLVX-CMV-MCS vector from which CMV has been excised and the EF1 promoter fragment from pCDH-EF1-MCS were recovered by agarose gel electrophoresis. The EF1 promoter fragment was ligated into the vector pLVX-MCS by using the DNA Ligation Kit (Takara) to obtain a plasmid, pLVX-EF1-MSC, which was then transformed into competent E. coli TOP10. The plasmid was extracted and verified by sequencing to obtain the correct pLVX-EF1-MCS lentiviral vector. The sequencing primers were: PLVX-PF (SEQ ID NO: 1) and PCDHI-R (SEQ ID NO: 2). The sequence is shown below:
   PLVX-PF: CATTCGATTAGTGAACGGATCT (SEQ ID NO: 1)
   PCDHI-R: GACGGCAATATGGTGGAA (SEQ ID NO: 2).
(4) The CD19-CAR nucleic acid molecule was digested with EcoRI (NEB) and NotI (NEB), and ligated into the lentiviral vector pLVX-EF1-MCS between *EcoRI* and *NotI* sites by DNA Ligation Kit (Takara) to obtain pLVX- EF1-CD19 CAR lentiviral vector: pLVX-EF1-002A (with CD19 CAR sequence of CD19 CAR-N1), CD19-CAR lentiviral vector pLVX-EF1-002B (with CD19 CAR sequence of CD19 CAR-N2), CD19-CAR lentiviral vector pLVX-EF1-002C (with CD19 CAR sequence is CD19 CAR-N3), CD19-CAR lentiviral vector pLVX-EF1-002D (the CD19 CAR sequence of CD19 CAR-N4), and then transformed into the competent E. coli TOP10. The resulting pLVX-EF1-CD19 CAR lentiviral vector was sequenced, and the sequencing primers were: pLVX-PF (SEQ ID NO: 1) and Xd-SR (SEQ ID NO: 3), and the sequences are as follows:
   pLVX-PF: CATTCGATTAGTGAACGGATCT (SEQ ID NO: 1)
   Xd-SR: AAAGCCATACGGGAAGCAATA (SEQ ID NO: 3).

The monoclonal colonies with correct sequences were selected for activation and inoculation, and the lentiviral vector was extracted using QIAGEN EndoFree Plasmid Extraction Kit.

The relevant sequences involved in Example 1 are as follows:
EF1 promoter: 
GGATCTGCGATCGCTCCGGTGCCCGTCAGTGGGCAGAGCGCACATCGCCCA
CAGTCCCCGAGAAGTTGGGGGGAGGGGTCGGCAATTGAACGGGTGCCTAG
AGAAGGTGGCGCGGGGTAAACTGGGAAAGTGATGTCGTGTACTGGCTCCG
CCTTTTTCCCGAGGGTGGGGGAGAACCGTATATAAGTGCAGTAGTCGCCGT
 
GAACGTTCTTTTCGCAACGGGTTTGCCGCCAGAACACAGCTGAAGCTTCG
AGGGGCTCGCATCTCTCCTTCACGCGCCCGCCGCCCTACCTGAGGCCGCCA
TCCACGCCGGTTGAGTCGCGTTCTGCCGCCTCCCGCCTGTGGTGCCTCCTG
AACTGCGTCCGCCGTCTAGGTAAGTTTAAAGCTCAGGTCGAGACCGGGCCT
TTGTCCGGCGCTCCCTTGGAGCCTACCTAGACTCAGCCGGCTCTCCACGCT
TTGCCTGACCCTGCTTGCTCAACTCTACGTCTTTGTTTCGTTTTCTGTTCTG
CGCCGTTACAGATCCAAGCTGTGACCGGCGCCTAC
(SEQ ID NO: 4)
Nucleotide sequence of human CD8α leading signal region: 
ATGGCCTTACCAGTGACCGCCTTGCTCCTGCCGCTGGCCTTGCTGCTCCAC
GCCGCCAGGCCG
(SEQ ID NO: 5)
Amino acid sequence of human CD8α leading signal region:
   MALPVTALLLPLALLLHAARP (SEQ ID NO: 6)
Nucleotide sequence of human CD8α hinge region: 
ACCACGACGCCAGCGCCGCGACCACCAACACCGGCGCCCACCATCGCGTC
GCAGCCCCTGTCCCTGCGCCCAGAGGCGTGCCGGCCAGCGGCGGGGGCG
CAGTGCACACGAGGGGGCTGGACTTCGCCTGTGAT
(SEQ ID NO: 7)
Amino acid sequence of human CD8α hinge region:
   TTTPAPRPPTPAPTIASQPLSLRPEACRPAAGGAVHTRGLDFACD (SEQ ID NO: 8)
Nucleotide sequence of human CD8α transmembrane region: 
ATCTACATCTGGGCGCCCTTGGCCGGGACTTGTGGGGTCCTTCTCCTGTCA
CTGGTTATCACCCTTTACTGC
(SEQ ID NO: 9)
Amino acid sequence of human CD8α transmembrane region:
   IYIWAPLAGTCGVLLLSLVITLYC (SEQ ID NO: 10)
Nucleotide sequence of human 4-1BB intracellular region: 
AAACGGGGCAGAAAGAAACTCCTGTATATATTCAAACAACCATTTATGAGA
CCAGTACAAACTACTCAAGAGGAAGATGGCTGTAGCTGCCGATTTCCAGAA
 
GAAGAAGAAGGAGGATGTGAACTG
(SEQ ID NO: 11)
Amino acid sequence of human 4-1BB intracellular region:
   KRGRKKLLYIFKQPFMRPVQTTQEEDGCSCRFPEEEEGGCEL (SEQ ID NO: 12)
Nucleotide sequence of human CD3ζ intracellular region: 
AGAGTGAAGTTCAGCAGGAGCGCAGACGCCCCCGCGTACAAGCAGGGCC
AGAACCAGCTCTATAACGAGCTCAATCTAGGACGAAGAGAGGAGTACGAT
GTTTTGGACAAGAGACGTGGCCGGGACCCTGAGATGGGGGGAAAGCCGA
GAAGGAAGAACCCTCAGGAAGGCCTGTACAATGAACTGCAGAAAGATAAG
ATGGCGGAGGCCTACAGTGAGATTGGGATGAAAGGCGAGCGCCGGAGGGG
CAAGGGGCACGATGGCCTTTACCAGGGTCTCAGTACAGCCACCAAGGACA
CCTACGACGCCCTTCACATGCAGGCCCTGCCCCCTCGC
(SEQ ID NO: 13)
Amino acid sequence of human CD3ζ intracellular region: 
RVKFSRSADAPAYKQGQNQLYNELNLGRREEYDVLDKRRGRDPEMGGKPRR
KNPQEGLYNELQKDKMAEAYSEIGMKGERRRGKGHDGLYQGLSTATKDTYD
ALHMQALPPR
(SEQ ID NO: 14)
Heavy chain variable region amino acid sequence of FMC63 antibody: 
EVKLQESGPGLVAPSQSLSVTCTVSGVSLPDYGVSWIRQPPRKGLEWLGVIWG
SETTYYNSALKSRLTIIKDNSKSQVFLKMNSLQTDDTAIYYCAKHYYYGGSYA
MDYWGQGTSVTVSS
(SEQ ID NO: 15)
Light chain variable region amino acid sequence of FMC63 antibody: 
DIQMTQTTSSLSASLGDRVTISCRASQDISKYLNWYQQKPDGTVKLLIYHTSR
LHSGVPSRFSGSGSGTDYSLTISNLEQEDIATYFCQQGNTLPYTFGGGTKLEITR
ADAAPTVSIFPP
(SEQ ID NO: 16)
Nucleotide sequence of CD19-N1scFv: 
GACATCCAGATGACACAGACTACATCCTCCCTGTCTGCCTCTCTGGGAGAC
AGAGTCACCATCAGTTGCAGGGCAAGTCAGGACATTAGTAAATATTTAAAT
TGGTATCAGCAGAAACCAGATGGAACTGTTAAACTCCTGATCTACCATACAT
 
CAAGATTACACTCAGGAGTCCCATCAAGGTTCAGTGGCAGTGGGTCTGGA
ACAGATTATTCTCTCACCATTAGCAACCTGGAGCAAGAAGATATTGCCACTT
ACTTTTGCCAACAGGGTAATACGCTTCCGTACACGTTCGGAGGGGGGACTA
AGTTGGAAATAACAGGTGGCGGTGGCTCGGGCGGTGGTGGGTCGGGTGGC
GGCGGATCTGAGGTGAAACTGCAGGAGTCAGGACCTGGCCTGGTGGCGCC
CTCACAGAGCCTGTCCGTCACATGCACTGTCTCAGGGGTCTCATTACCCGA
CTATGGTGTAAGCTGGATTCGCCAGCCTCCACGAAAGGGTCTGGAGTGGCT
GGGAGTAATATGGGGTAGTGAAACCACATACTATAATTCAGCTCTCAAATCC
AGACTGACCATCATCAAGGACAACTCCAAGAGCCAAGTTTTCTTAAAAATG
AACAGTCTGCAAACTGATGACACAGCCATTTACTACTGTGCCAAACATTATT
ACTACGGTGGTAGCTATGCTATGGACTACTGGGGCCAAGGAACCTCAGTCA
CCGTCTCCTCA
(SEQ ID NO: 17)
Amino acid sequence of CD19-N1scFv: 
DIQMTQTTSSLSASLGDRVTISCRASQDISKYLNWYQQKPDGTVKLLIYHTSR
LHSGVPSRFSGSGSGTDYSLTISNLEQEDIATYFCQQGNTLPYTFGGGTKLEITG
GGGSGGGGSGGGGSEVKLQESGPGLVAPSQSLSVTCTVSGVSLPDYGVSWIR
QPPRKGLEWLGVIWGSETTYYNSALKSRLTIIKDNSKSQVFLKMNSLQTDDTA
IYYCAKHYYYGGSYAMDYWGQGTSVTVSS
(SEQ ID NO: 18)
Nucleotide sequence of CD19-N2 scFv: 
GACATCCAGATGACACAGACTACATCCTCCCTGTCTGCCTCTCTGGGAGAC
AGAGTCACCATCAGTTGCAGGGCAAGTCAGGACATTAGTAAATATTTAAAT
TGGTATCAGCAGAAACCAGATGGAACTGTTAAACTCCTGATCTACCATACAT
CAAGATTACACTCAGGAGTCCCATCAAGGTTCAGTGGCAGTGGGTCTGGA
ACAGATTATTCTCTCACCATTAGCAACCTGGAGCAAGAAGATATTGCCACTT
ACTTTTGCCAACAGGGTAATACGCTTCCGTACACGTTCGGAGGGGGGACTA
AGTTGGAAATAACACGGGCTGGTGGCGGTGGCTCGGGCGGTGGTGGGTCG
GGTGGCGGCGGATCTGAGGTGAAACTGCAGGAGTCAGGACCTGGCCTGGT
GGCGCCCTCACAGAGCCTGTCCGTCACATGCACTGTCTCAGGGGTCTCATT
ACCCGACTATGGTGTAAGCTGGATTCGCCAGCCTCCACGAAAGGGTCTGGA
GTGGCTGGGAGTAATATGGGGTAGTGAAACCACATACTATAATTCAGCTCTC
AAATCCAGACTGACCATCATCAAGGACAACTCCAAGAGCCAAGTTTTCTTA
AAAATGAACAGTCTGCAAACTGATGACACAGCCATTTACTACTGTGCCAAA
 
CATTATTACTACGGTGGTAGCTATGCTATGGACTACTGGGGCCAAGGAACCT
CAGTCACCGTCTCCTCA
(SEQ ID NO: 19)
Amino acid sequence of CD19-N2 scFv: 
DIQMTQTTSSLSASLGDRVTISCRASQDISKYLNWYQQKPDGTVKLLIYHTSR
LHSGVPSRFSGSGSGTDYSLTISNLEQEDIATYFCQQGNTLPYTFGGGTKLEITR
AGGGGSGGGGSGGGGSEVKLQESGPGLVAPSQSLSVTCTVSGVSLPDYGVSW
IRQPPRKGLEWLGVIWGSETTYYNSALKSRLTIIKDNSKSQVFLKMNSLQTDD
TAIYYCAKHYYYGGSYAMDYWGQGTSVTVSS
(SEQ ID NO: 20)
Nucleotide sequence of CD19-N3 scFv: 
GACATCCAGATGACACAGACTACATCCTCCCTGTCTGCCTCTCTGGGAGAC
AGAGTCACCATCAGTTGCAGGGCAAGTCAGGACATTAGTAAATATTTAAAT
TGGTATCAGCAGAAACCAGATGGAACTGTTAAACTCCTGATCTACCATACAT
CAAGATTACACTCAGGAGTCCCATCAAGGTTCAGTGGCAGTGGGTCTGGA
ACAGATTATTCTCTCACCATTAGCAACCTGGAGCAAGAAGATATTGCCACTT
ACTTTTGCCAACAGGGTAATACGCTTCCGTACACGTTCGGAGGGGGGACTA
AGTTGGAAATAACACGGGCGGCCGCAGGTGGCGGTGGCTCGGGCGGTGGT
GGGTCGGGTGGCGGCGGATCTGAGGTGAAACTGCAGGAGTCAGGACCTGG
CCTGGTGGCGCCCTCACAGAGCCTGTCCGTCACATGCACTGTCTCAGGGGT
CTCATTACCCGACTATGGTGTAAGCTGGATTCGCCAGCCTCCACGAAAGGG
TCTGGAGTGGCTGGGAGTAATATGGGGTAGTGAAACCACATACTATAATTCA
GCTCTCAAATCCAGACTGACCATCATCAAGGACAACTCCAAGAGCCAAGTT
TTCTTAAAAATGAACAGTCTGCAAACTGATGACACAGCCATTTACTACTGT
GCCAAACATTATTACTACGGTGGTAGCTATGCTATGGACTACTGGGGCCAAG
GAACCTCAGTCACCGTCTCCTCA
(SEQ ID NO: 21)
Amino acid sequence of CD19-N3 scFv: 
DIQMTQTTSSLSASLGDRVTISCRASQDISKYLNWYQQKPDGTVKLLIYHTSR
LHSGVPSRFSGSGSGTDYSLTISNLEQEDIATYFCQQGNTLPYTFGGGTKLEITR
AAAGGGGSGGGGSGGGGSEVKLQESGPGLVAPSQSLSVTCTVSGVSLPDYGV
SWIRQPPRKGLEWLGVIWGSETTYYNSALKSRLTIIKDNSKSQVFLKMNSLQT
DDTAIYYCAKHYYYGGSYAMDYWGQGTSVTVSS
 (SEQ ID NO: 22)
Nucleotide sequence of CD19-N4 scFv: 
GACATCCAGATGACACAGACTACATCCTCCCTGTCTGCCTCTCTGGGAGAC
AGAGTCACCATCAGTTGCAGGGCAAGTCAGGACATTAGTAAATATTTAAAT
TGGTATCAGCAGAAACCAGATGGAACTGTTAAACTCCTGATCTACCATACAT
CAAGATTACACTCAGGAGTCCCATCAAGGTTCAGTGGCAGTGGGTCTGGA
ACAGATTATTCTCTCACCATTAGCAACCTGGAGCAAGAAGATATTGCCACTT
ACTTTTGCCAACAGGGTAATACGCTTCCGTACACGTTCGGAGGGGGGACTA
AGTTGGAAATAACACGGGCTGATGCTGCACCAACTGTATCCATCTTCCCAC
CAGGTGGCGGTGGCTCGGGCGGTGGTGGGTCGGGTGGCGGCGGATCTGAG
GTGAAACTGCAGGAGTCAGGACCTGGCCTGGTGGCGCCCTCACAGAGCCT
GTCCGTCACATGCACTGTCTCAGGGGTCTCATTACCCGACTATGGTGTAAGC
TGGATTCGCCAGCCTCCACGAAAGGGTCTGGAGTGGCTGGGAGTAATATGG
GGTAGTGAAACCACATACTATAATTCAGCTCTCAAATCCAGACTGACCATCA
TCAAGGACAACTCCAAGAGCCAAGTTTTCTTAAAAATGAACAGTCTGCAA
ACTGATGACACAGCCATTTACTACTGTGCCAAACATTATTACTACGGTGGTA
GCTATGCTATGGACTACTGGGGCCAAGGAACCTCAGTCACCGTCTCCTCA
(SEQ ID NO: 23)
Amino acid sequence of CD19-N4 scFv: 
DIQMTQTTSSLSASLGDRVTISCRASQDISKYLNWYQQKPDGTVKLLIYHTSR
LHSGVPSRFSGSGSGTDYSLTISNLEQEDIATYFCQQGNTLPYTFGGGTKLEITR
ADAAPTVSIFPPGGGGSGGGGSGGGGSEVKLQESGPGLVAPSQSLSVTCTVSG
VSLPDYGVSWIRQPPRKGLEWLGVIWGSETTYYNSALKSRLTIIKDNSKSQVF
LKMNSLQTDDTAIYYCAKHYYYGGSYAMDYWGQGTSVTVSS
(SEQ ID NO: 24)
Nucleotide sequence of CD19 CAR-N1: 
ATGGCCTTACCAGTGACCGCCTTGCTCCTGCCGCTGGCCTTGCTGCTCCAC
GCCGCCAGGCCGGACATCCAGATGACACAGACTACATCCTCCCTGTCTGCC
TCTCTGGGAGACAGAGTCACCATCAGTTGCAGGGCAAGTCAGGACATTAG
TAAATATTTAAATTGGTATCAGCAGAAACCAGATGGAACTGTTAAACTCCTG
ATCTACCATACATCAAGATTACACTCAGGAGTCCCATCAAGGTTCAGTGGC
AGTGGGTCTGGAACAGATTATTCTCTCACCATTAGCAACCTGGAGCAAGAA
GATATTGCCACTTACTTTTGCCAACAGGGTAATACGCTTCCGTACACGTTCG
 
GAGGGGGACTAAGTTGGAAATAACAGGTGGCGGTGGCTCGGGCGGTGGT
GGGTCGGGTGGCGGCGGATCTGAGGTGAAACTGCAGGAGTCAGGACCTGG
CCTGGTGGCGCCCTCACAGAGCCTGTCCGTCACATGCACTGTCTCAGGGGT
CTCATTACCCGACTATGGTGTAAGCTGGATTCGCCAGCCTCCACGAAAGGG
TCTGGAGTGGCTGGGAGTAATATGGGGTAGTGAAACCACATACTATAATTCA
GCTCTCAAATCCAGACTGACCATCATCAAGGACAACTCCAAGAGCCAAGTT
TTCTTAAAAATGAACAGTCTGCAAACTGATGACACAGCCATTTACTACTGT
GCCAAACATTATTACTACGGTGGTAGCTATGCTATGGACTACTGGGGCCAAG
GAACCTCAGTCACCGTCTCCTCAACCACGACGCCAGCGCCGCGACCACCA
ACACCGGCGCCCACCATCGCGTCGCAGCCCCTGTCCCTGCGCCCAGAGGC
GTGCCGGCCAGCGGCGGGGGGCGCAGTGCACACGAGGGGGCTGGACTTC
GCCTGTGATATCTACATCTGGGCGCCCTTGGCCGGGACTTGTGGGGTCCTTC
TCCTGTCACTGGTTATCACCCTTTACTGCAAACGGGGCAGAAAGAAACTCC
TGTATATATTCAAACAACCATTTATGAGACCAGTACAAACTACTCAAGAGGA
AGATGGCTGTAGCTGCCGATTTCCAGAAGAAGAAGGAGGATGTGAAC
TGAGAGTGAAGTTCAGCAGGAGCGCAGACGCCCCCGCGTACCAGCAGGG
CCAGAACCAGCTCTATAACGAGCTCAATCTAGGACGAAGAGAGGAGTACG
ATGTTTTGGACAAGAGACGTGGCCGGGACCCTGAGATGGGGGGAAAGCCG
AGAAGGAAGAACCCTCAGGAAGGCCTGTACAATGAACTGCAGAAAGATAA
GATGGCGGAGGCCTACAGTGAGATTGGGATGAAAGGCGAGCGCCGGAGGG
GCAAGGGGCACGATGGCCTTTACCAGGGTCTCAGTACAGCCACCAAGGAC
ACCTACGACGCCCTTCACATGCAGGCCCTGCCCCCTCGCTGATAA
(SEQ ID NO: 25)
Amino acid sequence of CD19 CAR-N1: 
MALPVTALLLPLALLLHAARPDIQMTQTTSSLSASLGDRVTISCRASQDISKYL
NWYQQKPDGTVKLLIYHTSRLHSGVPSRFSGSGSGTDYSLTISNLEQEDIATYF
CQQGNTLPYTFGGGTKLEITGGGGSGGGGSGGGGSEVKLQESGPGLVAPSQSL
SVTCTVSGVSLPDYGVSWIRQPPRKGLEWLGVIWGSETTYYNSALKSRLTIIK
DNSKSQVFLKMNSLQTDDTAIYYCAKHYYYGGSYAMDYWGQGTSVTVSSTT
TPAPRPPTPAPTIASQPLSLRPEACRPAAGGAVHTRGLDFACDIYIWAPLAGTCG
VLLLSLVITLYCKRGRKKLLYIFKQPFMRPVQTTQEEDGCSCRFPEEEEGGCEL
RVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYD
ALHMQALPPR
(SEQ ID NO: 26)
Nucleotide sequence of CD19 CAR-N2: 
ATGGCCTTACCAGTGACCGCCTTGCTCCTGCCGCTGGCCTTGCTGCTCCAC
GCCGCCAGGCCGGACATCCAGATGACACAGACTACATCCTCCCTGTCTGCC
TCTCTGGGAGACAGAGTCACCATCAGTTGCAGGGCAAGTCAGGACATTAG
TAAATATTTAAATTGGTATCAGCAGAAACCAGATGGAACTGTTAAACTCCTG
ATCTACCATACATCAAGATTACACTCAGGAGTCCCATCAAGGTTCAGTGGC
AGTGGGTCTGGAACAGATTATTCTCTCACCATTAGCAACCTGGAGCAAGAA
GATATTGCCACTTACTTTTGCCAACAGGGTAATACGCTTCCGTACACGTTCG
GAGGGGGGACTAAGTTGGAAATAACACGGGCTGGTGGCGGTGGCTCGGGC
GGTGGTGGGTCGGGTGGCGGCGGATCTGAGGTGAAACTGCAGGAGTCAGG
ACCTGGCCTGGTGGCGCCCTCACAGAGCCTGTCCGTCACATGCACTGTCTC
AGGGGTCTCATTACCCGACTATGGTGTAAGCTGGATTCGCCAGCCTCCACG
AAAGGGTCTGGAGTGGCTGGGAGTAATATGGGGTAGTGAAACCACATACTA
TAATTCAGCTCTCAAATCCAGACTGACCATCATCAAGGACAACTCCAAGAG
CCAAGTTTTCTTAAAAATGAACAGTCTGCAAACTGATGACACAGCCATTTA
CTACTGTGCCAAACATTATTACTACGGTGGTAGCTATGCTATGGACTACTGG
GGCCAAGGAACCTCAGTCACCGTCTCCTCAACCACGACGCCAGCGCCGCG
ACCACCAACACCGGCGCCCACCATCGCGTCGCAGCCCCTGTCCCTGCGCCC
AGAGGCGTGCCGGCCAGCGGCGGGGGGCGCAGTGCACACGAGGGGGCTG
GACTTCGCCTGTGATATCTACATCTGGGCGCCCTTGGCCGGGACTTGTGGG
GTCCTTCTCCTGTCACTGGTTATCACCCTTTACTGCAAACGGGGCAGAAAG
AAACTCCTGTATATATTCAAACAACCATTTATGAGACCAGTACAAACTACTC
AAGAGGAAGATGGCTGTAGCTGCCGATTTCCAGAAGAAGAAGAAGGAGG
ATGTGAACTGAGAGTGAAGTTCAGCAGGAGCGCAGACGCCCCCGCGTACC
AGCAGGGCCAGAACCAGCTCTATAACGAGCTCAATCTAGGACGAAGAGAG
GAGTACGATGTTTTGGACAAGAGACGTGGCCGGGACCCTGAGATGGGGGG
AAAGCCGAGAAGGAAGAACCCTCAGGAAGGCCTGTACAATGAACTGCAG
AAAGATAAGATGGCGGAGGCCTACAGTGAGATTGGGATGAAAGGCGAGCG
CCGGAGGGGCAAGGGGCACGATGGCCTTTACCAGGGTCTCAGTACAGCCA
CCAAGGACACCTACGACGCCCTTCACATGCAGGCCCTGCCCCCTCGCTGAT
AA
(SEQ ID NO: 27)
Amino acid sequence of CD19 CAR-N2: MALPVTALLLPLALLLHAARPDIQMTQTTSSLSASLGDRVTISCRASQDISKYL 
NWYQQKPDGTVKLLIYHTSRLHSGVPSRFSGSGSGTDYSLTISNLEQEDIATYF
CQQGNTLPYTFGGGTKLEITRAGGGGSGGGGSGGGGSEVKLQESGPGLVAPS
QSLSVTCTVSGVSLPDYGVSWIRQPPRKGLEWLGVIWGSETTYYNSALKSRLT
IIKDNSKSQVFLKMNSLQTDDTAIYYCAKHYYYGGSYAMDYWGQGTSVTVS
STTTPAPRPPTPAPTIASQPLSLRPEACRPAAGGAVHTRGLDFACDIYIWAPLAG
TCGVLLLSLVITLYCKRGRKKLLYIFKQPFMRPVQTTQEEDGCSCRFPEEEEGG
CELRVKFSRSADAPAYQQGQNQLYNELNLGRREEYDVLDKRRGRDPEMGGK
PRRKNPQEGLYNELQKDKMAEAYSEIGMKGERRRGKGHDGLYQGLSTATKD
TYDALHMQALPPR
(SEQ ID NO: 28)
Nucleotide sequence of CD19 CAR-N3: 
ATGGCCTTACCAGTGACCGCCTTGCTCCTGCCGCTGGCCTTGCTGCTCCAC
GCCGCCAGGCCGGACATCCAGATGACACAGACTACATCCTCCCTGTCTGCC
TCTCTGGGAGACAGAGTCACCATCAGTTGCAGGGCAAGTCAGGACATTAG
TAAATATTTAAATTGGTATCAGCAGAAACCAGATGGAACTGTTAAACTCCTG
ATCTACCATACATCAAGATTACACTCAGGAGTCCCATCAAGGTTCAGTGGC
AGTGGGTCTGGAACAGATTATTCTCTCACCATTAGCAACCTGGAGCAAGAA
GATATTGCCACTTACTTTTGCCAACAGGGTAATACGCTTCCGTACACGTTCG
GAGGGGGGACTAAGTTGGAAATAACAGGGCGGCCGCAGGTGGCGGTGG
CTCGGGCGGTGGTGGGTCGGGTGGCGGCGGATCTGAGGTGAAACTGCAGG
AGTCAGGACCTGGCCTGGTGGCGCCCTCACAGAGCCTGTCCGTCACATGC
ACTGTCTCAGGGGTCTCATTACCCGACTATGGTGTAAGCTGGATTCGCCAG
CCTCCACGAAAGGGTCTGGAGTGGCTGGGAGTAATATGGGGTAGTGAAAC
CACATACTATAATTCAGCTCTCAAATCCAGACTGACCATCATCAAGGACAAC
TCCAAGAGCCAAGTTTTCTTAAAAATGAACAGTCTGCAAACTGATGACACA
GCCATTTACTACTGTGCCAAACATTATTACTACGGTGGTAGCTATGCTATGGA
CTACTGGGGCCAAGGAACCTCAGTCACCGTCTCCTCAACCACGACGCCAG
CGCCGCGACCACCAACACCGGCGCCCACCATCGCGTCGCAGCCCCTGTCC
CTGCGCCCAGAGGCGTGCCGGCCAGCGGCGGGGGGCGCAGTGCACACGA
GGGGGCTGGACTTCGCCTGTGATATCTACATCTGGGCGCCCTTGGCCGGGA
CTTGTGGGGTCCTTCTCCTGTCACTGGTTATCACCCTTTACTGCAAACGGGG
CAGAAAGAAACTCCTGTATATATTCAAACAACCATTTATGAGACCAGTACA
AACTACTCAAGAGGAAGATGGCTGTAGCTGCCGATTTCCAGAAGAAGAAG
AAGGAGGATGTGAACTGAGAGTGAAGTTCAGCAGGAGCGCAGACGCCCC
CGCGTACCAGCAGGGCCAGAACCAGCTCTATAACGAGCTCAATCTAGGACG
 
AAGAGAGGAGTACGATGTTTTGGACAAGAGACGTGGCCGGGACCCTGAGA
TGGGGGGAAAGCCGAGAAGGAAGAACCCTCAGGAAGGCCTGTACAATGA
ACTGCAGAAAGATAAGATGGCGGAGGCCTACAGTGAGATTGGGATGAAAG
GCGAGCGCCGGAGGGGCAAGGGGCACGATGGCCTTTACCAGGGTCTCAGT
ACAGCCACCAAGGACACCTACGACGCCCTTCACATGCAGGCCCTGCCCCC
TCGCTGATAA
(SEQ ID NO: 29)
Amino acid sequence of CD19 CAR-N3: 
MALPVTALLLPLALLLHAARPDIQMTQTTSSLSASLGDRVTISCRASQDISKYL
NWYQQKPDGTVKLLIYHTSRLHSGVPSRFSGSGSGTDYSLTISNLEQEDIATYF
CQQGNTLPYTFGGGTKLEITRAAAGGGGSGGGGSGGGGSEVKLQESGPGLVA
PSQSLSVTCTVSGVSLPDYGVSWIRQPPRKGLEWLGVIWGSETTYYNSALKSR
LTIIKDNSKSQVFLKMNSLQTDDTAIYYCAKHYYYGGSYAMDYWGQGTSVT
VSSTTTPAPRPPTPAPTIASQPLSLRPEACRPAAGGAVHTRGLDFACDIYIWAPL
AGTCGVLLLSLVITLYCKRGRKKLLYIFKQPFMRPVQTTQEEDGCSCRFPEEEE
GGCELRVKFSRSADAPAYQQGQNQLYNELNLGRREEYDVLDKRRGRDPEMG
GKPRRKNPQEGLYNELQKDKMAEAYSEIGMKGERRRGKGHDGLYQGLSTAT
KDTYDALHMQALPPR
(SEQ ID NO: 30)
Nucleotide sequence of CD19 CAR-N4: 
ATGGCCTTACCAGTGACCGCCTTGCTCCTGCCGCTGGCCTTGCTGCTCCAC
GCCGCCAGGCCGGACATCCAGATGACACAGACTACATCCTCCCTGTCTGCC
TCTCTGGGAGACAGAGTCACCATCAGTTGCAGGGCAAGTCAGGACATTAG
TAAATATTTAAATTGGTATCAGCAGAAACCAGATGGAACTGTTAAACTCCTG
ATCTACCATACATCAAGATTACACTCAGGAGTCCCATCAAGGTTCAGTGGC
AGTGGGTCTGGAACAGATTATTCTCTCACCATTAGCAACCTGGAGCAAGAA
GATATTGCCACTTACTTTTGCCAACAGGGTAATACGCTTCCGTACACGTTCG
GAGGGGGGACTAAGTTGGAAATAACACGGGCTGATGCTGCACCAACTGTA
TCCATCTTCCCACCAGGTGGCGGTGGCTCGGGCGGTGGTGGGTCGGGTGG
CGGCGGATCTGAGGTGAAACTGCAGGAGTCAGGACCTGGCCTGGTGGCGC
CCTCACAGAGCCTGTCCGTCACATGCACTGTCTCAGGGGTCTCATTACCCG
ACTATGGTGTAAGCTGGATTCGCCAGCCTCCACGAAAGGGTCTGGAGTGGC
TGGGAGTAATATGGGGTAGTGAAACCACATACTATAATTCAGCTCTCAAATC
CAGACTGACCATCATCAAGGACAACTCCAAGAGCCAAGTTTTCTTAAAAAT
GAACAGTCTGCAAACTGATGACACAGCCATTTACTACTGTGCCAAACATTA
 
TTACTACGGTGGTAGCTATGCTATGGACTACTGGGGCCAAGGAACCTCAGT
CACCGTCTCCTCAACCACGACGCCAGCGCCGCGACCACCAACACCGGCGC
CCACCATCGCGTCGCAGCCCCTGTCCCTGCGCCCAGAGGCGTGCCGGCCA
GCGGCGGGGGGCGCAGTGCACACGAGGGGGCTGGACTTCGCCTGTGATAT
CTACATCTGGGCGCCCTTGGCCGGGACTTGTGGGGTCCTTCTCCTGTCACT
GGTTATCACCCTTTACTGCAAACGGGGCAGAAAGAAACTCCTGTATATATTC
AAACAACCATTTATGAGACCAGTACAAACTACTCAAGAGGAAGATGGCTGT
AGCTGCCGATTTCCAGAAGAAGAAGAAGGAGGATGTGAACTGAGAGTGA
AGTTCAGCAGGAGCGCAGACGCCCCCGCGTACCAGCAGGGCCAGAACCA
GCTCTATAACGAGCTCAATCTAGGACGAAGAGAGGAGTACGATGTTTTGGA
CAAGAGACGTGGCCGGGACCCTGAGATGGGGGGAAAGCCGAGAAGGAAG
AACCCTCAGGAAGGCCTGTACAATGAACTGCAGAAAGATAAGATGGCGGA
GGCCTACAGTGAGATTGGGATGAAAGGCGAGCGCCGGAGGGGCAAGGGG
CACGATGGCCTTTACCAGGGTCTCAGTACAGCCACCAAGGACACCTACGAC
GCCCTTCACATGCAGGCCCTGCCCCCTCGCTGATAA
(SEQ ID NO: 31)
Amino acid sequence of CD19 CAR-N4:

MALPVTALLLPLALLLHAARPDIQMTQTTSSLSASLGDRVTISCRASQDISKYL
NWYQQKPDGTVKLLIYHTSRLHSGVPSRFSGSGSGTDYSLTISNLEQEDIATYF
CQQGNTLPYTFGGGTKLEITRADAAPTVSIFPPGGGGSGGGGSGGGGSEVKLQ
ESGPGLVAPSQSLSVTCTVSGVSLPDYGVSWIRQPPRKGLEWLGVIWGSETTY
YNSALKSRLTIIKDNSKSQVFLKMNSLQTDDTAIYYCAKHYYYGGSYAMDYW
GQGTSVTVSSTTTPAPRPPTPAPTIASQPLSLRPEACRPAAGGAVHTRGLDFAC
DIYIWAPLAGTCGVLLLSLVITLYCKRGRKKLLYIFKQPFMRPVQTTQEEDGCS
CRFPEEEEGGCELRVKFSRSADAPAYQQGQNQLYNELNLGRREEYDVLDKRR
GRDPEMGGKPRRKNPQEGLYNELQKDKMAEAYSEIGMKGERRRGKGHDGL
YQGLSTATKDTYDALHMQALPPR
(SEQ ID NO: 32)

For the method of preparation of the positive control CTL109, see Patent Application WO2012079000A1, of which the application date is 09 December 2011 and the publication date is 14 June 2012. The sequences of the CD19-CAR in the Patent Application are as follows:
CD19-CAR nucleotide sequence of the positive control CTL109: ATGGCCTTACCAGTGACCGCCTTGCTCCTGCCGCTGGCCTTGCTGCTCCAC 
GCCGCCAGGCCGGACATCCAGATGACACAGACTACATCCTCCCTGTCTGCC
TCTCTGGGAGACAGAGTCACCATCAGTTGCAGGGCAAGTCAGGACATTAG
TAAATATTTAAATTGGTATCAGCAGAAACCAGATGGAACTGTTAAACTCCTG
ATCTACCATACATCAAGATTACACTCAGGAGTCCCATCAAGGTTCAGTGGC
AGTGGGTCTGGAACAGATTATTCTCTCACCATTAGCAACCTGGAGCAAGAA
GATATTGCCACTTACTTTTGCCAACAGGGTAATACGCTTCCGTACACGTTCG
GAGGGGGGACCAAGCTGGAGATCACAGGTGGCGGTGGCTCGGGCGGTGG
TGGGTCGGGTGGCGGCGGATCTGAGGTGAAACTGCAGGAGTCAGGACCTG
GCCTGGTGGCGCCCTCACAGAGCCTGTCCGTCACATGCACTGTCTCAGGGG
TCTCATTACCCGACTATGGTGTAAGCTGGATTCGCCAGCCTCCACGAAAGG
GTCTGGAGTGGCTGGGAGTAATATGGGGTAGTGAAACCACATACTATAATTC
AGCTCTCAAATCCAGACTGACCATCATCAAGGACAACTCCAAGAGCCAAG
TTTTCTTAAAAATGAACAGTCTGCAAACTGATGACACAGCCATTTACTACTG
TGCCAAACATTATTACTACGGTGGTAGCTATGCTATGGACTACTGGGGCCAA
GGAACCTCAGTCACCGTCTCCTCAACCACGACGCCAGCGCCGCGACCACC
AACACCGGCGCCCACCATCGCGTCGCAGCCCCTGTCCCTGCGCCCAGAGG
CGTGCCGGCCAGCGGCGGGGGGCGCAGTGCACACGAGGGGGCTGGACTT
CGCCTGTGATATCTACATCTGGGCGCCCTTGGCCGGGACTTGTGGGGTCCTT
CTCCTGTCACTGGTTATCACCCTTTACTGCAAACGGGGCAGAAAGAAACTC
CTGTATATATTCAAACAACCATTTATGAGACCAGTACAAACTACTCAAGAGG
AAGATGGCTGTAGCTGCCGATTTCCAGAAGAAGAAGGAGGATGTGAA
CTGAGAGTGAAGTTCAGCAGGAGCGCAGACGCCCCCGCGTACAAGCAGG
GCCAGAACCAGCTCTATAACGAGCTCAATCTAGGACGAAGAGAGGAGTAC
GATGTTTTGGACAAGAGACGTGGCCGGGACCCTGAGATGGGGGGAAAGCC
GAGAAGGAAGAACCCTCAGGAAGGCCTGTACAATGAACTGCAGAAAGATA
AGATGGCGGAGGCCTACAGTGAGATTGGGATGAAAGGCGAGCGCCGGAGG
GGCAAGGGGCACGATGGCCTTTACCAGGGTCTCAGTACAGCCACCAAGGA
CACCTACGACGCCCTTCACATGCAGGCCCTGCCCCCTCGCTAA
(SEQ ID NO: 33)
Nucleotide sequence of CD3ζ intracellular region: 
AGAGTGAAGTTCAGCAGGAGCGCAGACGCCCCCGCGTACCAGCAGGGCC
AGAACCAGCTCTATAACGAGCTCAATCTAGGACGAAGAGAGGAGTACGAT
GTTTTGGACAAGAGACGTGGCCGGGACCCTGAGATGGGGGGAAAGCCGA
GAAGGAAGAACCCTCAGGAAGGCCTGTACAATGAACTGCAGAAAGATAAG
ATGGCGGAGGCCTACAGTGAGATTGGGATGAAAGGCGAGCGCCGGAGGGG
 
CAAGGGGCACGATGGCCTTTACCAGGGTCTCAGTACAGCCACCAAGGACA
CCTACGACGCCCTTCACATGCAGGCCCTGCCCCCTCGC
(SEQ ID NO: 56)
Amino acid sequence of CD3ζ intracellular region:

RVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYD
ALHMQALPPR
(SEQ ID NO: 57)

For the method of the preparation of the positive control CTL-019 of the present disclosure, see the Patent Application WO2012079000A1, in which the CD19-CAR nucleotide sequence of the positive control CTL-109 is shown in SEQ ID NO: 33, and the CD19-CAR amino acid sequence of the positive control CTL-019 is in SEQ ID NO: 34.
CD19-CAR amino acid sequence of the positive control CTL109:

MALPVTALLLPLALLLHAARPDIQMTQTTSSLSASLGDRVTISCRASQDISKYL
NWYQQKPDGTVKLLIYHTSRLHSGVPSRFSGSGSGTDYSLTISNLEQEDIATYF
CQQGNTLPYTFGGGTKLEITGGGGSGGGGSGGGGSEVKLQESGPGLVAPSQSL
SVTCTVSGVSLPDYGVSWIRQPPRKGLEWLGVIWGSETTYYNSALKSRLTIIK
DNSKSQVFLKMNSLQTDDTAIYYCAKHYYYGGSYAMDYWGQGTSVTVSSTT
TPAPRPPTPAPTIASQPLSLRPEACRPAAGGAVHTRGLDFACDIYIWAPLAGTCG
VLLLSLVITLYCKRGRKKLLYIFKQPFMRPVQTTQEEDGCSCRFPEEEEGGCEL
RVKFSRSADAPAYKQGQNQLYNELNLGRREEYDVLDKRRGRDPEMGGKPRR
KNPQEGLYNELQKDKMAEAYSEIGMKGERRRGKGHDGLYQGLSTATKDTYD
ALHMQALPPR
(SEQ ID NO: 34)

For the method of preparation of the negative control CART-MSN, see Patent Application CN104159909A. During the process of preparing SS1CAR, the BamHI restriction site of SS1CAR. (which was located between the CD8α leading signal region and SS1 scFv) taught in the Patent Application was removed when synthesizing the whole gene sequence of SSI-CAR. The nucleotide sequence of MSN-CAR (SS1CAR) is shown in SEQ ID NO: 35, and the amino acid sequence of MSN-CAR (SS1CAR) is shown in SEQ ID NO: 36.
Nucleotide sequence of MSN-CAR (SS1CAR): ATGGCCTTACCAGTGACCGCCTTGCTCCTGCCGCTGGCCTTGCTGCTCCAC 
GCCGCCAGGCCGCAGGTACAACTGCAGCAGTCTGGGCCTGAGCTGGAGAA
GCCTGGCGCTTCAGTGAAGATATCCTGCAAGGCTTCTGGTTACTCATTCAC
TGGCTACACCATGAACTGGGTGAAGCAGAGCCATGGAAAGAGCCTTGAGT
GGATTGGACTTATTACTCCTTACAATGGTGCTTCTAGCTACAACCAGAAGT
TCAGGGGCAAGGCCACATTAACTGTAGACAAGTCATCCAGCACAGCCTAC
ATGGACCTCCTCAGTCTGACATCTGAAGACTCTGCAGTCTATTTCTGTGCA
AGGGGGGGTTACGACGGGAGGGGTTTTGACTACTGGGGCCAAGGGACCA
CGGTCACCGTCTCCTCAGGTGGAGGCGGTTCAGGCGGCGGTGGCTCTAGC
GGTGGTGGATCGGACATCGAGCTCACTCAGTCTCCAGCAATCATGTCTGC
ATCTCCAGGGGAGAAGGTCACCATGACCTGCAGTGCCAGCTCAAGTGTAA
GTTACATGCACTGGTACCAGCAGAAGTCAGGCACCTCCCCCAAAAGATGG
ATTTATGACACATCCAAACTGGCTTCTGGAGTCCCAGGTCGCTTCAGTGGC
AGTGGGTCTGGAAACTCTTACTCTCTCACAATCAGCAGCGTGGAGGCTGA
AGATGATGCAACTTATTACTGCCAGCAGTGGAGTAAGCACCCTCTCACGT
ACGGTGCTGGGACAAAGTTGGAAATCAAAAGCAGCGAGAGCAAGTACGG
CCCTCCCTGCCCCCCTTGCCCTGCCCCCGAGTTCCTGGGCGGACCCAGCGT
GTTCCTGTTCCCCCCCAAGCCCAAGGACACCCTGATGATCAGCCGGACCC
CCGAGGTGACCTGTGTGGTGGTGGACGTGTCCCAGGAGGACCCCGAGGTC
CAGTTCAACTGGTACGTGGACGGCGTGGAGGTGCACAACGCCAAGACCAA
GCCCCGGGAGGAGCAGTTCAATAGCACCTACCGGGTGGTGTCCGTGCTGA
CCGTGCTGCACCAGGACTGGCTGAACGGCAAGGAATACAAGTGTAAGGTG
TCCAACAAGGGCCTGCCCAGCAGCATCGAGAAAACCATCAGCAAGGCCA
AGGGCCAGCCTCGGGAGCCCCAGGTGTACACCCTGCCCCCTAGCCAAGAG
GAGATGACCAAGAACCAGGTGTCCCTGACCTGCCTGGTGAAGGGCTTCTA
CCCCAGCGACATCGCCGTGGAGTGGGAGAGCAACGGCCAGCCCGAGAAC
AACTACAAGACCACCCCCCCTGTGCTGGACAGCGACGGCAGCTTCTTCCT
GTACAGCCGGCTGACCGTGGACAAGAGCCGGTGGCAGGAGGGCAACGTC
TTTAGCTGCTCCGTGATGCACGAGGCCCTGCACAACCACTACACCCAGAA
GAGCCTGAGCCTGTCCCTGGGCAAGATGTTCTGGGTGCTGGTCGTTGTGG
GCGGCGTGCTGGCCTGCTACAGCCTGCTGGTGACAGTGGCCTTCATCATCT
TTTGGGTGAGGAGCAAGCGGAGCAGACTGCTGCACAGCGACTACATGAAC
ATGACCCCCCGGAGGCCTGGCCCCACCCGGAAGCACTACCAGCCCTACGC
CCCTCCCAGGGATTTCGCCGCCTACCGGAGCCGGGTGAAGTTCAGCCGGA
GCGCCGACGCCCCTGCCTACCAGCAGGGCCAGAACCAGCTGTACAACGAG
CTGAACCTGGGCCGGAGGGAGGAGTACGACGTGCTGGACAAGCGGAGAG
 
GCCGGGACCCTGAGATGGGCGGCAAGCCCCGGAGAAAGAACCCCCAGGA
GGGCCTGTATAACGAACTGCAGAAAGACAAGATGGCCGAGGCCTACAGC
GAGATCGGCATGAAGGGCGAGCGGAGGCGGGGCAAGGGCCACGACGGCC
TGTACCAGGGCCTGAGCACCGCCACCAAGGATACCTACGACGCCCTGCAC
ATGCAGGCCCTGCCCCCCAGA
(SEQ ID NO: 35)
Amino acid sequence of MSN-CAR (SS1CAR):

MALPVTALLLPLALLLHAARPQVQLQQSGPELEKPGASVKISCKASGYSFTGY
TMNWVKQSHGKSLEWIGLITPYNGASSYNQKFRGKATLTVDKSSSTAYMDLL
SLTSEDSAVYFCARGGYDGRGFDYWGQGTTVTVSSGGGGSGGGGSSGGGSDI
ELTQSPAIMSASPGEKVTMTCSASSSVSYMHWYQQKSGTSPKRWIYDTSKLAS
GVPGRFSGSGSGNSYSLTISSVEAEDDATYYCQQWSKHPLTYGAGTKLEIKSS
ESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSD
IAVEWESNGQPENNYKTTPPVLDSDGSFFLYSRLTVDKSRWQEGNVFSCSVM
HEALHNHYTQKSLSLSLGKMFWVLVVVGGVLACYSLLVTVAFIIFWVRSKRS
RLLHSDYMNMTPRRPGPTRKHYQPYAPPRDFAAYRSRVKFSRSADAPAYQQG
QNQLYNELNLGRREEYDVLDKRRGRDPEMGGKPRRKNPQEGLYNELQKDK
MAEAYSEIGMKGERRRGKGHDGLYQGLSTATKDTYDALHMQALPPR
(SEQ ID NO: 36)

### Example 2. Extraction of PBMCs

Healthy volunteers who had no symptom of cold or fever were recruited, and informed consents were signed. 100 ml of blood was taken from a vein by medical professionals to BD anticoagulant tubes (Cat. No. 367886). Blood was mixed with an equal amount of PBS buffer (containing 2% fetal bovine serum). 15 ml of Ficoll Buffer (GE healthcare, 17-5442-02) was added into PBMC-Separation Tube Sepmate-50 (STEMCELL Technology, Cat. No. 86450), and added with the mixture of blood and PBS. The pellet was resuspended in PBS after centrifugation. The resuspended cells were counted, and 10 µl of the suspension was taken out, added with 10 µl of 0.1% Trypan blue and mixed well. The cells were counted and survival rate was calculated.

### Example 3. Purification of T cells

PBMC cells were centrifuged at 300 g for 5 minutes, the supernatant was discarded, and the cells were resuspended by adding the corresponding amount of PBS buffer (containing 2 mM EDTA and 1% fetal calf serum), and the cell density was adjusted to 5 × 10⁷/ml. Human T cells were purified by using EasySep™ Human T Cell Enrichment Kit (STEMCELL, Cat. No. 17951). First, 50 µl/ml of Cooktail Protease Inhibitor (Biotool, B14001a) was added to PBMC suspension, mixed well and allowed to stand at room temperature for 10 minutes. Then, 50 µl/ml of EasySep™ D Magnetite Particles (STEMCELL, Cat. No. 19550) was added and mixed well, and allowed to stand at room temperature for 5 minutes. The cell suspension was added into a 5 ml flow tube and placed in magnetic pole for 5 minutes. The cell suspension was quickly decanted, PBS buffer was supplemented into the flow tube and resuspended, and the steps were repeated for 3 times. The resulting cell suspension was centrifuged at 300 g for 5 minutes, the supernatant was discarded, the cell pellet was resuspended in VIVO-15 medium (LONZA), and the density was adjusted to 1 × 10⁶ /ml. Then rIL-2 (R&D, Cat. No.: 202-IL-050) was added at a concentration of 100 IU/ml, and cultured in cell culture incubator at 37 °C.

### Example 4. Activation of T cells

Anti-CD3/anti-CD28 magnetic beads (Life Technology, Cat. No. 11131D) were resuspended in PBS buffer (containing 2 mM EDTA and 1% fetal bovine serum), then placed in a magnetic pole for 2 minutes, and the supernatant was discarded. The above steps were repeated for 4 times. The magnetic beads were washed, added with the purified T cells at a ratio of 1:1 (by magnetic bead number), mixed well, and cultured at 37 °C for 3 days. After 3 days, the magnetic beads were taken out, and the target cells were resuspended several times with a pipette. The cell suspension was placed in a magnetic pole, allowed to stand for two minutes and the magnetic beads on the wall of the tube were discarded.

### Example 5. Infection of T cells with pLVX-EF1-CD19 CAR virus

### (1) Packaging and concentration of pLVX-EF1-CD19 CAR Lentivirus:

Lentiviral plasmid pLVX-EF1-CD19 CAR and two helper plasmids, pCMV-dR8.91 (purchased from addgene) and pCMV-VSV-G (purchased from addgene), were extracted by using Maxi Plasmid Extraction Kit, Tiangen. One day before transfection, 293T cells (purchased from ATCC) were grown to full confluence in 75 cm² culture dishes, and passaged at 1:3 with 15 ml of culture medium per dish. Transfection was carried out according to Lipo3000 (Life technologies, Cat. No. L3000008). An exemplary transfection system is as follows:

| Transfection system 1 | Transfection system 2 |
|---|---|
| CART 19-N2: 7.5µg | CART 19-N2: 7.5µg |
| pCMV-dR8.91: 5.625µg | pCMV-dR8.91: 5.625µg |
| pCMV-VSV-G: 1.875µg | pCMV-VSV-G: 1.875µg |
| Opti-MEM (Gibco): 700µl | Opti-MEM (Gibco): 700µl |
| P3000: 30µl | Lipofectamine: 36µl |

The System 1 was well mixed with System 2, allowed to stand for 5 minutes, then the two systems were mixed again and allowed to stand for 10 minutes. 293T cells were carefully added. Fresh medium was replaced for the medium after 6 hours. After 48 hours, the culture medium was collected and stored at 4 °C, and 15 ml of new Opti-MEM medium (Gibco, Cat. No. 51985034) was added again. The supernatant was collected again after 24 hours. The resulting virus supernatant was filtered through 0.45 µm filter, centrifuged in an ultracentrifuge tub at 50000 g for 2 hours and 45 minutes at 4 °C. The supernatant was carefully removed thoroughly, and the white visible virus pellet was resuspended in PBS buffer at a volume of 1% of the supernatant. The resuspended virus was placed at 4 °C for about 30 minutes for dissolution. After complete dissolution, the virus solution was aliquoted and stored in a freezer at -80°C.

### (2) Infection of T Cells with pLVX-EF1-CD19 CAR Lentivirus

The human primary T cells were activated with anti-CD3/anti-CD28 magnetic beads for 1 day, resuspended, placed in a magnetic pole for two minutes, and the cell suspension was taken out for cell counting. Approximately 1 x 10⁷ cells were centrifuged at 300 g for 5 minutes, the medium was discarded, added with 1 ml of fresh medium and resuspended. The concentrated lentivirus was added to adjust MOI to 5, mixed, centrifuged at 2000g for 90 minutes at 32°C, the supernatant was discarded, and new medium Lonza X-VIVO 15 (containing 100IU/ml rIL-2, purchased from R&D, Cat. No.: 202-IL-050) was added to adjust the cell density to 1 × 10⁶/ml, and added with the newly isolated anti-CD3/anti-CD28 magnetic beads after resuspension. The culture was continued in an incubator at 37 °C to obtain CD19 CAR-T cells (CART19): CART19-N2. The magnetic beads were removed by using magnetic separator before *in vitro* and *in vivo* tests.

CD19 CAR-T cells: CART19-N1, CART19-N3, and CART19-N4 were obtained in a similar way.

### Example 6. Knockout of TCR, B2M and PD-1 genes in CART19 cells

### (1) crRNA design

Appropriate target regions were selected on the basis of the nucleotide sequences of TRAC, B2M and PD-1, and crRNAs with 17-20 nt in length were designed. The resulting crRNAs were ligated with tracrRNA sequence corresponding to the Cas9 protein to form sgRNAs, wherein the crRNAs were located at 5'-end of the tracrRNA. CrRNAs with high knockout efficiency and low off-target rate were selected by experiments. Some crRNA sequences selected are as follows:

**Table 1 crRNAs against the target gene**

| Target gene | crRNA name | sequence | SEQ ID NO |
|---|---|---|---|
| TRAC | crRNA-1 | CAAAUGUGUCACAAAGUA | 37 |
| | crRNA-2 | AAAACUGUGCUAGACAUG | 38 |
| | crRNA-3 | UCAAGAGCAACAGUGCUG | 39 |
| | crRNA-4 | CACCUUCUUCCCCAGCCC | 40 |
| | crRNA-5 | GAAUAAUGCUGUUGUUGA | 41 |
| | crRNA-6 | GAUUUAGAGUCUCUCAGC | 42 |
| | crRNA-7 | ACGGCAGGGUCAGGGUUC | 43 |
| | crRNA-8 | GUUCCUGUGAUGUCAAGC | 44 |
| | crRNA-9 | UCAAAACCUGUCAGUGAU | 45 |
| | crRNA-10 | GAAUCCUCCUCCUGAAAG | 46 |
| | crRNA-11 | GGUACACGGCAGGGUCA | 47 |
| | crRNA-12 | GAGAAUCAAAAUCGGUGAAU | 48 |
| B2M | crRNA-13 | GUAGCGCGAGCACAGCUA | 49 |
| PD-1 | crRNA-14 | CGACUGGCCAGGGCGCCUGU | 50 |
| | crRNA-15 | GUGCUACAACUGGGCUGG | 51 |
| | crRNA-16 | GGCGCCCUGGCCAGUCGUCU | 52 |

The Cas9 protein is derived from *Streptococcus Pyogenes* (Cas9 Nuclease NLS, *S. pyogenes* (BioLabs)), the corresponding tracrRNA sequence is shown in SEQ ID NO: 53, and the amino acid sequence of Cas9 protein (including NLS) is shown in SEQ ID NO: 54.

GUUUUAGAGCUAGAAAUAGCAAGUUAAAAUAAGGCUAGUCCGUUAUCA
ACUUGAAAAAGUGGCACCGAGUCGGUGCUUUU
SEQ ID NO: 53

Amino acid sequence of Cas9 protein (including NLS):

MDKKYSIGLAIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLV
EEDKKHERHPIFGNIVDEVAYHEKYPTIYHLRKKLVDSTDKADLRLIYLALAH
MIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSK
DTYDDDLDNLLAQIGDQYADLFLAAKNLSDAILLSDILRVNTEITKAPLSASMI
KRYDEHHQDLTLLKALVRQQLPEKYKEIFFDQSKNGYAGYIDGGASQEEFYK
FIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVD

KGASAQSFIERMTNFDKNLPNEKVLPKHSLLYEYFTVYNELTKVKYVTEGMR
KPAFLSGEQKKAIVDLLFKTNRKVTVKQLKEDYFKKIECFDSVEISGVEDRFN
ASLGTYHDLLKIIKDKDFLDNEENEDILEDIVLTLTLFEDREMIEERLKTYAHLF
DDKVMKQLKRRRYTGWGRLSRKLINGIRDKQSGKTILDFLKSDGFANRNFM
QLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPV
ENTQLQNEKLYLYYLQNGRDMYVDQELDINRLSDYDVDHIVPQSFLKDDSID
NKVLTRSDKNRGKSDNVPSEEVVKKMKNYWRQLLNAKLITQRKFDNLTKAE
RGGLSELDKAGFIKRQLVETRQITKHVAQILDSRMNTKYDENDKLIREVKVIT
LKSKLVSDFRKDFQFYKVREINNYHHAHDAYLNAVVGTALIKKYPKLESEFV
YGDYKVYDVRKMIAKSEQEIGKATAKYFFYSNIMNFFKTEITLANGEIRKRPLI
ETNGETGEIVWDKGRDFATVRKVLSMPQVNIVKKTEVQTGGFSKESILPKRNS
DKLIARKKDWDPKKYGGFDSPTVAYSVLVVAKVEKGKSKKLKSVKELLGITI
MERSSFEKNPIDFLEAKGYKEVKKDLIIKLPKYSLFELENGRKRMLASAGELQ
KGNELALPSKYVNFLYLASHYEKLKGSPEDNEQKQLFVEQHKHYLDEIIEQIS
EFSKRVILADANLDKVLSAYNKHRDKPIREQAENIIHLFTLTNLGAPAAFKYFD
TTIDRKRYTSTKEVLDATLIHQSITGLYETRIDLSQLGGDPKKKRKVMDK
SEQ ID NO: 54

### (2) in vitro transcription of sgRNA:

First, PCR amplification was performed with sgRNA as a template. The PCR amplification system was shown in the following table.

**Table 2 PCR amplification system with sgRNA as a template**

| Reaction system (20µl) | | PCR condition | |
|---|---|---|---|
| plasmid | 1 µl (0.5pg) | 98°C | 30sec |
| 5×HF buffer | 4µl | 98°C | 10sec |
| dNTP (10mM) | 0.4µl | 60°C | 25sec |
| primer-F(10µm) | 0.4 µl | 72°C | 2min |
| primer-R(10µm) | 0.4 µl | 72°C | 10min |
| Phusion | 0.2 µl | 38 times | |
| H₂O | 13.6 µl | 4°C | 10min |

### PCR product recovery:

See manual available from the Common DNA Product Purification Kit DP214, Tiangen. DNAs were obtained for *in vitro* transcription of sgRNAs. The transcription of sgRNAs was perfromed with Ambion *in vitro* Transcription Kit MEGAshortscript™ Kit (cat#AM1354). See manual available from Ambion MEGAclear™ Kit (cat#AM1908). The resulting sgRNAs were purified and detected by spectrophotometer and denaturing agarose gel electrophoresis. All sgRNAs were qualified and aliquoted for use.

### (3) Knockout of TRAC, B2M and PD-1gene was performed in CART19 cells by transfection of CRISPR-Cas9 via electroporation

The resulting CART19 cells were subjected to electroporation by using LONZA 4D Electroporator (this method can also be used to knock out primary T cells), with the kit: P3 Primary Cell 4D-Nucleofector™ X kit (LONZA, V4XP3024).

First, the following electroporation system was prepared:

**Table 3 Electroporation system**

| component | TCR, B2M, PD-1 single knockout system | TCR/B2M Double knockout system | TCR/B2M/PD-1triple knockout system |
|---|---|---|---|
| Nucleofector buffer | 10µl | 10µl | 10µl |
| Cas9 protein | 30µg | 30µg | 30µg |
| sgRNA | 4µg | 4µgTRAC+4µg B2M | 4µgTRAC+4µg B2M+PD-1 |

The above electroporation system was mixed and incubated at room temperature for 10 minutes. The CAR-T cells were activated for three days, and then the anti-CD3/anti-CD28 magnetic beads were removed with magnetic poles. 5×10⁶ cells/tube were centrifuged at 300g for 5 minutes, the supernatant was completely removed, the cell pellets were added with the pre-incubated electroporation system, together with 72 µl of Nucleofector buffer and 18 µl of Supplyment buffer, mixed, and added into a 100 µl LONZA electroporation cuvette. The cuvette was placed in LONZA-4D Electroporator and electroporation was performed according to E0-115 procedure. After the electroporation was finished, the electroporation cuvette was allowed to stand at room temperature for 5 minutes. The cells were transferred from the electroporation cuvette to pre-warmed X-VIVO-15 medium, cell density was adjusted to 1 × 10⁶ /ml, and cultured at 37 °C.

### Example 7. Screening of TCR and B2M negative CART19 cells

CART19 cells were cultured for 10 days after the knockout of TRAC by CRISPR-Cas9, and TCR-negative cells were enriched. All cells were first centrifuged at 300g for 5 minutes and washed twice with PBS buffer (containing 2 mM EDTA and 1% fetal bovine serum). The cell density was adjusted to 1 × 10⁷ cells/ml, then 100 µl/ml of Biotin-TCR antibody (purchased from Miltenyi Germany, Cat. No. 130-109-918) was added, and incubated at 4 °C in the dark for 10 minutes. After Centrifugation at 300g for 5 minutes, the cells were washed once with PBS buffer, and the cell density was adjusted to 1×10⁷ cells/ml. 50 µl/ml anti-Biotin Microbeads (purchased from Miltenyi, Cat. No. 130-090-485) were added and placed at 4°C in the dark for 15 minutes. Centrifugation was performed at 300g for 5 minutes, the cells were washed once with PBS buffer, and resuspended in 500 µl buffer. The LD column (purchased from Miltenyi, Cat. No. 130-042-901) was placed in magnetic poles, rinsed once with 2ml PBS and loaded with 500 µl cell suspension. The target cells were collected after flowing through the LD column, and the column was then washed twice with 2 ml of PBS buffer. The collected target cell suspension was centrifuged at 300g for 5 minutes and resuspended in pre-warmed medium, resulting in CART19 cells with knockout of TCR, i.e., UCART19^{TCR-/-}.

The enriched TCR-negative cells were washed twice with PBS buffer (containing 2 mM EDTA and 1% fetal bovine serum), the cell density was adjusted to 1×10⁷ cells/ml, then 100 µl/ml of Biotin-B2M antibody (purchased from Miltenyi, Cat. No. 130-090-485) was added, and incubated at 4°C in the dark for 10 minutes. After centrifugation at 300g for 5 minutes, the cells were washed once again with PBS buffer, the cell density was re-adjusted to 1×10⁷cells/ml, 50µl/ml anti-Biotin Microbeads (purchased from Miltenyi, Cat. No. 130-090-485) was added and placed at 4°C in the dark for 15 minutes. Centrifugation was performed at 300g for 5 minutes, the cells were washed once with PBS buffer, and resuspended in 500 µl buffer. The LD column (purchased from Miltenyi, Cat. No. 130-042-901) was placed in magnetic poles, rinsed once with 2ml PBS and loaded with 500 µl cell suspension. The target cells were collected after flowing through the LD column, and the column was then washed twice with 2 ml of PBS buffer. The collected target cell suspension was centrifuged at 300g for 5 minutes and resuspended in pre-warmed medium for culture, resulting in CD19-CART cells with double knockout of both TCR and B2M, i.e., UCART19^{TCR-/-B2M-/-}. The CD19-CART cells with triple knockout of TCR, B2M and PD-1 (i.e., UCART^{TCR-/-B2M-/-PD-1-/-}) were screened in a similar way, and the identification of PD-1 knockout cells was shown in Test Example 5.

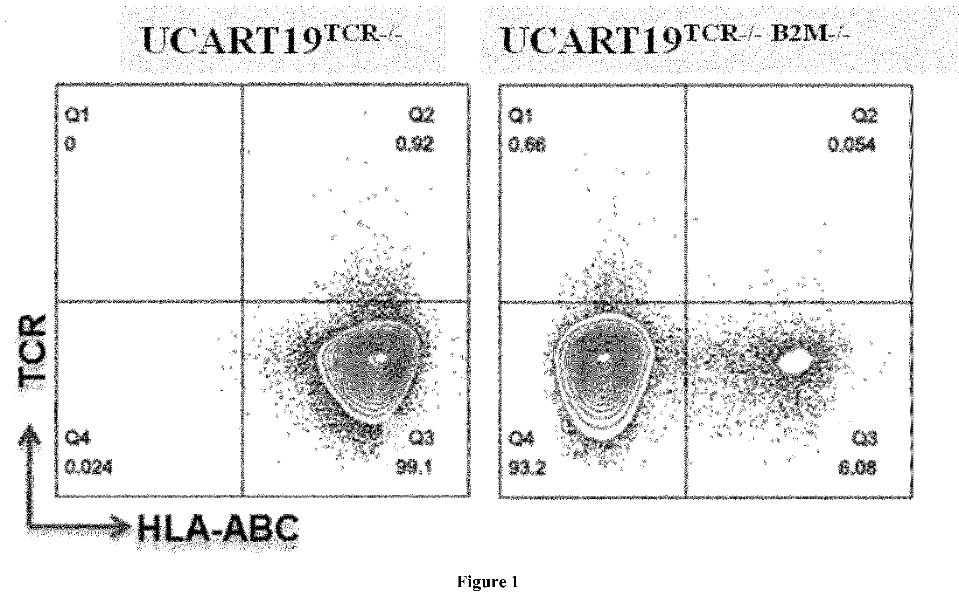

The enriched cells were sorted using BD Sorting Flow Cytometer, and the results of the purity of UCART^{TCR-/-} and UCART^{TCR-/-B2M-/-} were shown in Fig.1. From the results, it can be seen that UCART^{TCR-/-} cells obtained by the above screening method have a purity up to 99% or more, and the UCART^{TCR-/-B2M-/-} cells have a purity up to 90% or more.

### Test Examples

### Test Example 1. Determination of efficiency of pLVX-EF1-CD19 CAR lentivirus in transfecting T cells

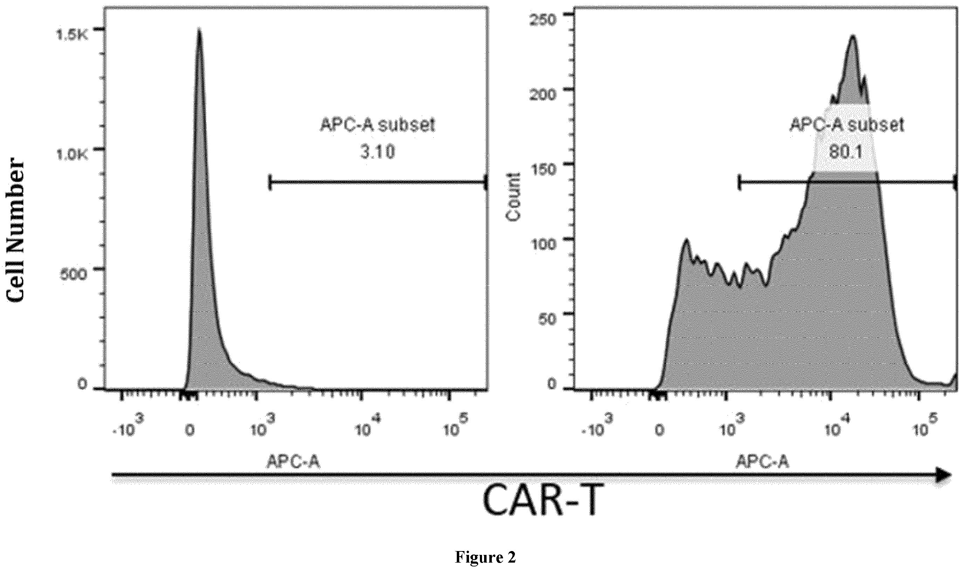

The T cells were transfected with the lentiviral plasmid pLVX-EF1-002B with MOI=5, mixed well, and centrifuged at 1600 g, 32 °C for 1.5 hours. After the centrifugation, the virus supernatant was carefully aspirated, and the density was adjusted to 5 × 10⁵ cells/ml with pre-warmed X-VIVO medium (containing 100 U/ml rhIL-2), and cultured in an incubator with carbon dioxide at 37 °C. Four days later, the transfection efficiency was measured by flow cytometry by using Biotin-Protein L (Genscript Biotech., Cat. No. M00097), and the results were shown in Fig. 2.

From the results, it can be seen that the transfection efficiency was up to 80%, indicating that the above lentivirus transfection method can be used for the preparation of CART cells.

### Test Example 2. Analysis of TCR knockout efficiency with various crRNA CRISPR-Cas9

The knockout efficiency was compared in the experiments by using the crRNAs against TRAC as designed in Example 6. After *in vitro* transcription, the resulting sgRNA and the Cas9 protein was electroporated into the activated primary T cells, and the expression of extracellular TCR protein was detected by flow cytometry 48 hours later. All the designed crRNAs can knock down the TRAC gene to varying degrees, and crRNA-11 has the highest knockout efficiency (data not shown).

### Test Example 3. Comparative analysis of different delivery systems

Three delivery systems: plasmid, mRNA and RNP (protein RNA complex).

CrRNA-11 is against TRAC, and large quantities of plasmids were extracted with Maxi Plasmid Extraction Kit, Tiangen.

*In vitro* transcription of Cas9 mRNA: First, a DNA template containing T7 promoter was obtained by PCR with T7 primer, and then Cas9 mRNA was obtained by *in vitro* transcription with T7 *in vitro* Transcription Kit, Ambion (Thermo, AM1345).

sgRNA and Cas9 protein complex were prepared according to Example 6.

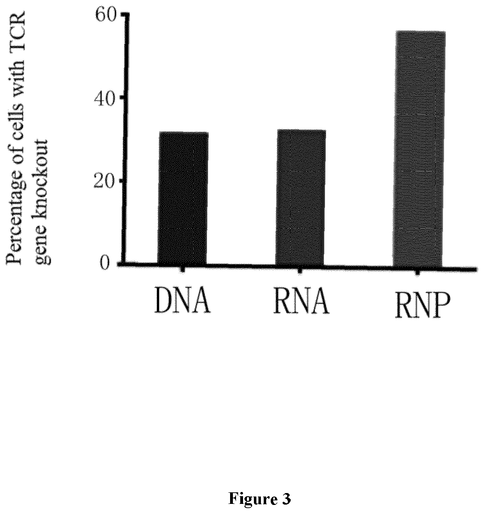

5×10⁶ Jurkat cells (purchased from ATCC) were centrifuged to remove the supernatant, and then were transfected with three different delivery systems via Electroporation System Neon MPK5000, Invitrogen. After 48 hours, 0.5×10⁶ cells were washed twice with PBS buffer, and were resuspended in 100 µl of buffer, added with 10 µl of PE-TCR antibody (eBioscience, Cat. No. H57-597), mixed well, and incubated at 4°C for 30 minutes. After washing once with PBS buffer, the cells were resuspended by adding 500 µl of buffer, and the expression level of TCR was detected by Flow Cytometry. The results were shown in Fig. 3.

The results indicate that the delivery system with sgRNA and Cas9 protein complex (RNP) can achieve the highest knockout efficiency and the system is a preferred method.

### Test Example 4. Random N-oligo or fish sperm DNA increases the TRAC knockout efficiency by CRISPR-Cas9

When gene knockout was performed using the RNP delivery system, RNP was mixed with random sequence of N-oligo (oligodeoxyribonucleic acid) or fish sperm DNA (R&D, Cat. No. 9610-5-D) and then was transfected via electroporation.

An exemplary N-oligo sequence:

TCATGTGGTCGGGGTAGCGGCTGAAGCACTGCACGCCGTACGTCAGGGTG
GTCACGAGGGTGGGCCAGGGCACGGGCAGCTTGCCGGTGGTGCAGATGAA
CTTCAGGGTCAGCTTGCCGTAGGTGGC
(SEQ ID NO: 55)

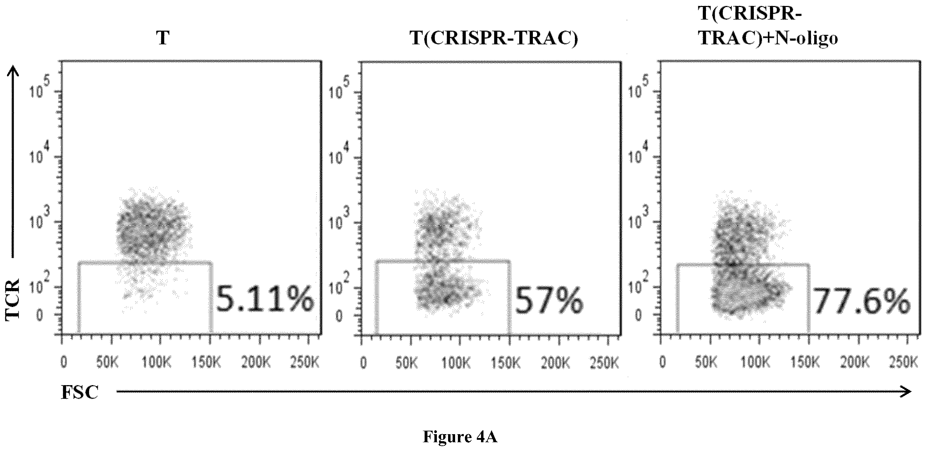

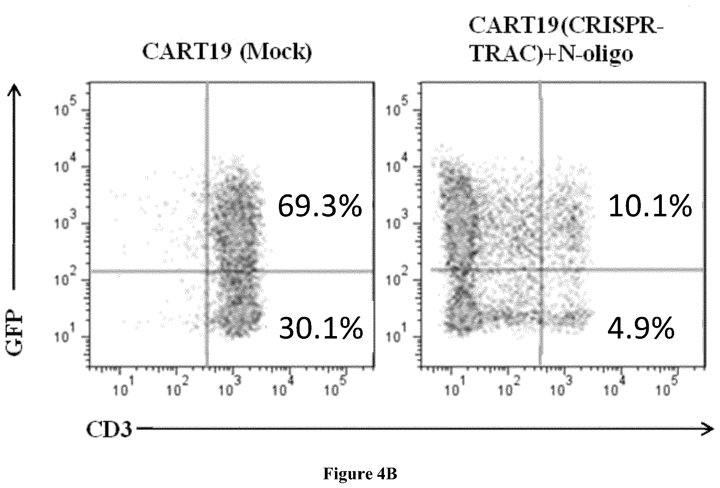

On the basis of Example 5 (3), 100-200 nM of N-oligo DNA was further added into the RNP complex, and the N-oligo DNA was Page Grade. The effect of N-oligo on TRAC knockout efficiency by CRISPR-Cas9 was shown in Figure 4A and Figure 4B. The results show that N-oligo can effectively increase TRAC gene knockout efficiency by CRISPR-Cas9 in both T cells and CART19 cells.

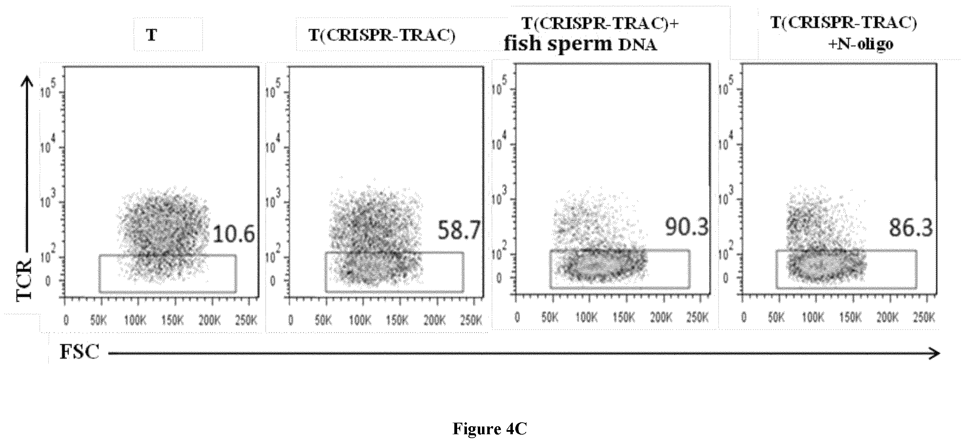

On the basis of Example 5 (3), 100-200 nM fish sperm DNA fragment was further added into the RNP complex, and the effect of the fish sperm DNA fragment on TRAC knockout efficiency was shown in Figure 4C. The results showed that the TARC knockout efficiency was 90.3% by adding fish sperm DNA, while the TRAC gene knockout efficiency was 86.3% by adding N-oligo, indicating that the addition of fish sperm DNA fragment can improve the knockout efficiency of TRAC gene.

### Test Example 5. Detection of B2M and PD-1 knockout efficiency in T cells

A number of crRNAs were designed in a similar way, and after a comparative analysis, the one with the highest knockout efficiency and the lowest off-target rate was selected for B2M gene knockout. The B2M and/or PD-1 gene of T cells were knocked out using RNP delivery system and N-oligo according to the same method as that in Example 5 (3).

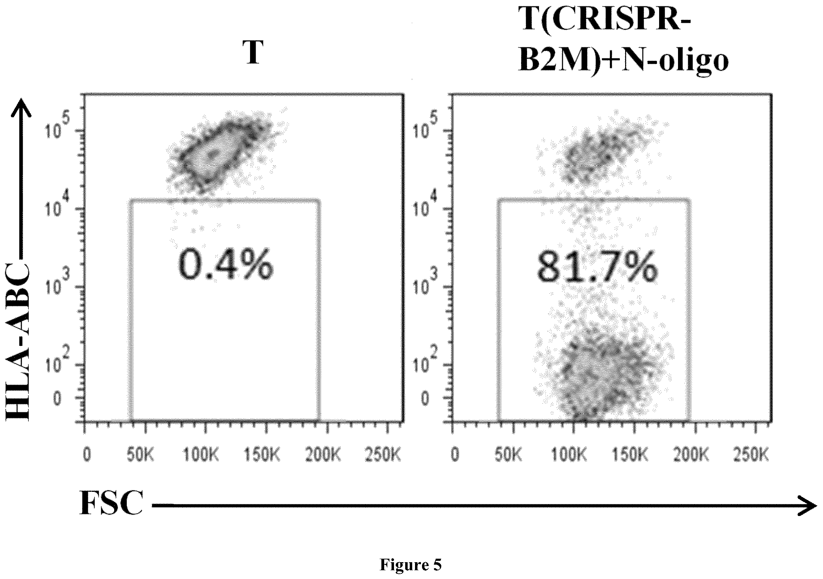

For B2M protein, the B2M gene expression was closely correlated with the display of HLA-ABC on the cell membrane, and the B2M gene knockdown efficiency was detected by using APC-HLA-ABC antibody (eBioscience, Cat. No. 12-9983-71). The results (Figure 5) showed that the B2M gene knockout efficiency was greater than 80%.

For PD-1 gene, RNP and N-oligo were mixed and transfected into the cells via electroporation, 48 hours later, 1×10⁶ cells were washed twice with PBS buffer and the supernatant was completely aspirated. T7E1 experiment was performed according to the manual provided in GeneArt® Genomic Cleavage Detection Kit (Thermo Fisher). The knockout efficiency was calculated by comparing the optical density of PCR fragment corresponding to the intact wild-type gene to the optical density of two small fragments generated after mutation. The specific calculation formula was as follows: $Knockout efficiency = 1 - \left[{\left(1-cleavage percentage\right)}^{1 / 2}\right] ,$ wherein the cleavage percentage = the sum of optical density of the small fragments after cleavage / the sum of optical density of the small fragments after cleavage + the optical density of the un-cleaved fragment).

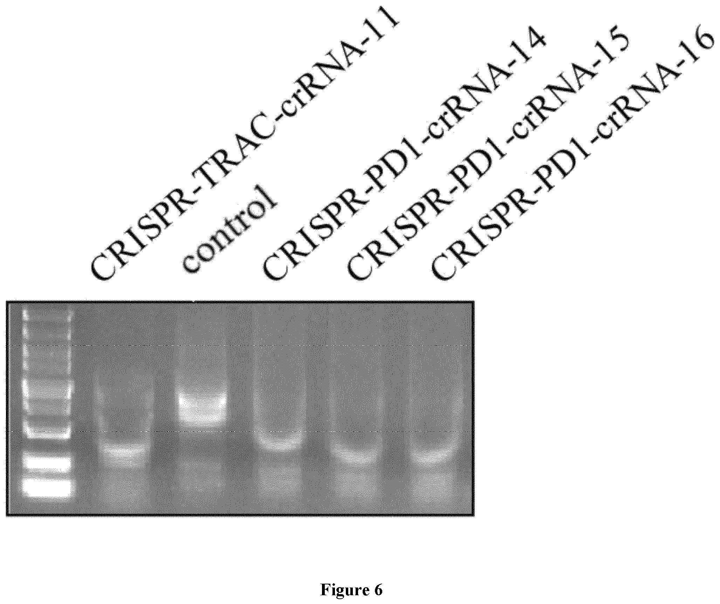

The results (shown in Figure 6) showed that the selected three crRNAs against PD-1 can effectively knock out the PD-1 gene, all of them had the knockout efficiency of more than 80%, and crRNA-16 had the highest knockout efficiency.

### Test Example 6. Analysis of gene mutations caused by CRISPR-Cas9

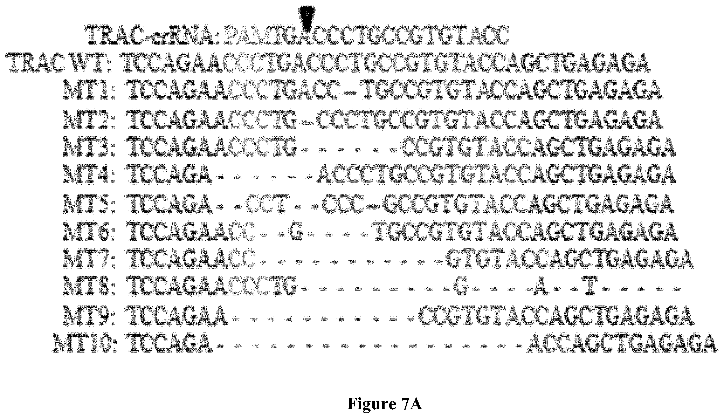

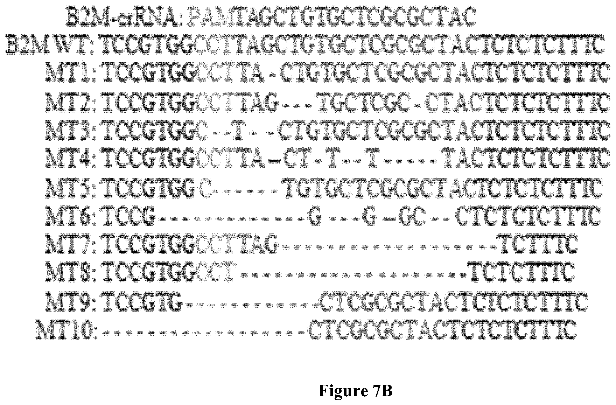

First, primers were designed near the target sites of the TRAC, B2M and PD-1 genes. For T cells, TRAC, B2M and PD-1 were knocked out by using RNP+N-oligo or fish sperm DNA fragments based on the CRISPR-Cas9 system. Genomic DNAs were extracted from 1×10⁶ normal T cells and gene knockout T cells, respectively. The resulting PCR product DNA fragment was ligated with the T blunt end vector (pEASY-Blunt Simple Cloning Kit, Beijing TransGen Biotech Co., Ltd., Cat. No. CB111-01). TOP10 competent cells were transformed with the ligation product and plated onto Amp-resistant solid plates. On the next day, the resulting clones were sequenced, and at least 30 clones per plate were tested. The obtained sequencing results were compared with wild-type sequences. The results were shown in Figures 7A-7B, in which the analysis results of PD-1 mutation were not shown and MT was the clone number.

From the results, it can be seen that for TRAC, B2M and PD-1, gene mutations were observed in the genomic DNAs corresponding to each crRNA, respectively, indicating that the TRAC, B2M and PD-1 genes were actually knocked out at the gene level.

### Test Example 7. Off-target analysis

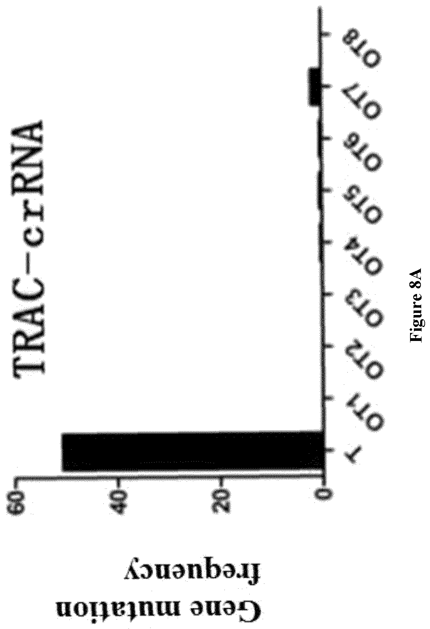

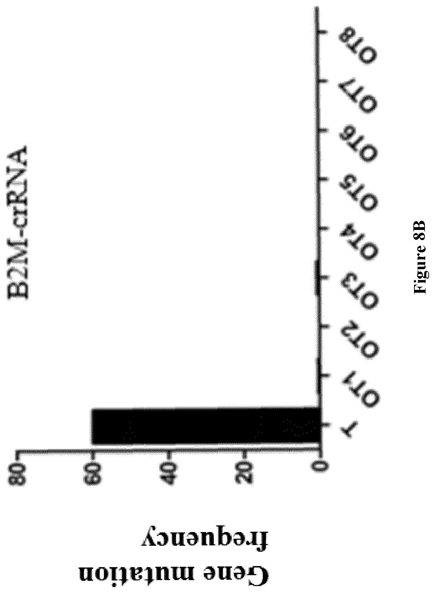

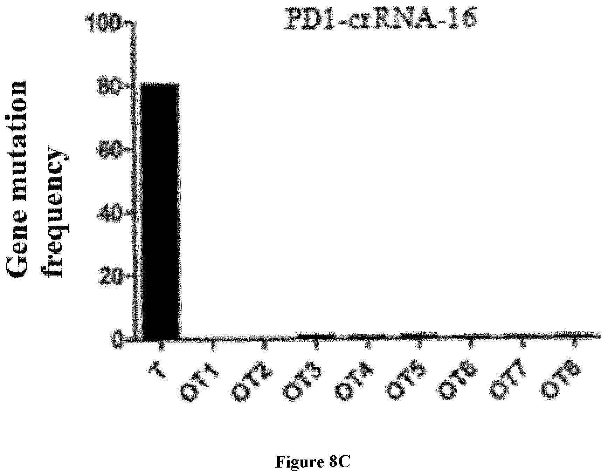

The off-target sites which might occur were predicted for the designed crRNAs (crRNA-11, crRNA-13 and crRNA-16) on http://crispr.mit.edu/. For each of TRAC, B2M and PD-1, 8-9 potential off-target sites (OT1-OT9) were selected and primers against these potential off-target sites were designed for PCR amplification. The peak map sequencing results of the off-target sites of the genomic DNA in gene knockout cells and the that of control (target gene TRAC, B2M or PD-1) were subjected to TIDE alignment analysis on https://tide.nki.nl/ with. The results (shown in Figures 8A-8C) showed the off-target rates were extremely low with the knockout method and the selected crRNAs against TRAC, B2M and PD-1.

### Test Example 8. Analysis of the effect of TCR knockout on cell signaling pathway and killing activity

96-well plates were coated with CD3 antibody (5 µg/ml) or CD28 antibody (5 µg/ml), 100 µl was added into per well, and coated at 37 °C for two hours. The plates were then washed twice with PBS, added with TCR-negative T cells and normal T cells respectively, at a cell density of 1×10⁶cells/ml. After incubation at 37°C for 24 hours, cells were stained with CD25 and CD69-antibodies, and the expression of CD25 and CD69 was detected by Flow Cytometry.

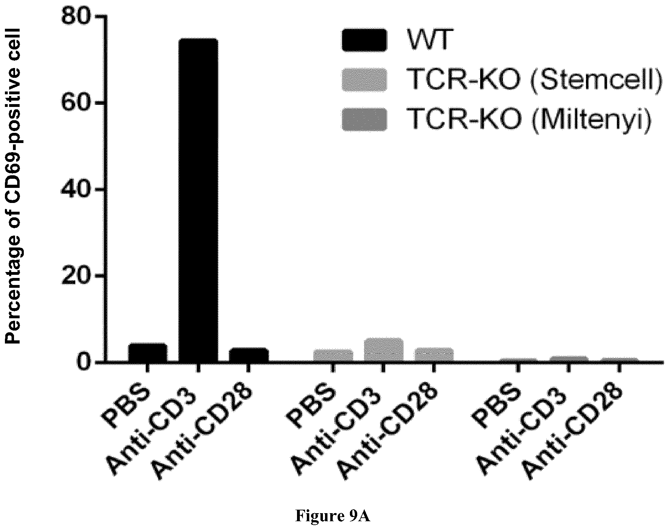

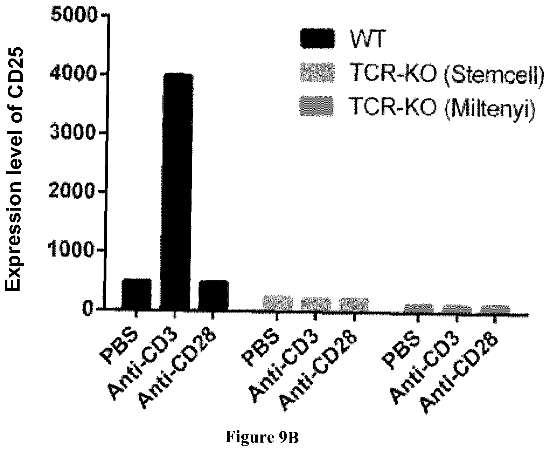

The results (shown in Figures 9A-9B) indicated that T cells, after TRAC gene knockout, cannot be induced to express CD25 and CD69 by CD3 or CD28 antibody, i.e., CATR cells with knockout of TCR cannot be activated by CD3 antibody or CD28.

### Test Example 9. Analysis of in vitro cytotoxicity of CD19-CART cells

Materials: K562, Raji and Daudi cells were purchased from ATCC, Nalm6 was purchased from BD, Human IL-2 ELISA Kit II (Cat. No. 550611) and Human IFN-γ ELISA Kit II (Cat. No. 550612) were purchased from BD, and anti-human CD107a (Cat. No. 555801) antibody was purchased from BD.

### Method and result:

### 9.1 In vitro killing of K562-CD19 cells by CART cells

The K562-CD19 cells were constructed as follows: CD19 antigen was designed with reference to the NCBI NM_001770.5 sequence, and was constructed into pLVX-EF1-CD19 plasmid. K562 cells were transfected and a single clone was picked up to obtain a K562-CD19 cell line.

The target cells (K562-CD19 cells and K562 cells) were adjusted to a density of 5×10⁵/ml, 100 µl of which were plated into a 96-well round bottom plate, added with effector cells at a range of ratio of effector cell (CART19) to target cell (E:T ratio=30:1 to 0.3:1) or at a specific ratio (30:1), and mixed by pipetting. Centrifugation was performed at 1000 rpm for 2 min, cell lysis was detected after incubation in an incubator for 4 h. 150 µl of the supernatant was collected and frozen at -20°C for subsequent experiments. 50 µl of supernatant was maintained in each well, added with 100µl of detection solution (Steady-Glo® Luciferase Assay System, E2520, Promega), incubated at room temperature for 5 min, and 100 µl was pipetted into a black plate. The bioluminescence value was measured by using a microplate reader, and the killing rate was calculated. $Killing rate = \left(Reading of Target only-Reading of Target plus Effector\right) / Reading of Target only .$

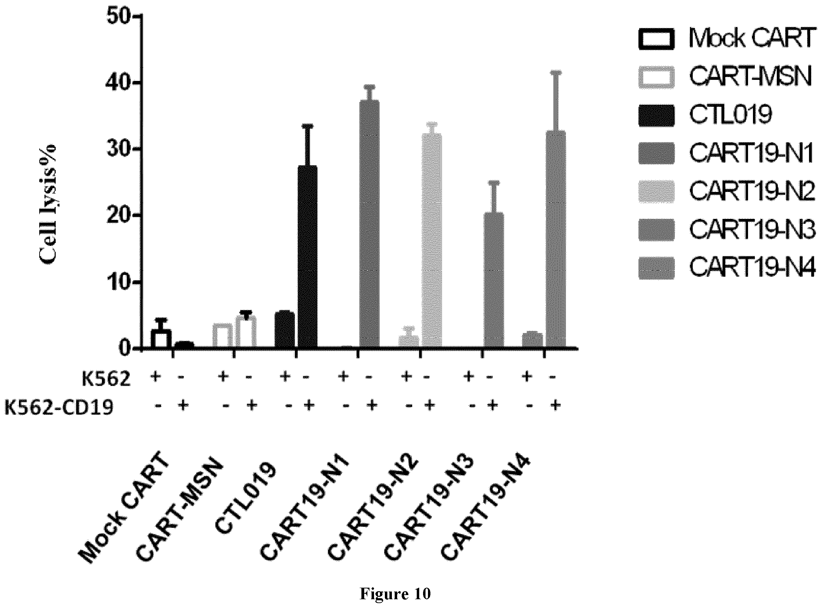

The killing results were shown in Figure 10, in which Mock CART indicated T cells containing a vehicle, and CART-MSN indicated CART cells against mesothelin. It can be seen from the results that CART19 cells had significant killing effect on K562-CD19 cells, but had no killing effect on K562 cells (CD19 negative cells), indicating that CART19 cells can specifically kill CD19-positive cells.

### 9.2 In vitro killing of Raji cells by CART cells

The target cells (Raji cells) were adjusted to a density of 5×10⁵/ml, 100 µl of which were plated into a 96-well round bottom plate, added with effector cells (CART19) at a range of ratio of effector cell to target cell (E:T ratio=30:1 to 0.3:1; 30:1, 10:1, 3:1, 1:1 and 0.3:1), and mixed by pipetting. CTL-019 cells were used as positive control and centrifuged at 1000 rpm for 2 min, incubated in an incubator for 4 h, and then cell lysis was detected. 150 µl of the supernatant was collected and frozen at -20°C for subsequent experiments. 50 µl of supernatant was maintained in each well, added with 100µl of detection solution (Steady-Glo® Luciferase Assay System, E2520, Promega), incubated at room temperature for 5 min, and 100 µl was pipetted into a black plate. The bioluminescence value was measured by using a microplate reader, and the killing rate was calculated. $Killing rate = \left(Reading of Target only-Reading of Target plus Effector\right) / Reading of Target only .$

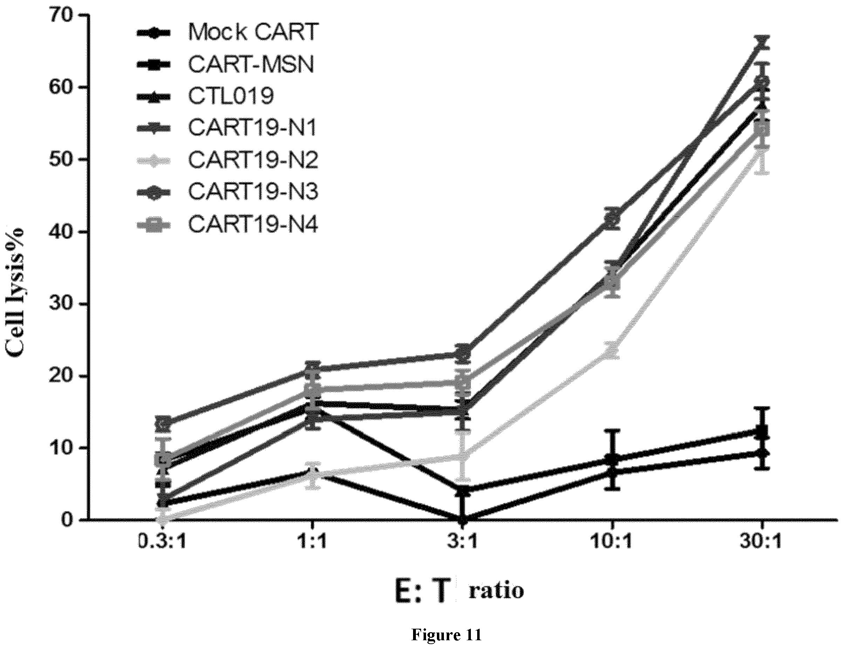

The measured killing results (Fig. 11) showed that CART19 cells had significant killing effect on leukemia tumor cells in a dose-dependent manner, and its killing ability was comparable to that of the positive control CTL-019.

### Test Example 10. Release of cytokines during killing of target cells by CART19 cells in vitro

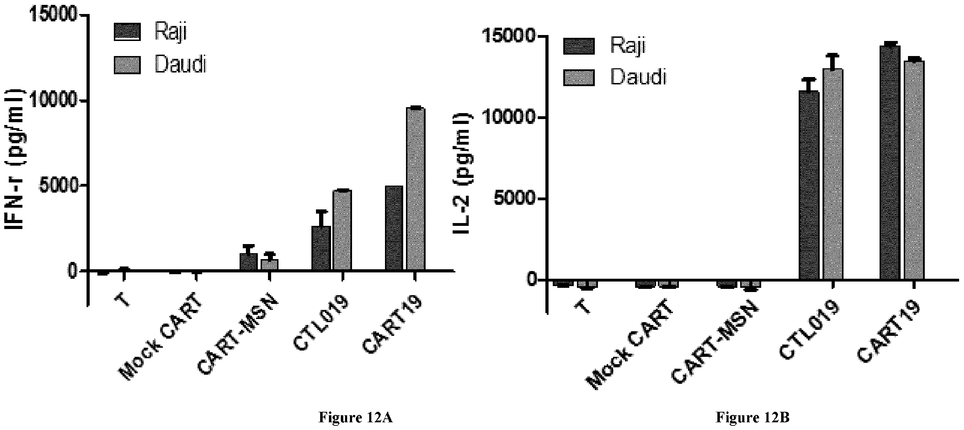

CART19-N2 cells were incubated with Raji tumor target cells and Daudi target cells for 4 h (E:T=5:1), respectively, and 150 µl of supernatant was taken for the measurement of the concentration of IL-2 and IFN-γ in the supernatant by using BD Human IL-2 ELISA Kit II (Cat. No. 550611), and Human IFN-γ ELISA Kit II (Cat. No. 550612). Results were shown in Fig. 12.

The results showed that both CTL-019 and CART19-N2 cells produced a large number of cytokines IFN-γ and IL-2 when being co-cultured with target cells, indicating that both CTL-019 and CART19-N2 can exhibit characteristics of killing T cells. Moreover, CART19-N2 cells released more IFN-γ compared to CTL-019 cells, indicating that CART19-N2 cells have stronger killing effect on target cells.

### Test Example 11. Detection of ability of CART19 and UCART19 cells in killing hematologic cancer cell in vitro

Target cells (Daudi, Raji and Nalm6 cells, all purchased from ATCC) were adjusted to a cell density of 5×10⁵/ml, 100 µl was plated into a 96-well round bottom plate, then added with effector cells: T cells, CART19-N2, UCART^{TCR-/-} and UCART^{TCR-/-B2M-/-} at a range of ratio of effector cell to target cell (E:T ratio=30: 1 to 1: 1; 30:1, 10:1, 3:1, and 1:1), and mixed by pipetting. Centrifugation was performed at 1000 rpm for 2 min, cell lysis was detected after incubation in an incubator for 4 h. 150 µl of the supernatant was collected and frozen at -20°C for subsequent experiments. 50 µl of supernatant was maintained in each well, added with 100µl of detection solution (Steady-Glo® Luciferase Assay System, E2520, Promega), incubated at room temperature for 5 min, and 100 µl was pipetted into a black plate. The bioluminescence value was measured by using a microplate reader, and the killing rate was calculated. $Killing rate = \left(Reading of Target only-Reading of Target plus Effector\right) / Reading of Target only .$

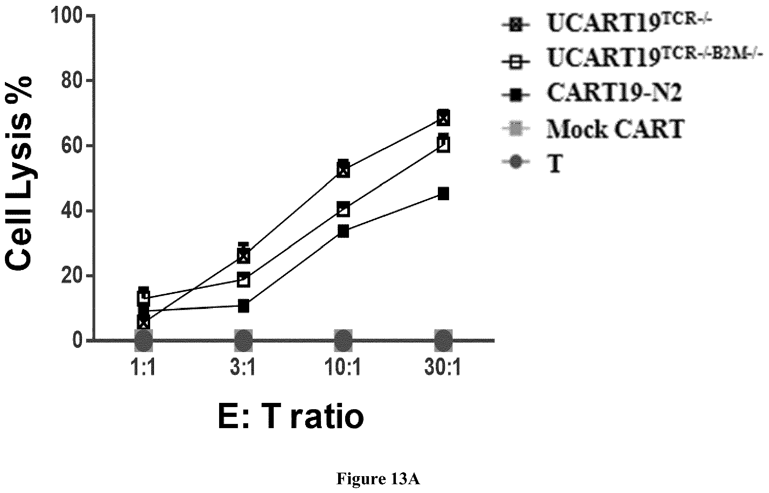

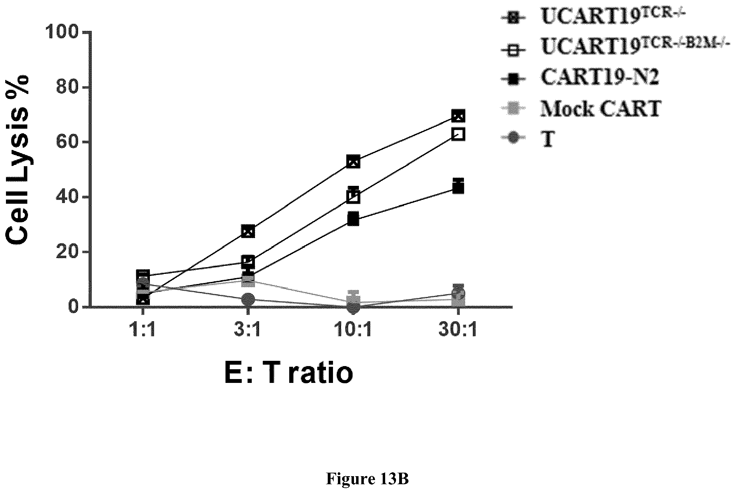

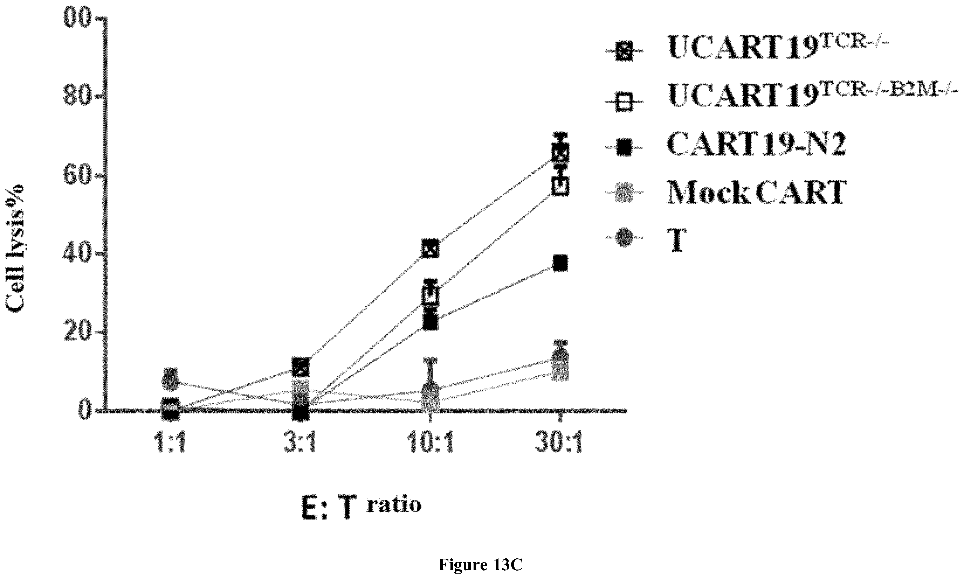

The measured killing results were shown in Figures 13A-13C. It can be seen from the results that CART19-N2 cells and UCART19 cells had a dose-dependent killing activity on hematological tumor cells, and the UCART^{TCR-/-B2M-/-} and UCART^{TCR-/-} cells had higher effect on killing tumor cells when compared to that of CART19.

### Test Example 12. Determination of the expression level of CD107a on the cell surface during the killing of target cells by CART19 and UCART19 cells in vitro

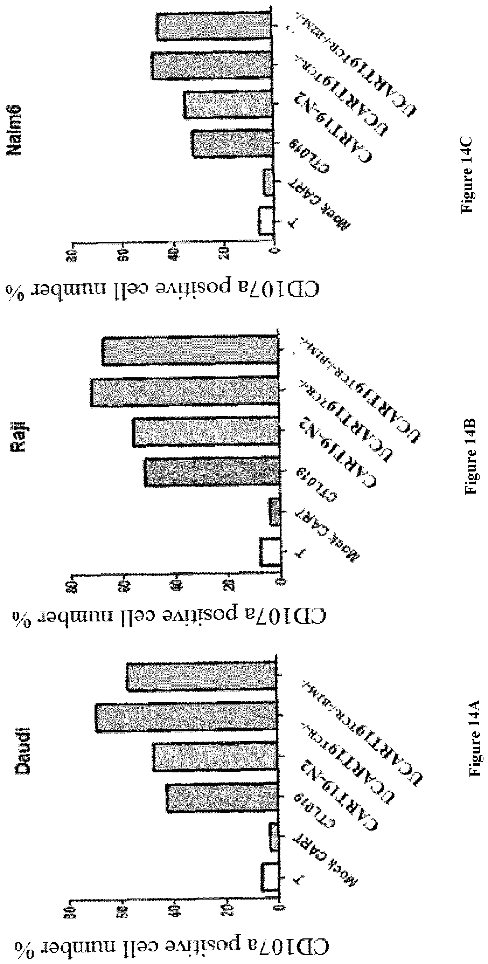

Target cells (Daudi, Raji and Nalm6 cells, all purchased from ATCC) were adjusted to a cell density of 5×10⁵/ml, 100 µl of each was plated into a 96-well round bottom plate, then added with effector cells CTL-019, CART19-N2, UCART19^{TCR-/-} and UCART19^{TCR-/-B2M-/-} at a ratio of effector cell to target cell (E:T ratio=10: 1), and mixed by pipetting. Centrifugation was performed at 1000 rpm for 2 min, the cells were incubated in an incubator for 4 hours, and the cells were stained with anti-human CD8 and anti-human CD107a antibody. The ratio of CD107a-positive cells was measured by flow cytometry. Results were shown in Figs. 14A to 14C.

From the results, it can be seen that CART19-N2 cells and UCART19^{TCR-/-} and UCART19^{TCR-/-B2M-/-} all showed significant up-regulated expression of CD107a, and such expression was significantly higher than that of CTL-019 cells, indicating that the killing CART cells, rather than helper CART cells, mainly contribute to the killing effect during the killing by CART cells.

### Test Example 13. Analysis of in vivo antitumor activity CART19 cells in mice

Raji-luciferase cells were constructed as follows: The gene sequence of luciferase was constructed into the pLVX-EF1 viral vector, packaged into a lentivirus and then transfected into Raji cells (purchased from ATCC). The Raji-luciferase positive cells were sorted by Flow Cell Sorter. The cells were expanded and cultured for use.

NOG mice (purchased from Beijing Vital River Laboratory Animal Technologies Co. Ltd), female, 6-8 weeks, feeding environment: SPF level. One week after adaptive feeding, the mice were randomly divided into 6 groups, 6 mice per group. Each mouse was injected intravenously with 3.5×10⁵ Raji-luciferase tumor cells, and the bioluminescence intensity of tumor cells was recorded 7 days later. Each mouse was injected with 1×10⁷ CART cells. The bioluminescence intensity of Raji-luciferase cells in mice was recorded every week by using PE Small Animal Imager. The effects of different CARTs on killing Raji tumor cells *in vivo* were compared. The grouping of NOG mice and the injection of CART19 cells were as follows:

**Table 4.**

| Group | Cell types for injection | Number of cells for injection | Number of mice |
|---|---|---|---|
| 1 | CART19-N1 | 1×10⁷ | 6 |
| 2 | CART 19-N2 | 1×10⁷ | 6 |
| 3 | CART 19-N3 | 1×10⁷ | 6 |
| 4 | CTL-019 | 1×10⁷ | 6 |
| 5 | Mock CART (vehicle) | 1×10⁷ | 6 |
| 6 | PBS | no | 6 |

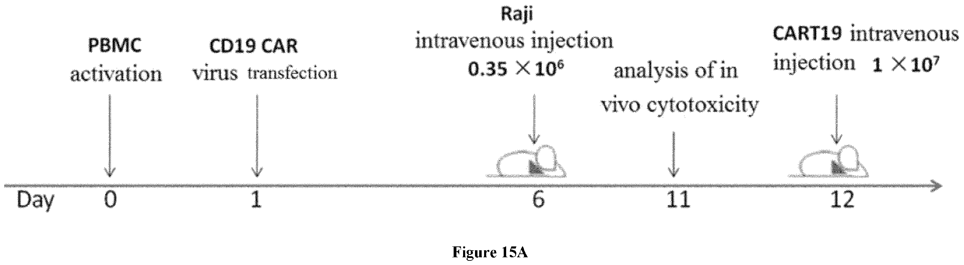

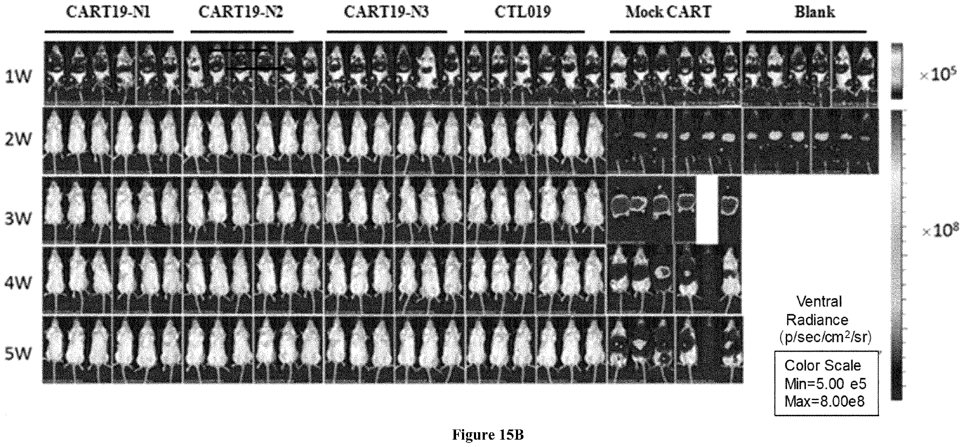

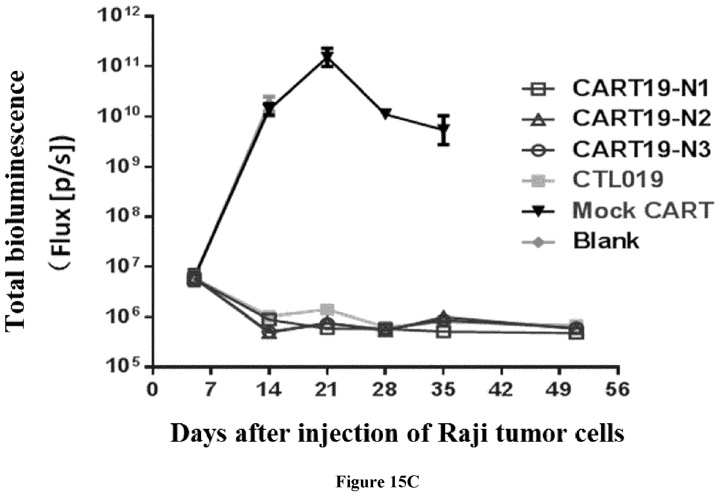

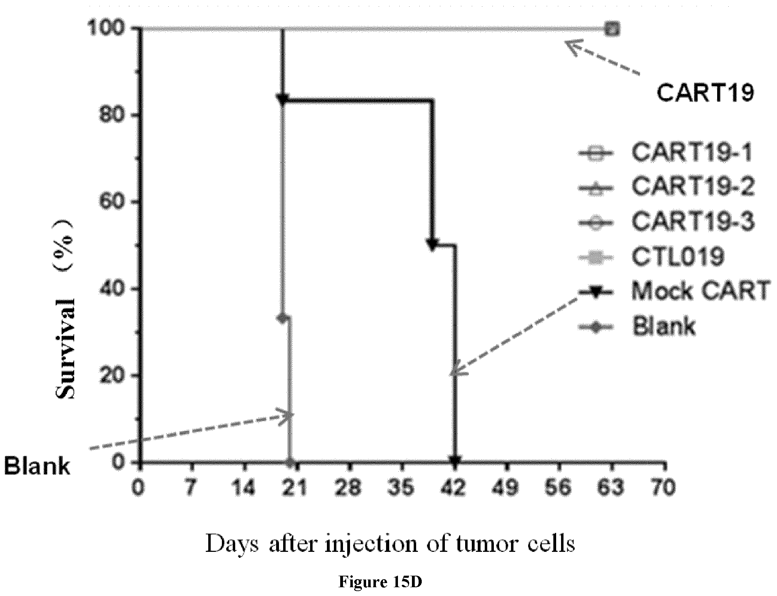

Five weeks after the establishment of mouse model and injection, the results of experimental photographs and statistical bioluminescence intensities were shown in Figures 15A-15B, and the overall survival rate was shown in Figure 15C. It can be seen from the figures that CART19-N1, CART19-N2 and CART19-N3 has similar anti-tumor effects to Novartis CTL-019, and all can effectively kill leukemia tumor Raji cells *in vivo.*

### Test Example 14. Analysis of antitumor activity of CART19 and UCART19 cells in mice after stimulation with K562 cells

CART19 and UCART19 cells were cultured for 12 days and stimulated with K562-CD19 cells. 1×10⁸ K562-CD19 cells were washed once with 1640+10% FBS, and then resuspended in 10 ml of 1640+10% FBS. 25µl of mytomycin (20 mg/ml, R&D, Cat. No. 3258) was added at 1:400 to a final concentration of 50µg/ml, and incubated at 37°C for 30 min. After centrifuging, the supernatant was discarded and the cells were washed three times with 15 ml of 1640 + 10% FBS, and the supernatant was thoroughly removed at the last time. The cell density was adjusted to 1 × 10⁸/ml by adding 1 ml of X-VIVO medium containing 100 IU/ml of rIL-2. Mytomycin-treated K562-CD19 cells were added at a ratio of CAR-T: K562-CD19=5:1, and incubated in an incubator at 37 ° C for further culture.

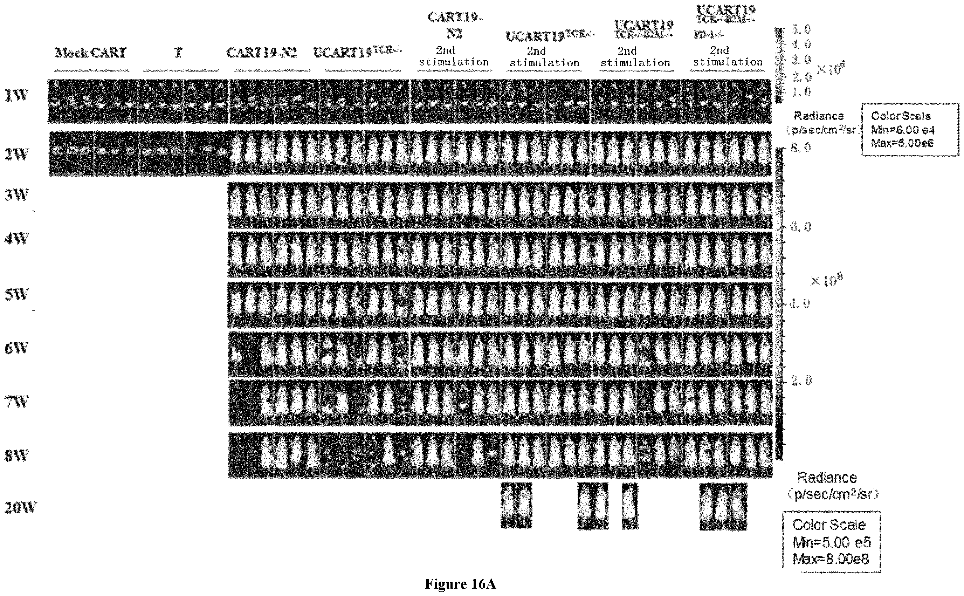

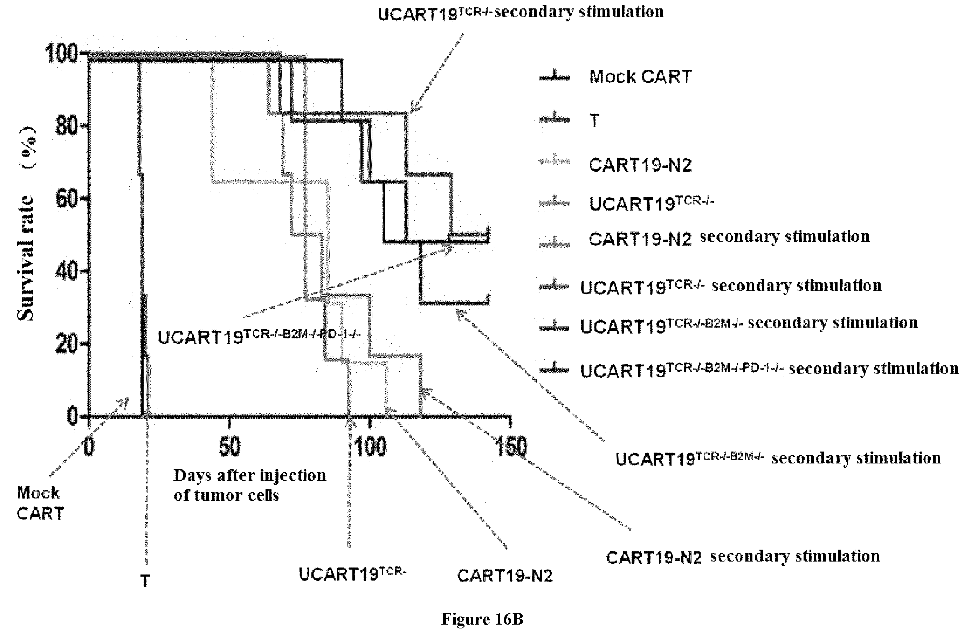

Method for establishment of model and *in vivo* injection was as follows: NOG mice (purchased from Beijing Vital River Laboratory Animal Technologies Co. Ltd, female, 6-8 weeks), feeding environment: SPF level. One week after adaptive feeding, the mice were randomly divided into 8 groups, 6 animals per group. Each mouse was injected intravenously with 3.5×10⁵ Raji-luciferase tumor cells for establishing NOG mouse model. After 7 days, 1×10⁷ CART19 cells were injected into each mouse, and the bioluminescence intensity of Raji-luciferase cells in mice was recorded every week by using PE Small Animal Imager after injection of CART cells. The effects of different CARTs on killing Raji tumor cells *in vivo* were compared. The effects of different CART19s on killing Raji tumor cells *in vivo* were compared. Five weeks after the establishment of mouse model and injection, the results of experimental photographs and statistical bioluminescence intensities were shown in Figure 16A, and the overall survival rate was shown in Figure 16B. The grouping of NOG mice was as follows:

**Table 5.**

| Group | Cell types for injection | Number of cells for injection | Number of mice |
|---|---|---|---|
| 1 | Mock (vehicle) | 1×10⁷ | 6 |
| 2 | T cells | 1×10⁷ | 6 |
| 3 | CART19-N2 | 1×10⁷ | 6 |
| 4 | UCART19^{TCR-/-} | 1×10⁷ | 6 |
| 5 | UCART^{TCR-/-B2M-/-PD-1-/-} | 1×10⁷ | 6 |
| 6 | CART 19-N2 undergoing secondary stimulation | 1×10⁷ | 6 |
| 7 | UCART19^{TCR-/-} undergoing secondary stimulation | 1×10⁷ | 6 |
| 8 | UCART1 9^{TCR-/- B2M-/-} undergoing secondary stimulation | 1×10⁷ | 6 |
| 9 | UCART^{TCR-/-B2M-/-PD-1-/-} undergoing secondary stimulation | 1×10⁷ | 6 |

From the results, it was found that the injection of CART19 cells and UCART19^{TCR-/-} cells can both significantly prolong the survival of the mouse model; the mice injected with UCART19^{TCR-/-} cells (secondarily stimulated with K562-CD19 cells) had a longer survival compared to those injected with CART19 cells, see Figure 16B.

### Test Example 15. Analysis of in vivo proliferation of CART cells in mice

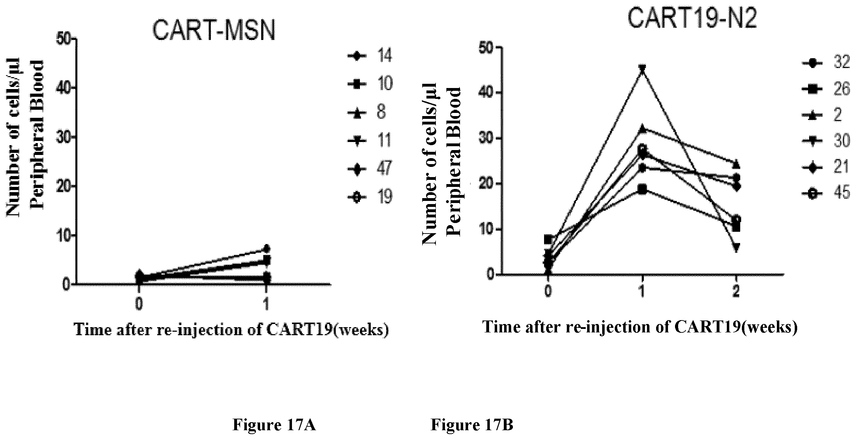

NOG mice (purchased from Beijing Vital River Laboratory Animal Technologies Co. Ltd, female, 6-8 weeks), feeding environment: SPF level. One week after adaptive feeding, the mice were randomly divided into 8 groups, 6 animals per group. Each mouse was injected intravenously with 3.5×10⁵ Raji tumor cells (purchased from ATCC) for the establishment of model. After 6 days, each mouse was injected with 1×10⁷ CART19-N2 cells and negative control CART-MSN cells. On the next day after injection of CART cells, blood was taken from mouse eye, and the number of CART cells in the peripheral blood of the mice was measured by using anti-human CD45 antibody (purchased from BD, Cat. No. 557748). The measure was performed every week (7-day interval from the previous blood collection) thereafter. Within 3 weeks after injection, the results of changes in the number of human T cells in peripheral blood of mice were shown in Figures 17A-17B. The numbers in the figure represent mouse numbers and groups were as follows:

**Table 6.**

| Group | 1 | 2 |
|---|---|---|
| Cell types for injection | CART-MSN | CART19-N2 |
| Number of cells for injection | 1×10⁷ | 1×10⁷ |
| NOG No. | 6 | 6 |
| Mouse No. | 14, 10, 8, 11, 47, 19 | 32, 26, 2, 30, 21, 45 |

As can be seen from the results, human CART19-N2 cells were significantly expanded in mice, after CART19-N2 cells were injected into mice carrying Raji tumor cells. However, the number of CART-MSN cells did not change significantly, indicating that CART19-N2 cells were specifically stimulated by Raji tumor cells in mice and exhibited amplification signals.

### Test Example 16. Determination of alloreactivity of T cells having knockout of TCR

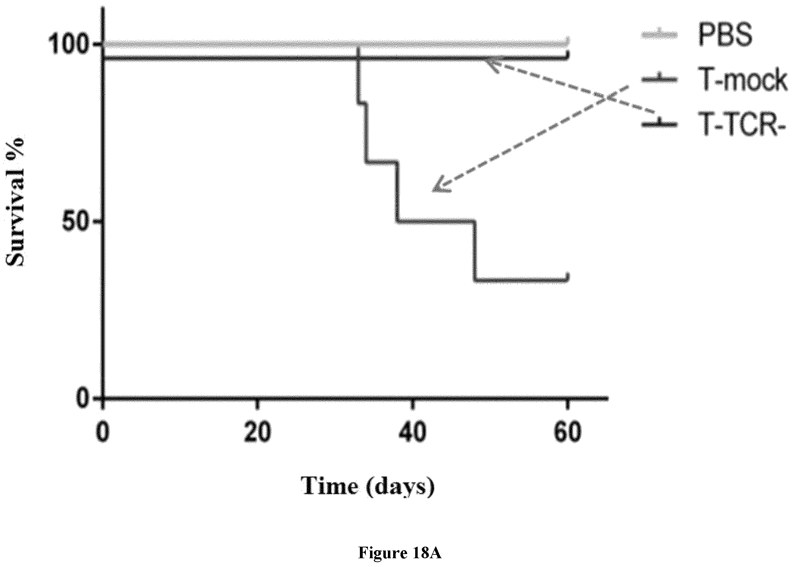

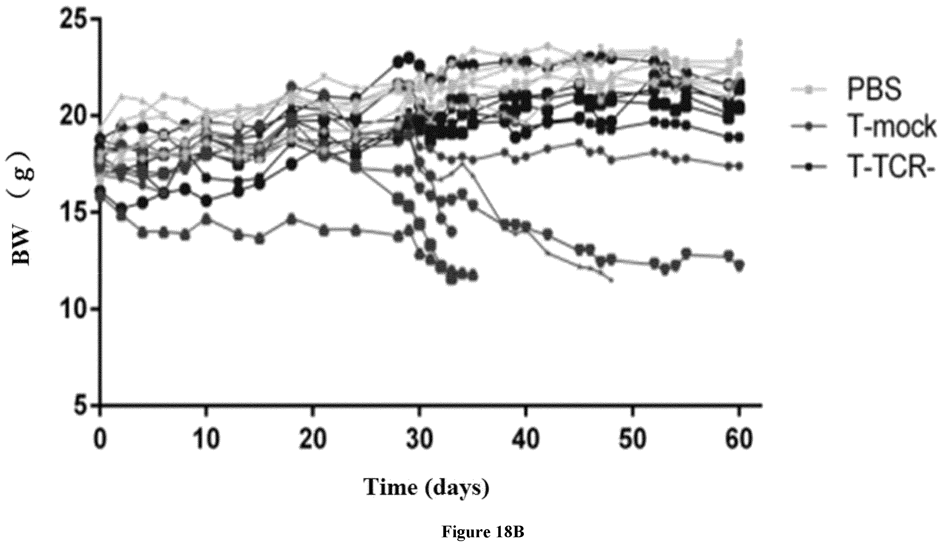

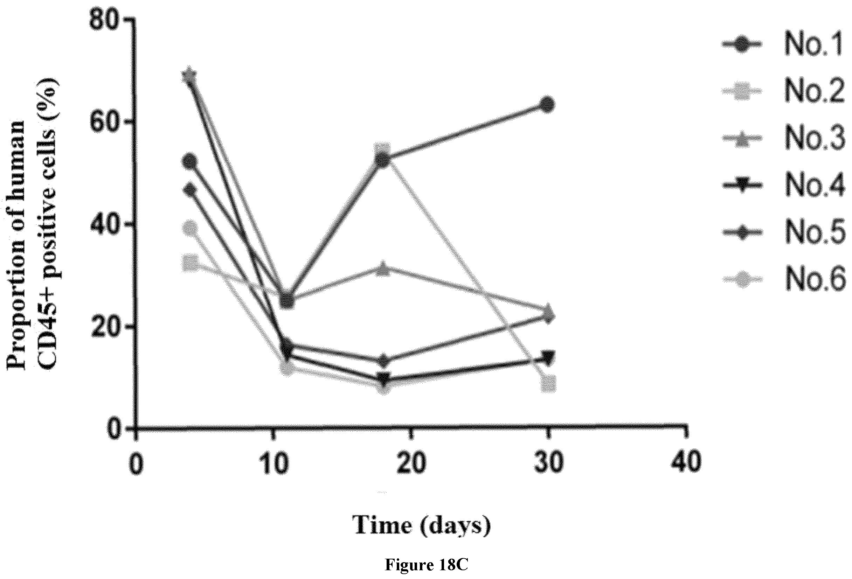

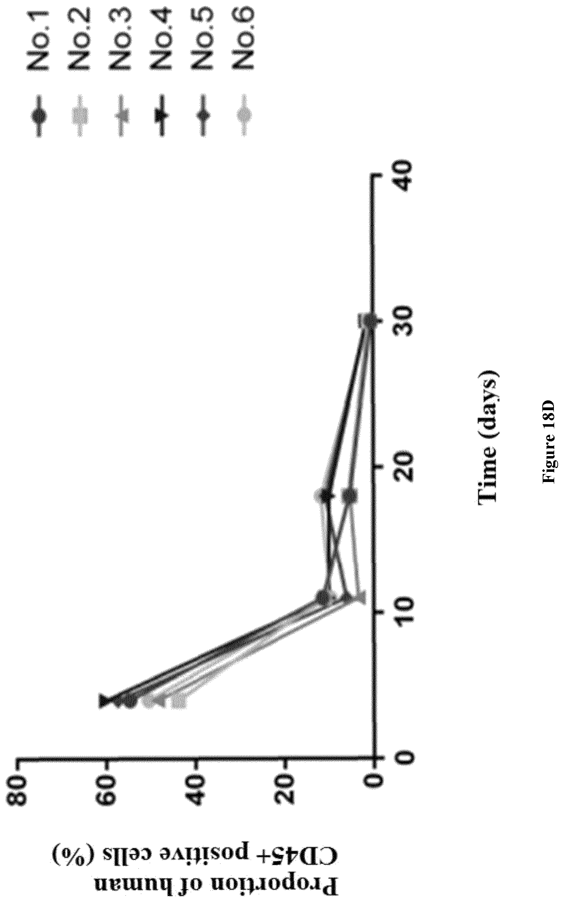

NOG mice (purchased from Beijing Vital River Laboratory Animal Technologies Co. Ltd, female, 6-8 weeks), feeding environment: SPF level. After one week of adaptive feeding, the mice were randomly divided into 5 groups, 6 animals per group. The mice were irradiated with a dose of 1 Gy from the irradiator. On day 3, the mice were injected into tail vein with PBS, 1×10⁷ TCR knockout human T cells (T-TCR⁻), 1×10⁷ mock TCR knockout human T cells (T-mock), 1×10⁷ CTL-019 cells and 1×10⁷ TCR knockout CTL-019^{TCR-/-} cells, respectively. For the preparation and screening procedures, see Example 5, Example 6 and Example 7. After 5 days, the mice were weighed every other day. Blood was taken from the fundus venous plexus of mice every week after the injection, and the number of human CD45-positive T cells in the peripheral blood of mice was measured. The groups of mice were shown in the table below. The results of the survival, body weight, and the number of human CD45-positive T cells *in vivo* were shown in Figs. 18A to 18D.

**Table 7.**

| Group | PBS group | T-mock group | T-TCR⁻ group | CTL-019 group | CTL-019^{TCR-/-} group |
|---|---|---|---|---|---|
| Cell types for injection | PBS | T-mock | T-TCR⁻ | CTL-019 | CTL-019^{TCR-/-} |
| Number of cells for injection | none | 1×10⁷ | 1×10⁷ | 1×10⁷ | 1×10⁷ |
| NOG No. | 6 | 6 | 6 | 6 | 6 |
| Mouse No. | 1, 2, 3, 4, 5, 6 | 1, 2, 3, 4, 5, 6 | 1, 2, 3, 4, 5, 6 | 1, 2, 3, 4, 5, 6 | 1, 2, 3, 4, 5, 6 |

The results showed that the mice injected with T-TCR⁻ cells had similar survival rate compared to that of the mice injected with PBS, both were significantly higher than that of the mice injected with human T-mock cells; and the body weight of the mice injected with human T-mock cells was decreased significantly, while the mice injected with T-TCR⁻ and PBS did not show significant body weight loss. The T cells in mice in the T-mock group were significant increased than those in the T-TCR⁻ group, indicating that TCR knockout can reduce the GvHD effect in mice. The proportion of human CD45⁺ cells in blood of the mice injected with CTL-019^{TCR-/-} was significantly decreased compared to that of the CTL-019 group, in which TCR was not knocked out, further indicating that TCR knockout can reduce the GvHD effect in mice.

## Claims

1. An isolated chimeric antigen receptor, comprising a CD19 antigen binding domain, wherein the CD19 antigen binding domain comprises the amino acid sequence shown in SEQ ID NO: 18, SEQ ID NO: 20, SEQ ID NO: 22 or SEQ ID NO: 24.

2. The isolated chimeric antigen receptor according to claim 1, further comprising a co-stimulatory signaling region and/or a CD3ζ signaling domain.

3. The isolated chimeric antigen receptor according to claim 2, wherein the co-stimulatory signaling region comprises an intracellular domain of a co-stimulatory molecule selected from the group consisting of CD27, CD28, 4-1BB, OX40, CD30, CD40, PD-1, ICOS, lymphocyte function associated antigen-1, CD2, CD7, LIGHT, NKG2C, B7-H3 and any combination thereof, preferably 4-1BB co-stimulatory signaling region shown in amino acid sequence SEQ ID NO: 12.

4. The isolated chimeric antigen receptor according to claim 2 or 3, wherein the CD3ζ signaling domain comprises the amino acid sequence shown in SEQ ID NO: 14 or SEQ ID NO: 57.

5. The isolated chimeric antigen receptor according to any one of claims 2 to 4, further comprising an extracellular hinge domain, preferably, the extracellular hinge domain comprises the human CD8α leading signal region as shown in amino acid sequence SEQ ID NO: 6 and the human CD8α hinge region as shown in amino acid sequence SEQ ID NO: 8.

6. The isolated chimeric antigen receptor according to claim 5, further comprising a CD8α transmembrane domain, preferably, the amino acid sequence of CD8α transmembrane domain is shown in SEQ ID NO: 10.

7. The isolated chimeric antigen receptor according to any one of claims 1 to 6, comprising the amino acid sequence shown in SEQ ID NO: 26, SEQ ID NO: 28, SEQ ID NO: 30 or SEQ ID NO: 32.

8. An isolated nucleic acid molecule encoding the chimeric antigen receptor according to any one of claims 1 to 7.

9. The isolated nucleic acid molecule of claim 8, comprising the nucleic acid sequence shown in SEQ ID NO: 17, SEQ ID NO: 19, SEQ ID NO: 21, or SEQ ID NO: 23.

10. The isolated nucleic acid molecule according to claim 9, comprising a nucleic acid sequence encoding co-stimulatory signaling region and/or a nucleic acid sequence encoding CD3ζ signaling domain, preferably, the nucleic acid sequence encoding co-stimulatory signaling region is shown in SEQ ID NO: 11, and the nucleic acid sequence encoding CD3ζ signaling domain is shown in SEQ ID NO: 13 or SEQ ID NO: 56.

11. The isolated nucleic acid molecule of claim 10, further comprising a nucleic acid sequence encoding extracellular hinge domain, preferably, the nucleic acid sequence encoding extracellular hinge domain comprises the human CD8α leading signal region shown in SEQ ID NO: 5 and the human CD8α hinge region shown in SEQ ID NO: 7.

12. The isolated nucleic acid molecule of claim 11, further comprising a CD8α transmembrane domain shown in SEQ ID NO: 9.

13. The isolated nucleic acid molecule according to any one of claims 8 to 12, comprising a nucleic acid sequence encoding CD19 chimeric antigen receptor shown in SEQ ID NO: 25, SEQ ID NO: 27, SEQ ID NO: 29 or SEQ ID NO: 31.

14. A vector comprising the isolated nucleic acid molecule of any one of claims 8 to 13; preferably, the vector is selected from the group consisting of DNA, RNA, plasmid, lentiviral vector, adenoviral vector, and retroviral vector; most preferably, the vector further comprises a promoter, preferably EF-1 promoter shown in SEQ ID NO: 4.

15. T cell, which comprises the isolated nucleic acid molecule according to any one of claims 8 to 13 or the vector according to claim 14, or is genetically modified to express the chimeric antigen receptor according to any one of claims 1 to 7.

16. The T cell according to claim 15, which is a modified T cell and comprises a nucleic acid capable of down-regulating endogenous gene(s) of the T cell, wherein the endogenous gene(s) is (are) any one or more selected from the group consisting of TRAC, TRBC, B2M and PD-1 genes.

17. The T cell according to claim 16, wherein the nucleic acid capable of down-regulating endogenous gene(s) of the T cell is selected from the group consisting of antisense RNA, antigomer RNA, siRNA, shRNA and CRISPR-Cas9 system, preferably CRISPR-Cas9 system;
wherein the CRISPR-Cas9 system further comprises sgRNA(s) targeting the endogenous gene coding sequence(s) or expression-regulating sequence(s) thereof of the T cell, wherein the sgRNA(s) is/are formed by linking, from 5' to 3', a crRNA targeting the endogenous gene(s) with 17 nt, 18 nt, 19 nt or 20 nt in length to a tracrRNA corresponding to the Cas9 protein.

18. The T cell according to claim 17, wherein the endogenous gene(s) is(are) one or more selected from the group consisting of TRAC, B2M and PD-1, wherein the crRNA(s) that target the endogenous gene TRAC is (are) any one or more crRNAs selected from the group consisting of SEQ ID NOs: 37, 38, 39, 40, 41, 42, 43, 44, 45, 46, 47 and 48, preferably SEQ ID NO: 47; the crRNA that targets the endogenous gene B2M is shown in SEQ ID NO: 49; and the crRNA(s) that target the endogenous gene PD-1 is (are) any one or more crRNAs selected from the group consisting of SEQ ID NOs: 50, 51 and 52, preferably SEQ ID NO: 52.

19. The T cell according to claim 18, wherein the crRNA that down-regulates the endogenous gene TRAC is shown in SEQ ID NO: 47, the crRNA that down-regulates the endogenous gene B2M is shown in SEQ ID NO: 49, and the crRNA that down-regulates the endogenous gene PD-1 is shown in SEQ ID NO: 52; and
the chimeric antigen receptor comprises the amino acid sequence shown in SEQ ID NO: 26, SEQ ID NO: 28, SEQ ID NO: 30 or SEQ ID NO: 32, and preferably comprises the amino acid sequence shown in SEQ ID NO: 28.

20. A method for preparing the T cell according to any one of claims 16 to 19, including:
a) introducing a nucleic acid encoding a chimeric antigen receptor into the T cell; and
b) introducing a sgRNA nucleic acid into the T cell via the CRISPR-Cas9 system, said sgRNA nucleic acid is capable of down-regulating the expression of endogenous target gene(s) of the T cell.

21. A pharmaceutical composition comprising one or more selected from the group consisting of:
i) the isolated chimeric antigen receptor according to any one of claims 1 to 7,
ii) the isolated nucleic acid molecule according to any one of claims 8 to 13,
iii) the vector according to claim 14, and
iv) the T cell according to any one of claims 15 to 19;
and, a pharmaceutically acceptable carrier, diluent or excipient.

22. Use of the pharmaceutical composition according to claim 21 or the T cell according to any one of claims 15 to 19 in the preparation of a medicament for treating or preventing tumor disease, autoimmune disease, infectious disease caused by virus or bacteria.

23. The use according to claim 22, wherein the tumor disease is CD19-mediated cancer, preferably selected from the group consisting of breast cancer, prostate cancer, ovarian cancer, cervical cancer, skin cancer, pancreatic cancer, colorectal cancer, kidney cancer, liver cancer, brain cancer, lung cancer, thyroid cancer and hematological cancer; more preferably is hematological cancer, wherein the hematological cancer is selected from the group consisting of acute leukemia including acute lymphocytic leukemia, acute myelocytic leukemia, acute myelogenous leukemia and myeloblastic leukemia, promyelocytic leukemia, myelomonocytic leukemia, monocytic leukemia and erythroleukemia; and chronic leukemia including chronic myelocytic (granulocytic) leukemia, chronic myelogenous leukemia and chronic lymphocytic leukemia and refractory CD19⁺ leukemia and lymphoma; polycythemia vera, lymphoma, mantle cell lymphoma, diffuse large B-cell lymphoma, Hodgkin's disease, non-Hodgkin's lymphoma, multiple myeloma, Waldenstrom's macroglobulinaemia, heavy chain disease, myelodysplastic syndrome, hairy cell leukemia and myelodysplasia; most preferably acute lymphocytic leukemia or chronic lymphocytic leukemia.
